# EUROPEAN PATENT APPLICATION

(11) **EP 2 511 291 A2**
(43) Date of publication of application: **17.10.2012**
(21) Application number: 12164042.9
(22) Date of filing: 13.06.2008
(51) Int. Cl.: C07K 14/295

(54) **Chlamydia antigens**

(30) Priority: 18.06.2007 EP 07110472
(62) Divisional of application: 08760988.9
(71) Applicant: Intercell AG, 1030 Vienna (AT)
(72) Inventor: Meinke, Andreas, 3013 Pressbaum (AT); Nagy, Eszter, 1070 Vienna (AT); Noiges, Birgit, 2114 Großrußbach (AT); Von Gabain, Alexander, 1180 Vienna (AT); Asklin, Johanna, 23635 Höllviken (SE)
(74) Representative: Bösl, Raphael Konrad

(57) **Abstract**

The present invention relates to isolated nucleic acid molecules which encode an antigen from a *Chlamydia* species, a vector which comprises such nucleic acid molecule, and a host cell comprising such vector. Furthermore, the invention provides antigens from a *Chlamydia* species, as well as fragments and variants thereof, a process for producing such antigens, and a process for producing a cell, which expresses such antigen. More specifically such antigens are produced by or associated with bacterial infections caused by *Chlamydia pneumoniae.* Moreover, the present invention provides antibodies binding to such antigen, a hybridoma cell producing such antibodies, methods for producing such antibodies, a pharmaceutical composition comprising such nucleic acid molecule, antigen, vector or antibody, the use of such nucleic acid molecule, antigen, vector or antibody for the preparation of a pharmaceutical composition, methods for identifying an antagonist capable of binding such antigen or of reducing or inhibiting the interaction activity of such antigen, methods for diagnosing an infection with *Chlamydia* and methods for the treatment or prevention of an infection with *Chlamydia.*

## Description

The present invention relates to isolated nucleic acid molecules which encode an antigen from a *Chlamydia* species, a vector which comprises such nucleic acid molecule, and a host cell comprising such vector. Furthermore, the invention provides antigens from a *Chlamydia* species, as well as fragments and variants thereof, a process for producing such antigens, and a process for producing a cell, which expresses such antigen. More specifically such antigens are produced by or associated with bacterial infections caused by *Chlamydia pneumoniae.* Moreover, the present invention provides antibodies binding to such antigen, a hybridoma cell producing such antibodies, methods for producing such antibodies, a pharmaceutical composition comprising such nucleic acid molecule, antigen, vector or antibody, the use of such nucleic acid molecule, antigen, vector or antibody for the preparation of a pharmaceutical composition, methods for identifying an antagonist capable of binding such antigen or of reducing or inhibiting the interaction activity of such antigen, methods for diagnosing an infection with *Chlamydia* and methods for the treatment or prevention of an infection with *Chlamydia.*

*Chlamydia pneumoniae (C. pneumoniae)* is an obligate intracellular bacterium and recognized as a significant human pathogen. It is a common cause of pneumonia and upper respiratory tract disease in hospital and outpatient settings, accounting for approximately 7 to 10% of cases of community-acquired pneumonia among adults (Montigiani, S. et al., 2002). Infection with *Chlamydia pneumoniae* has also been associated with other respiratory tract diseases such as bronchitis, sinusitis, asthmatic bronchitis, adult-onset asthma, and chronic obstructive pulmonary disease (COPD; Murdin, A.D. et al., 2000). Importantly, *Chlamydia pneumoniae* infection has also been associated with atherosclerosis and cardiovascular disease, which was indicated for example by seroepidemiologic studies or detection of *C*. *pneumoniae* in atherosclerotic plaques (Montigiani, S. et al., 2002).

It was recently suggested that the Gram-negative *Chlamydiaceae,* a family of uncertain origin and the only members of the order *Chlamydiales,* can be divided into two genera, *Chlamydia* and *Chlamydophila,* by 16S rRNA phylogeny (Everett, K.D. et al., 1999). According to this suggestion, three species are described within the genus *Chlamydia: Chlamydia trachomatis, Chlamydia muridarum* and *Chlamydia suis.* The species *Chlamydia psittaci, pecorum* and *pneumoniae* were suggested to be renamed to *Chlamydophila psittaci, pecorum* and *pneumoniae.* Nevertheless, bacteria of both genera share biological and biochemical properties. For the present invention, the newly suggested nomenclature has not been used yet, but for reasons of completeness it should be mentioned that the species *Chlamydia pneumoniae* and *Chlamydophila pneumoniae* are identical.

Sequencing of seven *Chlamydiaceae* genomes from four different species has demonstrated that profound differences in host range and disease can be caused by fairly subtle variations in gene content (Read, T.D. et al., 2003). The *Chlamydiaceae* are classified among the eubacteria as a well-isolated group, with only a very weak link to the planctomyces. The *Chlamydiaceae* therefore exhibit some unique characteristics within the eubacteria, in particular their development cycle and the structure of their membranes. They have a unique two-phase cell cycle: the elementary body (EB), a small extracellular form, which attaches to the host and is phagocytosed. Subsequently, it is converted in the phagosome to the replicative intracellular form, the reticulate body. As obligate intracellular bacteria, the *Chlamydiaceae* multiply in eukaryotic cells at the expense of their energy reserves and nucleotide pools; they are responsible for a wide variety of diseases in mammals and birds.

The species *Chlamydia trachomatis* is the best characterized. *C. trachomatis* is comprised of two human biovars: the trachoma and lymphogranuloma venereum (LGV). The mouse and swine strains that were previously grouped with this species now belong to separate species *(C. muridarum* and *C. suis,* respectively). Serovars in both *C. trachomatis* biovars cause trachoma, sexually transmitted disease, some forms of arthritis, and neonatal inclusion conjunctivitis and pneumonia. The trachoma biovar currently has 14 serovars and infection is limited primarily to epithelial cells of mucous membranes. It has also been detected in posterior bilaminar tissue removed from patients with disease of the temporomandibular joint. The LGV biovar consists of four serovars, L1, L2, L2a and L3, which can invade lymphatic tissue. While specific strains of the trachoma biovar are mainly found in eye infections, other strains of the trachoma biovar and LGV are essentially responsible for genital entry infections. It should be mentioned that the LGV strains are responsible for systemic diseases. Historically, the characterization of the *Chlamydia trachomatis* microorganism was only successfully carried out in 1957, after a series of isolations in cell cultures.

The species *Chlamydia psittaci* infects many animals, in particular birds, and is transmissible to humans. It is responsible for atypical pneumonia, for hepatic and renal dysfunction, for endocarditis and for conjunctivitis.

*Chlamydia pecorum* does not infect humans, but is rather a pathogen of ruminants.

It was in 1983 that *Chlamydia pneumoniae* was recognized as a human pathogen (Grayston, J.T. et al., 1986). Thereafter, special attention has been paid to this bacterium and it is estimated (Gaydos, C.A. et al., 1994) that 10% of pneumonias, and 5% of bronchitis and sinusitis are attributable to *Chlamydia pneumoniae* (Aldous, M.B. et al., 1992). More recently, the association of this bacterium with the pathogenesis of asthmatic disease and of cardiovascular impairments is increasingly of interest.

Serological studies have shown that *Chlamydia pneumoniae* infection is common in children between 5 and 16 years of age. Before this age, it is rare to find antibodies against *C*. *pneumoniae* and the best available data indicate that children begin to seroconvert at an age of about 5 years. The increase in the number of individuals carrying antibodies correlates then with age up to 20 years. Accordingly, 50% to 70% of adults are carriers of antibodies. Since the persistence of induced antibodies over time is limited to 3 or at most 5 years after a first infection, it is suggestive that frequent reinfection occurs during the entire lifespan. The annual seroconversion rate is about 6 to 8% between 8 and 16 years (Kuo, C.-C. et al., 1995) and the seroprevalence of the disease before the age of 15 years is identical between both sexes. After this age, men are more frequently infected than women in all regions worldwide.

These *Chlamydia* infections are geographically highly widespread throughout the world (Tong, C.Y. et al., 1993), with the lowest infection rates observed in developed countries of the north such as Canada and the Scandinavian countries. In contrast, the highest prevalence rates are found in the less developed countries of tropical regions where infections may occur before the age of 5 years. Humans are the only known reservoir for *Chlamydia pneumoniae* and it is probable that the infection is caused by person-to-person transmission by respiratory secretions (Aldous, M.B. et al., 1992). The chain of transmission may also appear to be indirect (Kleemola, M. et al., 1988), suggestive of an infection caused by an effective transmission and of the possibility that also asymptomatic carriers exist, which could explain the high prevalence of the disease. This is also in accordance with the finding that *Chlamydia pneumoniae* can survive for up to 30 hours in a hostile environment (Falsey, A.R. et al., 1993), although the infectivity of the microorganism in the open air decreases rapidly under conditions of high relative humidity. The incubation period is several weeks which is significantly longer than that of many other respiratory pathogenic bacteria.

The main clinical manifestations caused by *Chlamydia pneumoniae* are essentially respiratory diseases. Pneumonia and bronchitis are the most frequent, because they are clinically obvious and the infectious agent may be identified. Isolation of the etiologic agent is difficult though and paired acute- and convalescent-phase sera are required to confirm the diagnosis using antibody tests. The asymptomatic diseases caused by *Chlamydia pneumoniae* are probably numerous (e.g. Grayston, J.T., 1992). Other syndromes such as sinusitis, purulent otitis media (Ogawa, H. et al., 1992), or pharyngitis have been described, as well as infections with respiratory impairments similar to asthma (Hahn, D.L. et al., 1991). *Chlamydia pneumoniae* has also been associated with sarcoidosis, with erythema nodosum (Sundelof, B. et al., 1993) and one case of Guillain-Barré syndrome has been described (Haidl, S. et al., 1992). The involvement of *Chlamydia pneumoniae* in Reiter's syndrome has also been evaluated (Braun, J. et al., 1994).

Cardiovascular diseases are the major cause of death in the countries of the Western world. The association of *Chlamydia pneumoniae* with the development of cardiovascular diseases such as coronary heart disease and myocardial infarction was first suspected due to the observation of high antibody levels in patients with heart disease (e.g. Shor, A. et al., 1992). In addition, anatomicopathological and microbiological studies were able to detect *C. pneumoniae* in the vessels. Studies from several countries have also shown that *Chlamydia pneumoniae* infection correlates with atheromatous impairments in patients (e.g. Grayston, J.T. et al., 1996). Thus it also appears that the bacterium is more frequently found in old atheromatous lesions, than in early lesions, but it is not found in subjects free of atheromatous disease. It is therefore supported by these studies that the atheroma plaque is very strongly correlated with the presence of *Chlamydia pneumoniae.* Nevertheless, the role that the bacterium plays in vascular pathology is not yet defined.

For the treatment of *Chlamydia pneumoniae* infections, there are only limited data available from controlled clinical studies. Similar to Lyme disease and Mycoplasma infection and due to the intracellular nature of *C*. *pneumoniae,* long term antimicrobial treatment is needed. This extensive antimicrobial treatment is required for eradication of *C*. *pneumoniae* from macrophages and endothelial cells of infected arteries. Unlike penicillin, Ampicillin or the sulphamides, antibiotics such as erythromycin, tetracycline, doxycycline, ofloxacin, clinafloxacin, ciprofloxacin, azithromycin, Clindamycin and minocycline show an antibiotic activity *in vitro* against *Chlamydia pneumoniae.* However, any treatment at high doses should be continued for several weeks in order to avoid a recurrence of the infection. Accordingly, the use of two new macrolides, clarithromycin and azithromycin, whose diffusion, bioavailability and half-life allow shorter and better tolerated cures, is nowadays preferred. Unfortunately, many conventional treatments against *Chlamydia* still fail, resulting in a significant rate of recurrence and morbidity. In the absence of definitive proof based on the results of clinical studies, an effective and well-tolerated treatment of *Chlamydia pneumoniae* infections that also protects against reinfection therefore remains desirable.

A very important issue is the development of a specific and sensitive diagnosis, which can be carried out conveniently and rapidly, allowing early screening for the infection. Laboratory diagnosis of *C*. *pneumoniae* infection is based on isolation and culture of the microorganism, serology and/or detection of DNA by PCR. Unfortunately, methods based on *Chlamydia pneumoniae* culture are slow and require a considerable know-how because of the difficulty involved in the collection, preservation and storage of the strain under appropriate conditions.

*C. pneumoniae* can be isolated from nasopharyngeal or throat swabs, sputa or pleural fluid from the patients. The nasopharynx appears to be the optimal site for isolation (Block, S. et al., 1995). Upon isolation, the organism requires to be grown in tissue cultures. *C*. *pneumoniae* grows readily in cell lines derived from respiratory tract tissue, specifically Hep-2 and HL cell lines (Roblin, P.M. et al., 1992). After culturing the specimens for 72 hours, culture confirmation can be performed by staining with either a *C*. *pneumoniae* species-specific or a *Chlamydia* genus-specific fluorescein-conjugated monoclonal antibody.

Serological diagnosis in *Chlamydia* is usually performed by using a micro-immunofluorescence test. This method is based on the microscopic detection of antibodies specific to a chlamydial antigen fixed onto glass slides as distinct dots. The assay is considered positive if a clinical specimen contains antibodies reacting with the antigen. This immunoreaction is then visualized with the use of fluorescein-conjugated secondary antibodies (Tuuminen, T. et al., 2000). The micro-immunofluorescence test has proven to be a very specific and sensitive method; however the requirement of specialized fluorescent microscopy equipment and intact purified organisms as antigen for the test performance makes it inapplicable for use in a standard laboratory.

On the other hand, methods based on antigen detection (enzyme immunoassay (EIA), direct fluorescent antibody test (DFA)) or on nucleic acid amplification (Polymerase Chain Reaction (PCR)) provide tests, which are more suitable for laboratory practice. PCR appears to be the most promising technology in the development of a rapid method for detection of *C*. *pneumoniae.* There are at least 19 in-house PCR assays for detection of this pathogen in clinical specimens reported in the literature (Boman, J. et al., 1999). However, none of these assays are standardized or have been adequately validated in comparison to culture methods, and they are not commercially available or have the approval of the FDA. Major variations in these methods include the way of collecting and processing specimens, primer design, nucleic acid extraction, and amplification product detection and identification.

Therefore, a reliable, sensitive and convenient test, which allows distinction between serogroups and *a fortiori* between *Chlamydia pneumoniae* species, is highly desirable. This is all the more important, because the symptoms of *Chlamydia pneumoniae* infection appear slowly, and because not all of the pathologies associated with these infections have yet been identified. In addition, an association is suspected between these infections and serious chronic infections, asthma and atherosclerosis. Although sensitive and specific tests based on antigen detection have been developed, there remains a need for standardized PCR-based detection protocols and tests (Dowell, S.F. et al., 2001).

Chlamydial infections are often chronic and recurrent, suggesting that protective immunity against *Chlamydia* is weak and not necessarily bactericidal or sterilizing. There are currently no vaccines available against chlamydial infections. Although the number of studies and of animal models developed is high, the antigens used have not induced sufficient protective immunity to lead to the development of human vaccines.

A more detailed understanding of the biology of *Chlamydia pneumoniae,* the interactions of the bacteria with their hosts, their escape from immune defenses of the host in particular, but also their involvement in the development of the associated pathologies, will allow a better control, treatment or prevention of *Chlamydia* induced diseases. It is therefore essential to use novel molecular tools, which allow the development of new preventive and therapeutic treatments, new diagnostic methods and new vaccine strategies which are specific, effective and tolerated. This is precisely the object of the present invention.

A vaccine can contain a whole variety of different antigens. Examples of antigens are whole-killed or attenuated organisms, subfractions of these organisms/tissues, proteins, or, in their most simple form, peptides. Antigens can also be recognized by the immune system in form of glycosylated proteins or peptides and may also be or contain polysaccharides or lipids. Short peptides can be used since for example cytotoxic T-cells (CTL) recognize antigens in form of short, usually 8-11 amino acids long, peptides in conjunction with major histocompatibility complex (MHC). B-cells can recognize linear epitopes as short as 4-5 amino acids, as well as three-dimensional structures (conformational epitopes).

In order to obtain sustained, antigen-specific immune responses, adjuvants need to trigger immune cascades that involve all cells of the immune system. Primarily, adjuvants are acting, but are not restricted in their mode of action, on so-called antigen presenting cells (APCs). These cells usually first encounter the antigen(s) followed by presentation of processed or unmodified antigen to immune effector cells. Intermediate cell types may also be involved. Only effector cells with the appropriate specificity are activated in a productive immune response. The adjuvant may also locally retain antigens and other factors that may be co-injected. In addition, the adjuvant may act as a chemoattractant for other immune cells or may act locally and/or systemically as a stimulating agent for the immune system.

The antibodies produced against *Chlamydia* by the human immune system and present in human sera are indicative of the *in vivo* expression of the antigenic proteins and their immunogenicity. The recognition of linear epitopes recognized by serum antibodies can be based on sequences as short as 4-5 amino acids. Of course it does not necessarily mean that these short peptides are capable of inducing the given antibody *in vivo.* For that reason the defined epitopes, polypeptides and proteins are further to be tested in animals (mainly in mice) for their capacity to induce T cells and antibodies against the selected proteins *in vivo.*

*C. pneumoniae* as an obligate intracellular parasite has a unique biphasic life cycle with a smaller extracellular form; the infectious, non-replicating, metabolically relatively inert elementary body (EB), and a larger intracellular form; the infectious, replicating and metabolically active reticulate body. The EBs attach to susceptible host cells and are taken up by phagocytosis. Within the cell they revert to reticulate bodies and replicate before they revert to EBs prior to host cell lysis. Although the immune correlates of protection against *C*. *pneumoniae* are not well defined, studies using mouse models faithfully mimicking important aspects of human infection indicate that particularly CD8⁺ T cells and IFN-γ are critical for protection in mice (Wizel, B. et al., 2002). Furthermore, CD4+ T cells may also be important because they stimulate B cells to proliferate and differentiate into antibody secreting cells. As Th1 type CD4+ T cells can also produce IFN-γ, they can thus contribute to anti-chlamydial immunity in several ways. Since *C. pneumoniae* resides in the membrane bound vacuole, the preferred antigens have to reach the cytosol of infected cells and need to be subsequently recognized as MHC class I-peptide complex by CD8⁺ T cells. Most of the previously reported antigens - which seem to be therefore capable of reaching the cytosol - are located on the cell surface (e.g. outer membrane proteins) or are secreted (e.g. Murdin, A.D. et al., 2000; Wizel, B. et al., 2002). It has been shown that *C*. *pneumoniae* peptide specific CD8⁺ CTL and their soluble factors can inhibit chlamydial growth *in vitro* (Wizel, B. et al., 2002). In addition to the T cell-mediated immune response, antibodies against cell wall proteins induced by B cell epitopes may aid the T cell-mediated immune response and serve multiple purposes: they may inhibit adhesion, interfere with nutrient acquisition, inhibit immune evasion and promote phagocytosis (Hornef, M. et al., 2002). Antibodies against secreted proteins are potentially beneficial in neutralization of their function as toxin or virulence component. It is also known that bacteria communicate with each other through secreted proteins. Neutralizing antibodies against these proteins will interrupt growth-promoting cross-talk between or within chlamydial species. The described experimental approach is based on the use of antibodies specifically induced by *C*. *pneumoniae* purified from human serum. The antigens identified by the genomic screens are thereby shown to be expressed *in vivo* in the host and to be capable of inducing an antibody response. Since it has been shown for many proteins that B cell and T cell epitopes reside in the same protein, the most promising candidates identified by the genomic screens can be further evaluated for the induction of a potent T cell response *in vivo.* As a first step, bioinformatic analyses have been used to identify potential T cell epitopes *in silico,* which can then be tested in the appropriate murine model of infection. Thus the present invention combines the experimental identification of immunogenic proteins with the bioinformatic prediction of T cell epitopes in order to provide candidates for an efficient vaccine to treat or prevent chlamydial infections.

There have been concerns to develop an inactivated whole cell vaccine for humans, because of the potential risk that it may induce cross-reactive antibodies to human antigens. Therefore, subunit vaccines are considered to have the greatest potential in preventing infections by *Chlamydia pneumoniae.*

The complete genome sequences of three isolates of *C*. *pneumoniae* (AR39, J138, CWL029) were determined by various institutions (Kalman, S. et al., 1999; Read, T.D. et al., 2000; Shirai, M. et al., 2000; see also http://www.tigr.org/tigr-scripts/CMR2/CMRHomePage.spl). Although the two strains AR39 and CWL029 were isolated in the U.S.A. before 1987 and Japan in 1994, respectively, their sequence is to a high degree identical, indicating a divergence in recent human history. In addition to these three *C. pneumoniae* strains, the sequence of two *C*. *trachomatis* strains, D/UW-3/CX (serovar D) and HAR-13 (Kalman, S. et al., 1999; Stephens, R.S. et al., 1998; Carlson, J.H. et al., 2005), the related murine *C*. *muridarum* strain MoPn (Read, T.D. et al., 2000) as well as that of *C. psittaci* (Read, T.D. et al., 2003) have been determined.

The problem underlying the present invention was to provide means for the development of pharmaceutical compositions such as vaccines against infections caused by *Chlamydia.* More particularly, the problem was to provide an efficient, relevant and comprehensive set of nucleic acid molecules or antigens, or fragments or variants thereof, from *Chlamydia* that can be used for the preparation of said pharmaceutical compositions. A still further problem was to provide methods and means for producing an antigen, a fragment or variant thereof. Yet another problem was to provide pharmaceutical compositions comprising said nucleic acids or said antigens. A still further problem of the invention was to provide antibodies, pharmaceutical compositions comprising said antibodies, methods for the production of said antibodies and the use of said antibodies for the preparation of a pharmaceutical preparation. Furthermore, the object of the present invention was to provide methods for identifying an antagonist capable of binding an antigen, or a fragment or variant thereof, as well as to provide methods for identifying an antagonist capable of reducing or inhibiting the interaction activity of such an antigen to its interaction partner. A further problem of the present invention was to provide methods for diagnosing an infection with a *Chlamydia* organism. Still another problem underlying the invention was to provide methods for treating *Chlamydia* infections, and to provide methods for immunizing an animal or human.

The problem underlying the present invention is solved in one aspect by an isolated nucleic acid molecule encoding an antigen or a fragment thereof, comprising a nucleic acid sequence, which is selected from the group consisting of:
a) a nucleic acid molecule having at least 70% sequence identity to a nucleic acid molecule having a nucleotide sequence selected from the group consisting of Seq ID Nos 1 to 98,
b) a nucleic acid molecule which is complementary to the nucleic acid molecule of a),
c) a nucleic acid molecule comprising at least 15 sequential bases of the nucleic acid molecule of a) or b),
d) a nucleic acid molecule which anneals under stringent hybridisation conditions to the nucleic acid molecule of a), b), or c), and
e) a nucleic acid molecule which, but for the degeneracy of the genetic code, would hybridise to the nucleic acid molecule defined in a), b), c), or d).

In an embodiment of the invention the sequence identity to Seq ID Nos 1 to 98 is at least 80%, more preferably at least 90%, still more preferably at least 95%, 96%, 97%, 98%, or 99%, or most preferably 100%.

In an embodiment the nucleic acid is DNA.

In an alternative embodiment the nucleic acid is RNA.

In still another embodiment the nucleic acid molecule is isolated from a genomic DNA, preferably from a *Chlamydia* species, more preferably from *C*. *pneumoniae, C. trachomatis, C. psittaci, C. pecorum, C. muridarum,* or *C*. *suis,* and most preferably from *C*. *pneumoniae, C. trachomatis* or *C*. *psittaci.*

In an embodiment of the invention the encoded antigen fragment is an active fragment or an active variant thereof.

In an embodiment the nucleic acid encodes an antigen or fragment thereof, which comprises or consists of a polypeptide or peptide fragment from *C*. *pneumoniae, C. trachomatis, C. psittaci, C. pecorum, C. muridarum,* or *C*. *suis,* and more preferably from *C*. *pneumoniae, C. trachomatis* or *C*. *psittaci.*

The problem underlying the present invention is further solved by a vector comprising a nucleic acid molecule as described above.

In an embodiment the vector is adapted for recombinant expression of the antigen, or fragment thereof, encoded by the nucleic acid molecule as defined above.

The present invention also relates to a host cell comprising the vector as defined above.

The problem underlying the present invention is solved in a further aspect by an antigen that is immunologically reactive with sera from a human having a *Chlamydia* infection, or an uninfected healthy human who was previously infected with *Chlamydia,* wherein the antigen comprises an isolated polypeptide or an active fragment or an active variant thereof from *Chlamydia,* preferably from *C*. *pneumoniae, C. trachomatis, C. psittaci, C. pecorum, C. muridarum,* or *C*. *suis,* and more preferably from *C*. *pneumoniae, C. trachomatis* or *C*. *psittaci.*

The term "uninfected healthy human" as used herein comprises those individuals who have or had multiple encounters with the pathogen, which may result in colonization, but which either do not result in any symptoms, or which result in mild diseases. Said term and the rationale of selecting sera of uninfected healthy humans for antigen identification is further defined in Nagy, E. et al. (2003).

Another aspect of the present invention relates to an antigen, comprising or consisting of an isolated polypeptide selected from the group consisting of Seq ID Nos 99 to 196, or an active fragment or an active variant thereof.

In an embodiment of the invention said polypeptide is encoded by a nucleic acid molecule as defined above.

In another embodiment the active fragment of the antigen consists of at least 50%, especially at least 60%, preferably at least 70%, more preferably at least 80%, still more preferably at least 90%, even more preferably at least 95%, 96%, 97% or 98%, most preferably 99% of said polypeptide, especially of a polypeptide as defined by any of the Seq ID Nos 99 to 196.

In an embodiment the active variant of the antigen has at least 50%, especially at least 60%, preferably at least 70%, more preferably at least 80%, still more preferably at least 90%, even more preferably at least 95%, 96%, 97% or 98%, most preferably 99% sequence identity to said polypeptide, especially to a polypeptide as defined by any of the Seq ID Nos 99 to 196.

In one embodiment of the present invention the active fragment of the antigen comprises or consists of amino acids 2-872 of Seq ID No 123, amino acids 2-812 of Seq ID No 124, amino acids 2-514 or 516 to 1090 of Seq ID No 125, amino acids 2-252 of Seq ID No 127, amino acids 2-324 of Seq ID No 128, amino acids 2-235 of Seq ID No 130, amino acids 2-792 of Seq ID No 131, amino acids 2-252 of Seq ID No 132, amino acids 8-245 of Seq ID No 135, amino acids 2-393 of Seq ID No 138, amino acids 2-237 of Seq ID No 139, amino acids 16-620 of Seq ID No 140, amino acids 2-287 of Seq ID No 155, amino acids 27-231 of Seq ID No 156, amino acids 2-550 of Seq ID No 157, amino acids 2-644 of Seq ID No 165, amino acids 2-343 of Seq ID No 168, amino acids 2-325 of Seq ID No 170, amino acids 2-458 of Seq ID No 171, or amino acids 31-379 of Seq ID No 172.

The fragments as listed above are further defined in separate Seq ID Nos; i.e. amino acids 2-872 of Seq ID No 123 are identical to Seq ID No 99; amino acids amino acids 2-812 of Seq ID No 124 are identical to Seq ID No 100, amino acids 2-514 of Seq ID No 125 are identical to Seq ID No 101, amino acids 516 to 1090 of Seq ID No 125 are identical to Seq ID No 102, amino acids 2-252 of Seq ID No 127 are identical to Seq ID No 104, amino acids 2-324 of Seq ID No 128 are identical to Seq ID No 105, amino acids 2-235 of Seq ID No 130 are identical to Seq ID No 106, amino acids 2-792 of Seq ID No 131 are identical to Seq ID No 107, amino acids 2-252 of Seq ID No 132 are identical to Seq ID No 108, amino acids 8-245 of Seq ID No 135 are identical to Seq ID No 109, amino acids 2-393 of Seq ID No 138 are identical to Seq ID No110, amino acids 2-237 of Seq ID No 139 are identical to Seq ID No 111, amino acids 16-620 of Seq ID No 140 are identical to Seq ID No 112, amino acids 2-287 of Seq ID No 155 are identical to Seq ID No 114, amino acids 27-231 of Seq ID No 156 are identical to Seq ID No115, amino acids 2-550 of Seq ID No 157 are identical to Seq ID No 116, amino acids 2-644 of Seq ID No 165 are identical to Seq ID No 117, amino acids 2-343 of Seq ID No 168 are identical to Seq ID No 118, amino acids 2-325 of Seq ID No 170 are identical to Seq ID No 120, amino acids 2-458 of Seq ID No 171 are identical to Seq ID No 121, and amino acids 31-379 of Seq ID No 172 are identical to Seq ID No 122.

As three of the identified antigen amino acid sequences were identical to the entire open reading frame (ORF), Seq ID No 103 is identical to Seq ID No 126; Seq ID No 113 is identical to Seq ID No 151; Seq ID No 119 is identical to Seq ID No 169.

In another embodiment, the active variant of the antigen has at least 50%, especially at least 60%, preferably at least 70%, more preferably at least 80%, still more preferably at least 90%, even more preferably at least 95%, 96%, 97% or 98%, most preferably 99% sequence identity to amino acids 2-872 of Seq ID No 123, amino acids 2-812 of Seq ID No 124, amino acids 2-514 or 516 to 1090 of Seq ID No 125, amino acids 2-252 of Seq ID No 127, amino acids 2-324 of Seq ID No 128, amino acids 2-235 of Seq ID No 130, amino acids 2-792 of Seq ID No 131, amino acids 2-252 of Seq ID No 132, amino acids 8-245 of Seq ID No 135, amino acids 2-393 of Seq ID No 138, amino acids 2-237 of Seq ID No 139, amino acids 16-620 of Seq ID No 140, amino acids 2-287 of Seq ID No 155, amino acids 27-231 of Seq ID No 156, amino acids 2-550 of Seq ID No 157, amino acids 2-644 of Seq ID No 165, amino acids 2-343 of Seq ID No 168, amino acids 2-325 of Seq ID No 170, amino acids 2-458 of Seq ID No 171, or amino acids 31-379 of Seq ID No 172.

In still another embodiment, the antigen is further defined by
a) 1 to 570 additional amino acid residue(s), preferably 1 to 500, 1 to 400, 1 to 300, 1 to 250, 1 to 200, or 1 to 150, even more preferably 1 to 100, still more preferably 1 to 50, most preferably 1, 2, 3, 4, 5, 10, 20, 30 or 40 additional amino acid residue(s) to the active fragment of the antigen comprising or consisting of amino acids 2 to 514 of Seq ID No 125; or
b) 1 to 510 additional amino acid residue(s), preferably 1 to 400, 1 to 300, 1 to 250, 1 to 200, or 1 to 150, even more preferably 1 to 100, still more preferably 1 to 50, most preferably 1, 2, 3, 4, 5, 10, 20, 30 or 40 additional amino acid residue(s) to the active fragment of the antigen comprising or consisting of amino acids 516 to 1090 of Seq ID No 125; or
c) 1 to 25 additional amino acid residue(s), preferably 1, 2, 3, 4, 5, 10, 15, or 20 additional amino acid residue(s) to the active fragment of the antigen comprising or consisting of amino acids 31-379 of Seq ID No 172; or
d) 1 to 20 additional amino acid residue(s), preferably 1, 2, 3, 4, 5, 10, or 15 additional amino acid residue(s) to the active fragment of the antigen comprising or consisting of amino acids 27-231 of Seq ID No 156; or
e) 1 to 10 additional amino acid residue(s), preferably 1, 2, 3, 4, 5, 6, 7, 8, or 9 additional amino acid residue(s) to the active fragment of the antigen comprising or consisting of amino acids 16 to 620 of Seq ID No 140; or
f) 1 to 5 additional amino acid residue(s), preferably 1, 2, 3, or 4 additional amino acid residue(s) to the active fragment of the antigen comprising or consisting of amino acids 8 to 245 of Seq ID No 135.

The additional amino acid residue(s) may be homologous to the antigen as defined above. Homologous refers to any amino acid residue(s) which is/are identical or similar to the amino acid sequence of the *Chlamydia* antigen from which the fragment is derived.

Alternatively or additionally, the polypeptide may comprise or consist of the antigen, optionally the additional sequence as defined above and at least one amino acid residue heterologous to the antigen.

In an embodiment of the invention, the antigen further comprises or consists of at least one amino acid residue heterologous to the antigen, preferably an amino acid sequence of a marker protein.

The additional sequence or amino acid residue(s) as defined above consist(s) of (an) amino acid residue(s), which may be any amino acid, which may be either an L-and/or a D-amino acid, naturally occurring and otherwise. Preferably the amino acid is any naturally occurring amino acid such as alanine, cysteine, aspartic acid, glutamic acid, phenylalanine, glycine, histidine, isoleucine, lysine, leucine, methionine, asparagine, proline, glutamine, arginine, serine, threonine, valine, tryptophan or tyrosine.

However, the amino acid may also be a modified or unusual amino acid. Examples of those are 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxylysine, allo-hydroxylysine, 3-hydroxyproloine, 4-hydroxyproloine, isodesmosine, allo-isoleucine, N-methylglycine, N-methylisoleucine, 6-N-methyllysine, N-methylvaline, norvaline, norleucine or ornithine. Additionally, the amino acid may be subject to modifications such as posttranslational modifications. Examples of modifications include acetylation, amidation, blocking, formylation, gamma-carboxyglutamic acid hydroxylation, glycosilation, methylation, phosphorylation and sulfatation. If more than one additional or heterologous amino acid residue is present in the peptide, the amino acid residues may be the same or different from one another.

The feature "heterologous amino acid" or "amino acid heterologous to the antigen" refers to any amino acid which is different from that amino acid located adjacent to the antigen in any naturally occurring protein of *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.* Therefore, the protein of the invention encompassing at least one heterologous amino acid refers to a protein which is different from any naturally occurring protein of *Chlamydiae* or fragments thereof, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

In one embodiment, the additional amino acid residue(s) is/are flanking the antigen N-terminally, C-terminally or N- and C-terminally.

In another embodiment, the additional amino acid residue(s) is/are flanking the antigen defined by
a) amino acids 2 to 514 of Seq ID No 125 or the variant derived thereof C-terminally, or
b) amino acids 516 to 1090 of Seq ID No 125, 8 to 245 of Seq ID No 135, 16 to 620 of Seq ID No 140, 27-231 of Seq ID No 156, or 31-379 of Seq ID No 172, or the variant derived thereof N-terminally.

In another embodiment, the antigen further comprises or consists of either a leader or a secretory sequence, a sequence employed for purification, or a proprotein sequence.

The problem underlying the present invention is solved in another aspect by an antigen, whereby the antigen comprises a core amino acid sequence as indicated in column "Predicted immunogenic aa" or "Predicted class II-restricted T cell epitope/regions" or "Location of identified immunogenic region (aa)" of Table 1, whereby more preferably the core amino acid sequence is selected from the group consisting of:
amino acids 20-28, 32-40, 45-56, 85-96, 102-118, 120-130, 132-154, 158-172, 174-181, 193-202, 208-221, 228-241, 250-259, 274-283, 285-291, 296-309, 317-324, 328-339, 348-354, 383-391, 402-412, 418-428, 443-455, 467-482, 484-490, 519-525, 545-557, 568-582, 601-606, 613-638, 646-656, 661-676, 687-694, 706-724, 741-766, 776-782, 789-798, 812-822 and 847-869 of Seq ID No 123; amino acids 28-37, 44-88, 95-102, 107-115, 142-150, 212-226, 228-233, 235-245, 262-269, 278-311, 319-338, 343-354, 372-405, 409-418, 426-435, 439-445, 447-462, 471-478, 481-490, 502-514, 522-529, 539-565, 568-589, 595-605, 608-622, 645-651, 656-662, 665-670, 679-685, 696-715, 731-744, 775-781 and 789-809 of Seq ID No 124; amino acids 6-14, 21-44, 52-70, 74-93, 106-132, 136-157, 162-168, 174-184, 186-223, 232-248, 250-262, 264-270, 278-284, 303-324, 342-348, 352-357, 409-415, 423-442, 444-454, 458-478, 480-494, 499-509, 514-521, 529-538, 541-559, 561-575, 582-605, 611-619, 630-638, 647-665, 672-683, 696-719, 727-739, 748-791, 800-806, 815-822, 848-855, 860-868, 876-884, 891-896, 898-950, 956-962, 971-980, 995-1008, 1036-1043, 1045-1061 and 1069-1074 of Seq ID No 125; amino acids 7-13, 21-27, 33-57, 63-72, 74-80, 82-88, 94-105, 107-119, 121-132, 134-152, 162-199, 201-225, 229-237, 239-246, 252-258, 261-267, 277-298, 300-325, 340-366, 373-383 and 391-401 of Seq ID No 126; amino acids 4-17, 22-32, 45-58, 60-70, 72-86, 88-98, 100-108, 112-121, 128-138, 148-162, 167-176, 184-198, 216-231 and 241-248 of Seq ID No 127; amino acids 6-23, 32-52, 60-77, 84-90, 92-107, 117-163, 165-239, 249-254, 273-280 and 289-308 of Seq ID No 128; amino acids 5-49, 52-59, 65-95, 102-108, 115-123, 126-139, 142-153, 180-186, 193-204, 206-214, 225-247, 270-278, 280-297, 303-319, 321-337, 344-358, 363-371, 378-389, 399-406 and 412-419 of Seq ID No 129; amino acids 4-30, 32-38, 43-50, 55-62, 64-72, 76-83, 97-119, 125-132, 139-156, 158-172, 204-210 and 221-227 of Seq ID No 130; amino acids 4-16, 21-31, 39-57, 61-67, 73-99, 105-111, 119-128, 135-147, 150-159, 163-196, 213-246, 248-255, 257-284, 286-293, 295-317, 333-360, 369-387, 389-413, 415-422, 424-438, 443-454, 461-467, 478-490, 508-520, 527-532, 534-551, 557-564, 566-576, 578-588, 596-684, 687-711, 713-724, 729-741, 749-758 and 770-782 of Seq ID No 131; amino acids 4-13, 38-59, 80-90, 95-102, 108-123, 130-183, 190-223 and 239-249 of Seq ID No 132; amino acids 4-13, 27-38, 43-48, 53-74, 82-100, 104-110, 112-137, 147-154, 169-179, 184-192, 196-223, 234-249 and 252-262 of Seq ID No 133; amino acids 7-36, 45-50, 52-92, 103-125, 129-138, 140-154, 163-176, 178-188, 194-203, 207-222, 230-239, 244-297, 299-314, 324-332, 335-343, 361-368, 370-404, 409-416, 426-443, 451-456, 459-469, 477-493, 496-513, 515-522, 529-535, 540-546, 549-561, 578-586, 588-596, 609-628, 636-645, 659-665, 668-687, 708-724, 726-739, 761-770, 788-795, 818-825, 842-869, 872-883, 887-897, 905-972, 976-984, 988-998, 1015-1030, 1033-1051, 1076-1082, 1084-1107, 1141-1151, 1167-1180, 1184-1189, 1193-1205, 1221-1228, 1235-1254, 1258-1305, 1307-1314, 1326-1335, 1350-1369 and 1371-1395 of Seq ID No 134; amino acids 4-14, 22-32, 48-61, 63-71, 79-111, 119-145, 147-167, 179-186, 188-201, 217-223 and 227-235 of Seq ID No 135; amino acids 14-29, 31-38, 50-56, 68-74, 81-89, 108-118, 125-138, 144-151, 167-175, 215-228, 252-262, 272-279, 281-288, 322-344, 358-374, 386-395, 406-418, 426-441, 455-465, 485-490, 513-519, 521-528, 538-543, 548-555, 558-574, 577-583, 585-596, 608-624, 636-643, 665-672, 680-686, 699-707, 715-730, 747-755, 775-786, 812-818, 824-841, 856-868, 892-899, 930-946, 963-972, 979-984, 1008-1015, 1032-1038, 1044-1050, 1052-1060, 1092-1110, 1143-1148, 1157-1181, 1184-1196, 1215-1222, 1229-1244, 1248-1255, 1267-1272, 1282-1295, 1300-1311, 1323-1329, 1390-1396, 1399-1407, 1423-1431, 1452-1458, 1478-1484, 1488-1501, 1504-1511, 1521-1551, 1573-1599, 1622-1641, 1644-1663, 1669-1675, 1681-1688 and 1692-1711 of Seq ID No 136; amino acids 7-33, 39-47, 53-66, 72-104, 117-126, 132-138, 146-154, 160-167, 182-188, 199-213, 259-270, 283-289, 291-308, 314-326, 328-334, 352-358, 364-370, 372-379, 407-417, 424-445, 461-475, 477-485, 488-497, 510-516, 539-553, 567-581, 589-595, 668-687, 697-707, 709-731, 733-752, 756-765, 772-780, 797-827, 845-852, 860-876, 884-891, 896-903 and 915-922 of Seq ID No 137; amino acids 17-23, 26-34, 36-42, 45-51, 53-63, 69-79, 89-97, 106-119, 146-161, 175-184, 186-201, 217-244, 252-265, 268-278, 280-294, 304-315, 324-340, 343-351 and 353-374 of Seq ID No 138; amino acids 17-27, 34-40, 47-65, 68-112, 114-126, 133-148, 150-157, 163-187, 190-204 and 207-234 of Seq ID No 139; amino acids 4-16, 20-42, 60-66, 75-108, 112-117, 131-138, 148-153, 157-174, 201-206, 213-228, 235-243, 246-259, 266-271, 295-301, 311-316, 331-337, 344-353, 364-377, 383-389, 405-412, 452-458, 470-487, 491-505, 508-517, 540-555, 557-565, 576-587, 593-603 and 612-617 of Seq ID No 140; amino acids 7-14, 18-25, 30-35, 43-48, 82-98, 102-111, 139-149, 165-185, 189-197, 203-219, 231-236, 268-277, 291-301, 303-311, 315-330, 332-340, 344-354, 365-374, 381-400, 404-409, 419-426, 431-441, 457-467, 469-475, 481-490, 556-562, 605-613 and 649-655 of Seq ID No 141; amino acids 4-15, 23-32, 48-67, 76-83, 86-94, 97-105, 107-120, 127-136, 180-185, 206-212, 251-257, 313-322, 325-331, 340-353, 374-381, 397-403, 407-413, 420-432, 450-457, 471-483, 492-503, 505-515, 519-530, 539-548, 558-579, 602-611, 625-633, 636-643, 650-656, 658-666, 677-683, 686-694, 707-714, 716-730, 745-763, 765-786, 791-830, 841-857, 864-870, 873-885, 887-895, 912-919, 921-938 and 940-949 of Seq ID No 142; amino acids 4-32, 34-61, 65-75, 81-91, 99-106, 121-132, 150-156, 162-170, 174-182, 189-196, 201-214, 230-236, 239-246, 276-285, 287-295, 301-310, 335-354, 366-383, 390-398, 408-433, 439-445, 466-478, 499-519, 525-544, 550-555, 572-580, 589-603, 624-633, 648-655, 659-671 and 677-682 of Seq ID No 143; amino acids 5-29, 42-60, 72-77, 114-143, 151-157, 162-174, 177-182, 185-193, 233-251, 266-274, 279-298, 301-308, 321-335, 338-365, 378-386, 388-396, 401-407, 412-434, 439-459, 475-480 and 499-505 of Seq ID No 144; amino acids 4-26, 28-34, 57-64, 75-81, 95-100, 126-141, 144-150, 158-163, 200-210, 216-221, 243-249, 291-309, 326-339, 363-369, 380-389, 396-403, 410-422, 436-455, 485-498, 516-525, 541-576, 608-619, 635-641, 664-674, 683-692, 697-703, 707-723, 737-749, 760-765, 775-808, 827-839, 851-859, 869-875, 877-884, 886-906 and 910-927 of Seq ID No 145; amino acids 4-29, 43-48, 56-66, 70-82, 93-102, 107-113, 127-152, 159-164, 179-184, 201-207, 216-224, 231-239, 244-250, 262-269, 291-305, 307-313, 333-340, 368-374, 379-387, 414-422, 432-438, 440-461, 487-498, 518-524, 539-548, 557-567, 610-615, 644-651, 667-675, 681-693, 709-718, 721-726, 734-758, 780-817, 833-843, 856-865, 872-879, 893-900, 902-908 and 913-921 of Seq ID No 146; amino acids 4-22, 58-73, 85-106, 115-129, 139-145, 170-181, 185-191, 199-204, 242-249, 270-276, 293-303, 332-339, 353-359, 387-392, 424-439, 459-472, 532-538, 555-563, 593-604, 631-636, 654-659, 679-686, 689-705, 718-733, 740-751, 756-771, 789-796, 798-805, 834-842, 851-856, 885-894, 922-930, 934-947, 953-960, 963-975, 983-988, 1008-1019, 1026-1038, 1041-1083, 1086-1098, 1121-1157, 1159-1166, 1169-1182, 1192-1207, 1209-1216 and 1232-1260 of Seq ID No 147; amino acids 5-12, 38-50, 70-91, 109-157, 169-182, 185-191, 205-229, 234-251, 262-279, 287-314, 321-331, 335-351, 356-364, 366-373, 377-383, 386-394, 396-415, 417-429, 437-448, 456-467, 490-496, 504-512, 518-530, 533-543, 545-555, 577-586, 603-610, 617-628, 635-662, 670-682, 689-699, 713-719, 724-741, 745-769, 779-784, 790-808, 816-850, 866-879 and 894-905 of Seq ID No 148; amino acids 6-23, 26-31, 35-99, 108-120, 134-146, 154-167, 171-178, 183-189, 194-203, 206-212, 241-250, 252-270, 278-298 and 302-338 of Seq ID No 149; amino acids 21-26, 31-37, 42-92, 100-127, 131-149, 182-188, 190-214, 216-231, 249-257, 264-289, 305-326, 332-342, 348-357, 360-368, 379-385, 387-393, 396-410, 421-427 and 435-452 of Seq ID No 150; amino acids 4-9, 16-21, 23-29, 34-41, 46-51, 58-73, 104-113, 115-122, 125-134 and 141-168 of Seq ID No 151; amino acids 4-11, 16-34, 82-98, 103-114, 132-147, 151-158, 164-182, 215-221, 236-244, 253-259, 261-267, 272-283 and 285-307 of Seq ID No 152; amino acids 4-18, 20-34, 50-56, 64-69, 100-109, 114-131, 142-150 and 159-165 of Seq ID No 153; amino acids 10-23, 36-48, 55-66, 68-75, 95-109, 116-151, 156-169, 173-192, 199-205, 208-213, 219-226, 232-238, 246-256, 276-284, 295-314, 332-343, 358-373, 381-393, 406-426, 431-438, 454-469, 481-492, 519-533, 536-551, 556-561, 567-572, 594-608, 612-619, 629-638, 665-681, 685-697, 710-727, 759-777 and 792-798 of Seq ID No 154; amino acids 7-13, 29-39, 54-60, 62-68, 99-104, 126-138, 140-153, 159-190, 204-210, 217-223, 234-240, 243-262 and 270-278 of Seq ID No 155; amino acids 4-22, 50-58, 71-77, 83-93, 108-127, 143-153, 158-191, 205-213 and 221-228 of Seq ID No 156; amino acids 4-22, 26-35, 38-61, 63-76, 85-99, 101-109, 145-151, 153-160, 162-172, 180-187, 194-202, 208-215, 223-231, 235-247, 253-273, 278-295, 302-323, 337-358, 366-404, 420-434, 436-450, 454-468, 474-485, 491-514, 524-533 and 539-545 of Seq ID No 157; amino acids 7-30, 35-45, 53-70, 78-105, 120-127, 129-143, 151-158, 162-193, 208-216, 219-249, 252-258, 277-308, 311-321, 323-347, 359-364, 375-385, 389-395, 401-409, 414-420, 436-449, 467-474, 491-507 and 513-519 of Seq ID No 158; amino acids 12-22, 27-35, 38-53, 60-81, 87-94, 96-113, 115-136, 144-172, 189-196, 208-237, 239-251, 258-265, 272-292, 296-305, 319-331, 351-371, 378-389, 404-425, 428-436, 443-448, 458-477, 501-513, 535-541, 547-554, 557-565, 574-584 and 590-598 of Seq ID No 159; amino acids 10-30, 36-46, 56-67, 78-105, 116-124, 126-142, 146-160, 166-172, 186-198, 207-224, 249-256, 278-289, 297-304, 307-315, 318-350, 352-367, 373-388, 395-413, 427-438, 444-455, 457-472, 482-490, 495-501, 512-522, 529-535, 537-552, 572-580 and 589-620 of Seq ID No 160; amino acids 4-14, 16-22, 26-34, 40-50, 56-65, 75-86, 88-101, 105-117, 126-132, 134-142, 145-170, 172-191, 193-205, 214-223, 227-235, 247-290, 294-319, 332-339, 345-355, 363-369, 394-403, 407-412, 429-466, 468-475, 494-510, 515-522, 528-534, 539-545, 556-562, 574-589, 596-604, 609-627, 637-647, 663-671, 679-689, 694-702, 708-715, 718-727, 738-744, 750-758, 774-808, 815-836, 840-883, 890-906, 910-929, 933-943, 951-967, 972-986, 988-1027, 1032-1041, 1045-1058, 1069-1079, 1081-1102, 1117-1123, 1125-1143, 1145-1168, 1173-1178, 1186-1199, 1202-1209, 1214-1220, 1225-1242, 1244-1251, 1257-1266, 1269-1275, 1295-1313, 1339-1345, 1364-1377, 1390-1410, 1412-1431, 1438-1459, 1472-1499, 1511-1537, 1543-1549, 1553-1560, 1566-1579, 1585-1609, 1621-1632, 1636-1665, 1669-1718, 1721-1730, 1734-1761, 1763-1787, 1796-1802 and 1805-1823 of Seq ID No 161; amino acids 11-21, 27-48, 58-69, 73-89, 100-110, 142-152, 165-178, 187-196, 203-216, 238-246, 254-262, 278-288, 294-299, 308-315, 334-340, 366-374, 376-386, 392-401, 406-412, 444-459, 463-469, 474-479, 484-490, 500-517, 524-534, 542-552, 566-578, 586-594, 603-628, 643-664, 668-692, 699-706, 714-726, 729-735, 747-755, 765-771, 778-785, 804-814, 822-837, 848-855, 860-886, 898-909, 912-919, 925-933, 936-942, 945-957, 966-975, 978-997, 1015-1035, 1045-1055, 1066-1076, 1082-1107, 1141-1179, 1193-1214, 1240-1246 and 1252-1259 of Seq ID No 162; amino acids 7-15, 17-28, 34-39, 83-96, 100-107, 119-149, 152-158, 168-174, 176-213, 220-231, 237-262, 264-273, 280-286, 318-327, 338-348, 358-369, 371-386, 405-410, 413-421, 427-443, 445-462, 467-473, 483-490, 498-503, 513-527, 529-537, 540-546 and 581-590 of Seq ID No 163; amino acids 4-16, 18-24, 57-69, 121-140, 151-157, 159-166, 175-183, 206-212, 261-272, 276-283, 285-305, 321-327, 343-350, 352-367, 383-389, 410-416, 424-435, 448-455, 457-464, 469-478, 493-499, 509-528, 543-555, 564-575, 584-594, 608-631, 635-658, 667-676, 683-689, 692-698, 708-731, 737-750, 757-774, 778-784, 798-805 and 809-815 of Seq ID No 164; amino acids 4-30, 35-47, 49-67, 73-86, 114-120, 123-132, 140-160, 168-176, 195-201, 211-218, 220-256, 261-285, 313-328, 330-349, 367-373, 375-385, 388-422, 449-488, 509-547, 553-569, 582-597 and 599-606 of Seq ID No 165; amino acids 6-11, 19-44, 62-72, 78-87, 101-108, 128-142, 149-185, 191-198, 204-222, 224-241, 284-294, 296-301, 304-311 and 327-356 of Seq ID No 166; amino acids 6-23, 34-59, 66-75, 79-96, 106-117, 119-130, 133-143, 164-172, 179-193, 195-207, 215-241, 247-261, 285-293, 300-307, 317-329, 346-352, 357-362, 368-384, 397-408, 416-424, 432-453, 461-485, 493-501, 518-527, 533-544, 552-562, 574-588 and 604-613 of Seq ID No 167; amino acids 4-13, 15-21, 32-41, 43-54, 56-84, 86-93, 95-113, 120-126, 130-165, 175-189, 195-202, 210-222, 244-250, 253-270, 273-280, 283-291, 301-313 and 315-339 of Seq ID No 168; amino acids 6-25, 28-87, 93-100, 105-112, 118-133, 138-145, 157-165, 167-184, 198-209, 220-237, 245-252, 254-262, 265-277, 288-299, 310-326 and 335-362 of Seq ID No 169; amino acids 8-19, 21-27, 29-36, 49-67, 84-101, 103-122, 128-157, 159-166, 168-177, 181-187, 192-205, 207-217, 222-251, 255-266, 272-280, 284-297 and 306-322 of Seq ID No 170; amino acids 9-25, 33-41, 48-73, 78-86, 89-97, 100-106, 117-135, 141-147, 164-181, 189-195, 200-215, 226-251, 267-283, 289-300, 318-324, 332-340, 355-362, 374-382, 389-394, 397-402, 422-448 and 450-455 of Seq ID No 171; amino acids 4-31, 36-45, 52-76, 78-118, 120-135, 145-164, 167-228, 241-250, 269-279, 283-312, 315-333, 337-362 and 364-376 of Seq ID No 172; amino acids 24-34, 39-50, 52-60, 70-93, 96-105, 124-130, 146-168, 173-183, 195-213, 217-231, 248-259, 267-285, 304-312, 314-322, 334-350, 365-388, 391-403, 408-416, 418-443, 450-477, 496-503, 516-522, 544-550, 565-592, 594-600, 614-635, 639-670, 691-705, 707-714, 733-744, 751-764, 777-787, 797-829, 848-856, 864-887, 901-913, 919-938, 941-950, 955-961, 966-990, 1004-1023, 1027-1033, 1040-1047, 1053-1058, 1063-1073, 1079-1090, 1104-1109, 1112-1124, 1128-1134, 1138-1145 and 1149-1155 of Seq ID No 173; amino acids 4-28, 59-66, 93-98, 128-134, 140-164, 166-186, 198-208, 211-227, 233-240, 246-258, 264-277, 282-291, 297-302, 318-349, 362-370, 372-386, 393-417, 420-427, 437-462, 464-473, 481-503, 508-514, 519-533, 543-549, 554-568, 584-592, 636-647, 655-662, 680-688, 702-707, 716-723, 726-731 and 738-746 of Seq ID No 174; amino acids 16-31, 78-84, 131-140, 145-155, 168-174, 176-193, 219-225, 253-292, 303-368, 376-406, 420-436, 444-453 and 468-485 of Seq ID No 175; amino acids 4-14 of Seq ID No 176; amino acids 18-35, 47-55, 65-82, 106-112 and 117-140 of Seq ID No 177; amino acids 15-38 of Seq ID No 178; amino acids 31-48 and 50-55 of Seq ID No 179; amino acids 6-14 of Seq ID No 181; amino acids 4-10 of Seq ID No 182; amino acids 9-18 and 21-30 of Seq ID No 183; amino acids 4-13, 31-39, 56-74, 76-83 and 85-91 of Seq ID No 184; amino acids 8-32, 38-49 and 57-66 of Seq ID No 185; amino acids 4-19 and 25-31 of Seq ID No 186; amino acids 1-9, 17-25, 38-49 and 52-58 of Seq ID No 187; amino acids 4-16 and 20-27 of Seq ID No 188; amino acids 20-26 and 32-41 of Seq ID No 189; amino acids 4-15, 21-41, 45-55, 63-92 and 105-123 of Seq ID No 190; amino acids 4-17 and 19-25 of Seq ID No 192; amino acids 5-31 and 33-50 of Seq ID No 193; amino acids 10-17 of Seq ID No 194; amino acids 4-21 and 52-69 of Seq ID No 195; amino acids 11-29 of Seq ID No 196; amino acids 48-59, 267-277, 452-467, 497-512 and 659-673 of Seq ID No 123; amino acids 315-326, 428-442, 741-756 and 789-808 of Seq ID No 124; amino acids 24-38, 108-123, 599-620, 775-788 and 939-949 of Seq ID No 125; amino acids 17-30, 40-59, 71-83, 93-113, 216-231, 260-277, 371-385 and 392-404 of Seq ID No 126; amino acids 3-37, 42-52, 58-67, 72-83, 94-114, 123-137, 142-160, 197-209 and 218-250 of Seq ID No 127; amino acids 7-22, 24-35, 78-93, 97-110, 124-136, 162-176, 193-209, 213-225 and 230-239 of Seq ID No 128; amino acids 12-27, 125-138, 190-202, 260-277 and 394-417 of Seq ID No 129; amino acids 9-21, 26-37, 49-63, 84-97, 107-120, 127-137, 142-154, 197-208 and 223-235 of Seq ID No 130; amino acids 526-543, 609-624, 654-673 and 682-699 of Seq ID No 131; amino acids 7-25, 45-63, 91-122, 173-183 and 216-227 of Seq ID No 132; amino acids 1-11, 30-43, 70-86, 142-150, 192-207 and 217-229 of Seq ID No 133; amino acids 428-440, 561-572, 859-870, 919-930 and 1055-1067 of Seq ID No 134; amino acids 12-25, 44-60, 72-88, 97-117, 145-158, 178-201 and 211-221 of Seq ID No 135; amino acids 6-26, 1430-1441 and 1648-1666 of Seq ID No 136; amino acids 84-94, 113-125, 141-152, 331-342, 716-728 and 743-763 of Seq ID No 137; amino acids 17-27, 110-120, 166-179, 220-234, 251-262, 282-294, 322-339, 354-366 and 379-387 of Seq ID No 138; amino acids 5-25, 34-51, 115-125, 140-152, 166-181, 191-201 and 215-233 of Seq ID No 139; amino acids 20-31, 159-177, 235-244, 362-371 and 444-454 of Seq ID No 140; amino acids 8-23, 122-130, 289-300, 289-300, 336-345, 388-398 and 590-614 of Seq ID No 141; amino acids 84-97, 141-152, 181-189, 318-328, 439-447, 498-512, 656-668 and 819-828 of Seq ID No 142; amino acids 3-17, 53-72, 187-199, 452-465, 468-483 and 592-601 of Seq ID No 143; amino acids 7-21, 74-84, 91-101, 119-130, 363-372 and 387-396 of Seq ID No 144; amino acids 2-13, 542-552, 627-637, 652-660 and 798-807 of Seq ID No 145; amino acids 7-23, 173-185, 530-540 and 726-734 of Seq ID No 146; amino acids 10-24, 1065-1079, 1101-1110 and 1141-1156 of Seq ID No 147; amino acids 80-90, 212-221, 233-243, 286-296, 356-367, 531-541 and 859-869 of Seq ID No 148; amino acids 3-14, 31-45, 51-69, 70-91, 152-165, 257-268 and 299-311 of Seq ID No 149; amino acids 42-51, 62-75, 79-88, 264-276, 336-353 and 398-406 of Seq ID No 150; amino acids 17-32, 44-70, 89-103, 122-133 and 150-165 of Seq ID No 151; amino acids 4-13, 23-40, 61-74, 169-191, 203-212 and 289-297 of Seq ID No 152; amino acids 3-20, 48-59, 75-83 and 104-122 of Seq ID No 153; amino acids 191-200, 276-285, 307-321, 523-535, 568-577, 721-729 and 772-780 of Seq ID No 154; amino acids 6-17, 79-88, 98-107, 125-137, 159-167, 173-191 and 267-276 of Seq ID No 155; amino acids 6-27, 36-45, 147-157 and 172-197 of Seq ID No 156; amino acids 175-187, 242-254, 377-389, 450-467, 493-503 and 528-538 of Seq ID No 157; amino acids 6-27, 94-106, 135-145, 255-265 and 289-311 of Seq ID No 158; amino acids 36-49, 59-70, 100-113, 121-130, 147-155 and 351-360 of Seq ID No 159; amino acids 17-28, 291-300, 340-352, 362-371 and 387-396 of Seq ID No 160; amino acids 425-443 of Seq ID No 161; amino acids 685-693, 699-709, 1065-1075 and 1147-1158 of Seq ID No 162; amino acids 66-80, 169-178, 262-271, 333-348, 360-370 and 554-564 of Seq ID No 163; amino acids 5-17, 131-141, 183-198 and 376-385 of Seq ID No 164; amino acids 33-43, 187-200, 223-232, 259-272, 306-316, 443-452 and 461-472 of Seq ID No 165; amino acids 62-70, 141-161, 224-238, 297-305 and 339-354 of Seq ID No 166; amino acids 12-22, 82-94, 186-194, 371-382, 434-447 and 580-589 of Seq ID No 167; amino acids 27-38, 56-38, 77-102, 174-185, 259-270, 298-309 and 319-331 of Seq ID No 168; amino acids 10-18, 28-51, 59-68, 127-138, 167-177 and 338-347 of Seq ID No 169; amino acids 52-67, 86-98, 127-138, 144-167, 227-237, 282-296 and 307-319 of Seq ID No 170; amino acids 1-18, 124-137, 244-258, 298-310, 324-343, 368-384 and 431-450 of Seq ID No 171; amino acids 9-25, 75-88, 104-116, 121-133, 180-197, 215-227 and 324-338 of Seq ID No 172; amino acids 234-244, 336-346, 457-468 and 1002-1012 of Seq ID No 173; amino acids 3-18, 332-340, 445-454 and 580-591 of Seq ID No 174; amino acids 251-270, 342-352, 380-405, 407-417 and 443-451 of Seq ID No 175; amino acids 1-27 of Seq ID No 176; amino acids 22-46, 56-70, 79-96, 98-107 and 119-135 of Seq ID No 177; amino acids 10-38 of Seq ID No 178; amino acids 10-20 and 29-43 of Seq ID No 179; amino acids 3-18 of Seq ID No 180; amino acids 8-19 of Seq ID No 181; amino acids 5-22 of Seq ID No 182; amino acids 7-34 of Seq ID No 183; amino acids 21-34, 61-72 and 74-92 of Seq ID No 184; amino acids 5-14, 21-34 and 42-54 of Seq ID No 185; amino acids 4-32 of Seq ID No 186; amino acids 1-9 and 38-55 of Seq ID No 187; amino acids 4-12 and 20-30 of Seq ID No 188; amino acids 20-42 of Seq ID No 189; amino acids 41-52, 66-81 and 104-121 of Seq ID No 190; amino acids 1-17 of Seq ID No 191; amino acids 4-32 of Seq ID No 192; amino acids 10-45 of Seq ID No 193; amino acids 2-23 of Seq ID No 194; amino acids 8-29, 41-50 and 52-72 of Seq ID No 195; amino acids 3-26 of Seq ID No 196; amino acids 249-260 of Seq ID No 123; amino acids 569-636 of Seq ID No 124; amino acids 697-758 and 808-875 of Seq ID No 125; amino acids 3-23 of Seq ID No 126; amino acids 139-199 of Seq ID No 127; amino acids 20-89 of Seq ID No 128; amino acids 168-178 of Seq ID No 129; amino acids 112-182 and 167-216 of Seq ID No 130; amino acids 518-538 of Seq ID No 131; amino acids 31-105 of Seq ID No 132; amino acids 119-187 of Seq ID No 133; amino acids 34-43 of Seq ID No 134; amino acids 223-241 of Seq ID No 135; amino acids 91-170, 1369-1393 and 1423-1486 of Seq ID No 136; amino acids 397-456 of Seq ID No 137; amino acids 32-48 of Seq ID No 138; amino acids 5-66 of Seq ID No 139; amino acids 271-367 of Seq ID No 140; amino acids 270-279 and 621-632 of Seq ID No 141; amino acids 146-221 and 782-847 of Seq ID No 142; amino acids 216-283 of Seq ID No 143; amino acids 245-264 and 367-374 of Seq ID No 144; amino acids 716-781 of Seq ID No 145; amino acids 692-760 of Seq ID No 146; amino acids 1189-1244 of Seq ID No 147; amino acids 446-462 of Seq ID No 148; amino acids 79-94 and 136-145 of Seq ID No 149; amino acids 97-165 of Seq ID No 150; amino acids 106-123 of Seq ID No 151; amino acids 16-80 of Seq ID No 152; amino acids 36-113 of Seq ID No 153; amino acids 303-330 of Seq ID No 154; amino acids 154-168 of Seq ID No 155; amino acids 28-49 of Seq ID No 156; amino acids 406-485 of Seq ID No 157; amino acids 421-488 of Seq ID No 158; amino acids 442-492 and 526-610 of Seq ID No 159; amino acids 194-207 of Seq ID No 160; amino acids 1058-1076 and 1643-1700 of Seq ID No 161; amino acids 1068-1077 of Seq ID No 162; amino acids 228-287 of Seq ID No 163; amino acids 786-836 of Seq ID No 164; amino acids 306-370 of Seq ID No 165; amino acids 259-277 and 298-320 of Seq ID No 166; amino acids 498-506 of Seq ID No 167; amino acids 138-205 of Seq ID No 168; amino acids 247-293 of Seq ID No 169; amino acids 61-74 of Seq ID No 170; amino acids 201-262 of Seq ID No 171; amino acids 208-288 of Seq ID No 172; amino acids 938-1000 of Seq ID No 173; amino acids 309-374 of Seq ID No 174; amino acids 36-109 and 375-388 of Seq ID No 175; amino acids 10-21 of Seq ID No 176; amino acids 71-134 of Seq ID No 177; amino acids 5-26 of Seq ID No 178; amino acids 21-36 of Seq ID No 179; amino acids 1-10 of Seq ID No 180; amino acids 13-23 of Seq ID No 181; amino acids 5-11 of Seq ID No 182; amino acids 22-32 of Seq ID No 183; amino acids 11-72 of Seq ID No 184; amino acids 4-18 of Seq ID No 185; amino acids 13-26 of Seq ID No 186; amino acids 8-77 of Seq ID No 187; amino acids 5-27 of Seq ID No 188; amino acids 4-18 of Seq ID No 189; amino acids 30-45 of Seq ID No 190; amino acids 4-16 of Seq ID No 191; amino acids 12-25 of Seq ID No 192; amino acids 37-48 of Seq ID No 193; amino acids 4-21 of Seq ID No 194; amino acids 18-44 of Seq ID No 195 and amino acids 11-22 of Seq ID No 196.

In one embodiment the antigen further consists of
a) 1 to 50 additional amino acid residue(s), preferably 1 to 40, more preferably 1 to 30, even more preferably at most 1 to 25, still more preferably at most 1 to 10, most preferably 1, 2, 3, 4 or 5 additional amino acid residue(s); and/or
b) at least one amino acid residue heterologous to the core amino acid sequence.

Said additional amino acid residue(s) are further defined above.

In another embodiment said amino acid residue(s) is/are flanking the core amino acid sequence N-terminally, C-terminally, or N- and C-terminally.

In an embodiment of the invention the antigen comprises at least 2, at least 3, at least 4, at least 5 or at least 6 core amino acid sequences as defined above.

The problem underlying the present invention is solved in another aspect by a process for producing an antigen, or an active fragment or an active variant thereof, as defined in the present invention, comprising expressing the nucleic acid molecule as defined above.

The present invention further relates to a process for producing a cell which expresses an antigen, or an active fragment or an active variant thereof, as defined above, comprising transforming or transfecting a suitable host cell with the vector as defined above.

In an embodiment, the antigen, or the active fragment or the active variant thereof, is isolated from *Chlamydiae,* preferably from *C*. *pneumoniae, C. trachomatis, C. psittaci, C. pecorum, C. muridarum,* or *C*. *suis,* and more preferably from *C*. *pneumoniae, C. trachomatis* or *C*. *psittaci.*

The problem underlying the present invention is solved in another aspect by a pharmaceutical composition, preferably a vaccine, comprising an antigen, or an active fragment or an active variant thereof, as defined above, or a nucleic acid molecule as defined above, or a vector as defined above.

Antoher aspect of the present invention provides a pharmaceutical composition, preferably a vaccine, comprising an antigen, or an active fragment or an active variant thereof, as defined above, or a nucleic acid molecule as defined above, or a vector as defined above, for the treatment or prevention of an infection with *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

In a preferred embodiment the pharmaceutical composition of the present invention further comprises an immunostimulatory substance, preferably polycationic polymers, especially polycationic peptides, immunostimulatory oligo-deoxynucleotides (ODNs), especially Oligo(dIdC)₁₃, peptides containing at least two LysLeuLys motifs, especially KLKLLLLLKLK, neuroactive compounds, especially human growth hormone, alum, Freund's complete or incomplete adjuvants, or combinations thereof.

In a more preferred embodiment of the pharmaceutical composition of the present invention the immunostimulatory substance is a combination of either a polycationic polymer and immunostimulatory deoxynucleotides, or of a peptide containing at least two LysLeuLys motifs and immunostimulatory deoxynucleotides, preferably a combination of KLKLLLLLKLK and Oligo(dIdC)₁₃.

In a still more preferred embodiment of the pharmaceutical composition of the present invention the polycationic polymer is a polycationic peptide, especially polyarginine.

Still another aspect of the present invention provides an antigen, or an active fragment or an active variant thereof, as defined above, or a nucleic acid molecule as defined above, or a vector as defined above for the treatment or prevention of an infection with *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

Another preferred embodiment of the invention relates to the use of an antigen, an active fragment or an active variant thereof as defined above, or a nucleic acid molecule as defined above, or a vector as defined above for the preparation of a pharmaceutical composition, especially for the preparation of a vaccine, for treating or preventing infections with *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

The problem underlying the present invention is solved in a further aspect by an antibody, or at least an effective part thereof, which binds to at least a selective part of an antigen or a fragment thereof, preferably an active fragment thereof, or a variant thereof, preferably an active variant thereof, as defined above.

In a preferred embodiment the antibody is a monoclonal antibody.

In another preferred embodiment said effective part comprises a Fab fragment, a F(ab) fragment, a F(ab) N fragment, a F (ab)₂ fragment or a Fᵥ fragment.

In still another embodiment of the invention the antibody is a chimeric antibody.

In yet another embodiment the antibody is a humanized antibody.

In a further embodiment the antibody is an IgA antibody.

Another aspect of the invention relates to a hybridoma cell line, which produces an antibody as defined above.

The problem underlying the present invention is furthermore solved by a method for producing an antibody as defined above, characterized by the following steps:
a) initiating an immune response in a non-human animal by administrating an antigen, or an active fragment or an active variant thereof, as defined above, to said animal,
b) removing an antibody containing body fluid from said animal, and
c) producing the antibody by subjecting said antibody containing body fluid to further purification steps.

The invention further relates to a method for producing an antibody as defined above, characterized by the following steps:
a) initiating an immune response in a non-human animal by administrating an antigen or an active fragment or an active variant thereof, as defined above, to said animal,
b) removing the spleen or spleen cells from said animal,
c) producing hybridoma cells of said spleen or spleen cells,
d) selecting and cloning hybridoma cells specific for said antigen or for said active fragment or for said active variant thereof,
e) producing the antibody by cultivation of said cloned hybridoma cells, and
f) optionally conducting further purification steps.

Another aspect of the present invention is related to a pharmaceutical composition comprising an antibody as specified above.

Still another aspect relates to an antibody as defined above or a pharmaceutical composition comprising an antibody as defined above for the treatment or prevention of an infection with *Chlamydia,* preferably *C*. *pneumoniae, C. trachomatis, C. psittaci, C. pecorum, C. muridarum,* or *C. suis,* and more preferably *C*. *pneumoniae, C. trachomatis* or *C*. *psittaci.*

The problem underlying the present invention is solved in another aspect by the use of an antibody as defined above for the preparation of a pharmaceutical composition for treating or preventing infections with *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

According to another aspect the present invention provides an antagonist, which binds or is capable of binding to an antigen, or an active fragment or active variant thereof as disclosed in the present invention. According to a still further aspect the antagonist according to the present invention is an antagonist which is capable of reducing or inhibiting the interaction activity of an antigen, or an active fragment thereof or an active variant thereof, according to the present invention to its interaction partner. Such interaction partner is, in a preferred embodiment, an antibody or a receptor, preferably a physiological receptor, of said antigen, or an active fragment thereof or an active variant thereof.

According to another aspect the present invention provides a method for identifying an antagonist capable of binding to an antigen or an active fragment or an active variant thereof, as defined above, comprising:
a) contacting an isolated or immobilized antigen or an active fragment or an active variant thereof, as defined above, with a candidate antagonist under conditions to permit binding of said candidate antagonist to said antigen, or an active fragment or active variant thereof, in the presence of a component capable of providing a detectable signal in response to the binding of the candidate antagonist to said antigen, or an active fragment or an active variant thereof; and
b) detecting the presence or absence of a signal generated in response to the binding of the antagonist to said antigen, or an active fragment or active variant thereof.

The problem underlying the present invention is further solved by a method for identifying an antagonist capable of reducing or inhibiting the interaction activity of an antigen or an active fragment or an active variant thereof, as defined above, to its interaction partner comprising:
a) providing an antigen, or an active fragment or active variant thereof, as defined above,
b) providing an interaction partner to said antigen, or said active fragment or active variant thereof, especially an antibody as defined above,
c) allowing interaction of said antigen or said active fragment or active variant thereof, to said interaction partner to form an interaction complex,
d) providing a candidate antagonist,
e) allowing a competition reaction to occur between the candidate antagonist and the interaction complex,
f) determining whether the candidate antagonist inhibits or reduces the interaction activities of the antigen, or the active fragment or the active variant thereof, with the interaction partner.

The present invention further relates to the use of any of the antigens, or an active fragment or an active variant thereof, as defined above, for the isolation and/or purification and/or identification of an interaction partner of said antigen, or said active fragment or active variant thereof.

Another aspect of the present invention relates to a method for diagnosing an infection with a *Chlamydia* organism comprising the steps of:
a) contacting a sample obtained from a subject with an antigen, or an active fragment or active variant thereof, as defined above; and
b) detecting the presence of an antibody against said *Chlamydia* organism in the sample.

According to an embodiment of said method, the presence of one or more antibodies against said *Chlamydia* organism is indicative for the *Chlamydia* infection.

In another embodiment of said method the antibody is an IgA antibody.

In yet another aspect the present invention provides a method for diagnosing an infection with a *Chlamydia* organism comprising the steps of:
a) contacting a sample obtained from a subject with the antibody as defined above; and
b) detecting the presence of an antigen of said *Chlamydia* organism in the sample.

In an embodiment of said method the antigen of said *Chlamydia* organism is an antigen, or an active fragment or an active variant thereof, as defined above.

According to an embodiment of said method, the presence of one or more antigens of said *Chlamydia* organism is indicative for the *Chlamydia* infection.

In another embodiment of said method the antibody is an IgA antibody.

Still another aspect relates to a method for diagnosing an infection with a *Chlamydia* organism comprising the steps of:
a) contacting a sample obtained from a subject with a primer or a probe specific for a nucleic acid molecule, or a fragment thereof, as defined above; and
b) detecting the presence of such nucleic acid molecule or fragment thereof in the sample.

According to an embodiment of said method, the presence of one or more of said nucleic acid molecules or fragments thereof is indicative for the *Chlamydia* infection.

The present invention also provides a process for *in vitro* diagnosing a disease related to expression of a antigen or a fragment thereof according to the present invention comprising determining the presence of a nucleic acid sequence encoding said antigen or fragment thereof according to the present invention or determining the presence of the antigen or fragment thereof according to the present invention.

In an embodiment of any of the above described methods for diagnosing an infection with a *Chlamydia* organism, the *Chlamydia* organism is a pathogenic *Chlamydia* organism, more preferably *C*. *pneumonia, C. trachomatis, C. psittaci, C. pecorum, C. muridarum,* or *C*. *suis,* and most preferably *C*. *pneumoniae, C. trachomatis* or *C*. *psittaci.*

Moreover, the present invention provides the use of an antigen, or a fragment or a variant thereof, as defined in the present invention for the generation of a peptide binding to said antigen, or a fragment thereof or a variant thereof, wherein the peptide is an anticaline.

Moreover, the present invention provides the use of an antigen, or an active fragment or active variant thereof, as defined above, for the preparation of a functional nucleic acid, wherein the functional nucleic acid is selected from the group consisting of aptamers and spiegelmers.

In another aspect, the present invention provides the use of a nucleic acid molecule as defined above for the preparation of a functional ribonucleic acid, wherein the functional ribonucleic acid is selected from the group consisting of ribozymes, antisense nucleic acids and siRNA.

The problem underlying the present invention is further solved by a method for the treatment of a *Chlamydia* infection in an animal or human preferably in need thereof, comprising the step of administering to said animal or human a therapeutically effective amount of an antigen, or an active fragment or an active variant thereof, or a nucleic acid molecule, or a vector, or an antibody or a pharmaceutical composition as defined in any of the preceding aspects.

In an embodiment said *Chlamydia* infection is an infection with *C*. *pneumoniae, C. trachomatis, C. psittaci, C. pecorum, C. muridarum,* or *C*. *suis,* and preferably *C*. *pneumoniae, C. trachomatis* or *C*. *psittaci.*

The problem underlying the present invention is solved in another aspect by a method for immunizing an animal or human against infection with a *Chlamydia* organism, comprising the step of administering to said animal or human an effective amount of the antigen, or an active fragment or active variant thereof, as defined above, or the nucleic acid molecule as defined above, or a vector as defined above, or an antibody as defined above, or a pharmaceutical composition as defined above, wherein the effective amount is suitable to elicit an immune response in said animal or human.

In an embodiment of said method for immunizing an animal or human against infection with a *Chlamydia* organism, the *Chlamydia* organism is preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

The problem underlying the present invention is solved in yet another aspect by a method for stimulating an immune response in an animal or human against a *Chlamydia* organism, comprising the step of administering to said animal or human an effective amount of the antigen, or an active fragment or an active variant thereof, as defined above, or of the nucleic acid molecule as defined above or of a vector as defined above, or an antibody as defined above, or a pharmaceutical composition as defined above, wherein the effective amount is suitable to stimulate the immune response in said animal or human.

In an embodiment of said method for stimulating an immune response in an animal or human against a *Chlamydia* organism, the *Chlamydia* organism is preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

It is within the present invention that the various methods and uses, respectively, where an antigen as defined in the present invention is used, can also be performed or practiced using a fragment of such antigen, preferably an active fragment thereof, or a variant of such antigen, preferably an active variant thereof, each as preferably described herein. It is also within the present invention that the various kinds of compounds disclosed herein as interacting with or targeting the antigen according to the present invention, can additionally or alternatively interact with or target the active fragment or active variant of said antigen.

It is also within the present invention that each and any method in the practice of which an antibody is used, can, in principle, also be practiced when instead of the antibody the anticalines or the functional nucleic acids as defined herein are used, whereby it is preferred that such functional nucleic acid is selected from the group consisting of aptamers and spiegelmers. This applies equally to the various uses of the present application.

In a preferred embodiment a fragment of an antigen as disclosed herein is a part of such antigen which exhibits at least one feature of such antigen. Preferably such feature is a feature selected from the group consisting of suitability for the treatment of infections, immunization of an animal including human, and/or stimulation of an immune response in an animal including human.

It is also within the present invention that any disclosure made herein in relation to *Chlamydia* and more specifically *C*. *pneumoniae* is equally applicable to any *Chlamydia* species, whereby the *Chlamydia* species is preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

The terms "polypeptide", "peptide", "protein" or "antigen" are used interchangeably throughout the present specification and refer in a comprehensive manner to the antigen according to the present invention, including each and any variant, fragment, analogue or derivative thereof, particularly as described herein. Insofar, whenever the term polypeptide, peptide, protein or antigen is used herein, and if not explicitly stated otherwise, the respective disclosure is also made for or in relation to any antigen according to the present invention, including each and any variant, fragment, analogue or derivative thereof, particularly as described herein. Also it is to be understood that any use or aspect described in connection with any of the above mentioned compounds covered by the term polypeptide, peptide, protein or antigen according to the present invention shall be applicable also to each and any other of the above mentioned compounds covered by the term polypeptide, peptide, protein or antigen according to the present invention.

The present invention advantageously provides an efficient, relevant and comprehensive set of isolated nucleic acid molecules and antigens encoded by them, including the active fragments and the active variants thereof, using an antibody preparation from multiple human plasma pools and surface expression libraries derived from the genome of *C*. *pneumoniae.* Thus, the present invention fulfils a widely felt demand for *C*. *pneumoniae* antigens, vaccines, diagnostics and products useful in procedures for preparing antibodies and for identifying compounds effective against infections caused by pathogenic *Chlamydia* species, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

An effective vaccine should be composed of proteins or polypeptides, which are expressed by all strains and are able to induce high affinity, abundant antibodies against cell surface components of said pathogenic *Chlamydiae,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.* The antibodies should be IgG1 and/or IgG3 for opsonisation, any IgG subtype, and/or IgA for neutralisation of adherence and toxin action.

*C. pneumoniae* infects the host via the mucosal epithelia of the respiratory tract and mucosal immunity is therefore believed to be important to control infection. IgA is the primary immune globuline (Ig) isotype induced at mucosal sites and is thought to mediate defense functions at these sites.

In fact, it was observed that IgA-deficient mice were more susceptible to infection than wild-type mice (Rodriguez, A. et al., 2006), indicating that local IgA responses may play a role in the protection of the respiratory tract against *C*. *pneumoniae* infections.

IgA can be detected in patients after infection with *C*. *pneumoniae* in both, serum and sputum samples (Ciarrocchi, G. et al., 2004). Therefore, IgA antibodies have been selected to identify relevant antigens in the methods of the present invention. Thus, the antigens identified by said IgA screen, as well as respective IgA antibodies produced by the methods according to the present invention are well suited to be used as pharmaceutical compositions to treat or prevent infections with a *Chlamydia* species, especially *C*. *pneumoniae* infections.

A chemically defined vaccine must be definitely superior compared to a whole cell vaccine (attenuated or killed), since components of said pathogenic *Chlamydiae,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.,* which cross-react with human tissues or inhibit opsonisation can be eliminated, and the individual polypeptides inducing protective antibodies and/or a protective immune response can be selected.

In a preferred embodiment of the present invention, the nucleic acid molecules exhibit 70% identity over their entire length to a nucleotide sequence set forth in Seq ID No 1 to 98. More preferred are nucleic acids that comprise a region that is at least 80% or at least 85% identical over their entire length to a nucleic acid molecule set forth in Seq ID No 1 to 98. In this regard, nucleic acid molecules, which are at least 90%, 91%, 92%, 93%, 94%, 95%, or 96% identical over their entire length to the same are particularly preferred. Furthermore, those with at least 97% are highly preferred, those with at least 98% and at least 99% are particularly highly preferred, with at least 99% or 99.5% being the more preferred, with 100% identity being especially preferred. Moreover, preferred embodiments in this respect are nucleic acids, which encode antigens or fragments thereof (polypeptides), which retain substantially the same biological function or activity as the mature polypeptide set forth in the Seq ID Nos 99 to 196. It is also within the present invention that the nucleic acid molecules according to the present invention are coding for a protein which is preferably an antigen. Still further it is within the present invention, that the molecules defined by Seq ID Nos 99 to 196 are proteins, which are preferably antigens.

Identity, as known in the art and used herein, is the relationship between two or more polypeptide sequences or two or more polynucleotide sequences, as determined by comparing the sequences. In the art, identity also means the degree of sequence relatedness between polypeptide or polynucleotide sequences, as the case may be, as determined by the match between strings of such sequences. Identity can be readily calculated. While a number of methods exist to measure identity between two polynucleotide or two polypeptide sequences, the term is well known to skilled artisans (e.g. Sequence Analysis in Molecular Biology, von Heinje, G., Academic Press, 1987). Preferred methods to determine identity are designed to give the largest match between the sequences tested. Methods to determine identity are codified in computer programs. Preferred computer program methods to determine identity between two sequences include, but are not limited to, GCG program package (Devereux, J. et al., 1984), BLASTP, BLASTN, and FASTA (Altschul, S. et al., 1990).

As a second alternative to the nucleic acid molecules described herein by reference to Seq ID Nos 1 to 98, the description of which is also referred to herein as first alternative, the nucleic acid molecules according to the present invention can also be nucleic acid molecules, which are at least essentially complementary to the nucleic acids described in accordance with the first alternative herein. It will be acknowledged by the ones skilled in the art that an individual nucleic acid molecule is at least essentially complementary to another individual nucleic acid molecule. As used herein complementary means that a nucleic acid strand is base pairing via Watson-Crick base pairing with a second nucleic acid strand. Essentially complementary as used herein means that the base pairing is not occurring for all of the bases of the respective strands but leaves a certain number or percentage of the bases unpaired or wrongly paired. The percentage of correctly pairing bases is preferably at least 70%, more preferably 80%, even more preferably 90% and most preferably any percentage higher than 90%. Such higher percentage includes 91, 92, 93, 94, 95, 96, 97, 98, 99 and 100%, whereby such definition is applicable to each aspect of the present application where this kind of terminology is used. It is to be noted that a percentage of 70% matching bases is considered as homology and the hybridisation having this extent of matching base pairs is considered as stringent. Hybridisation conditions for this kind of stringent hybridisation may be taken from Current Protocols in Molecular Biology (John Wiley and Sons, Inc., 1987). More particularly, the hybridisation conditions can be as follows:
- Hybridisation performed e.g. in 5x SSPE, 5x Denhardt's reagent, 0.1% SDS, 100 g/mL sheared DNA at 68°C
- Moderate stringency wash in 0.2x SSC, 0.1% SDS at 42°C
- High stringency wash in 0.1x SSC, 0.1% SDS at 68°C

Genomic DNA with a GC content of 50% has an approximate T_{M} of 96°C. For 1% mismatch, the T_{M} is reduced by approximately 1°C.

In addition, any of the further hybridisation conditions described herein are in principle applicable as well.

Of course, all nucleic acid sequence molecules which encode the same polypeptide molecule as those identified by the present invention are encompassed by any disclosure of a given coding sequence, since the degeneracy of the genetic code is directly applicable to unambiguously determine all possible nucleic acid molecules which encode a given polypeptide molecule, even if the number of such degenerated nucleic acid molecules may be high. This is also applicable for active fragments or active variants of a given antigen, as long as the fragments or variants encode an antigen being suitable to be used such that the same effect can be obtained as if the full-length antigen was used. Preferably, such antigens or active fragments or active variants thereof may be used in a vaccination application, e.g. as an active or passive vaccine.

As a third alternative, the nucleic acid molecule according to the present invention can also be a nucleic acid which comprises a stretch of at least 15 bases of the nucleic acid molecule according to the first or second alternative of the nucleic acid molecules according to the present invention as outlined above. Preferably, the bases form a contiguous stretch of bases. However, it is also within the scope of the present invention that the stretch consists of two or more moieties, which are separated by a number of bases.

The nucleic acid molecules according to the present invention may preferably consist of at least 20, even more preferred at least 30, especially at least 50 contiguous bases from the sequences disclosed herein. The suitable length may easily be optimised due to the intended field of use (e.g. as (PCR) primers, probes, capture molecules (e.g. on a (DNA) chip), etc.). Preferred nucleic acid molecules contain at least a contiguous 15 base portion of one or more of the immunogenic amino acid sequences listed in Tables 1. Specifically preferred are nucleic acids containing a contiguous portion of a DNA sequence of any sequence contained in the sequence protocol of the present application which shows 1 or more, preferably more than 2, especially more than 5, non-identical nucleic acid residues compared to the genome sequences of *C*. *pneumoniae* AR39, CWL029, TW183 and J138 that are available (http://www.ncbi.nlm.nih.gov/genomes/lproks.cgi, or http://pedant.gsf.de/, and http://cmr.tigr.org/). Specifically preferred non-identical nucleic acid residues are residues, which lead to a non-identical amino acid residue. Preferably, the nucleic acid sequences encode polypeptides, proteins, or antigens having at least 1, preferably at least 2, preferably at least 3 different amino acid residues compared to the published or listed *C*. *pneumoniae* counterparts mentioned above. Preferably, this kind of polypeptides, proteins, or antigens still has at least one of the characteristics of the molecules disclosed herein having identical amino acid residues. Also preferred are such isolated polypeptides, which are fragments of the proteins or of the antigens disclosed herein, e.g. in the Sequence Listing, having at least 6, 7, or 8 amino acid residues and being encoded by the nucleic acids as described herein.

The nucleic acid molecule according to the present invention can, as a fourth alternative, also be a nucleic acid molecule which anneals under stringent hybridisation conditions to any of the nucleic acids of the present invention according to the first, second, or third alternative as disclosed herein. Stringent hybridisation conditions are typically those described herein.

Finally, the nucleic acid molecule according to the present invention can, as a fifth alternative, also be a nucleic acid molecule which, but for the degeneracy of the genetic code, would hybridise to any of the nucleic acid molecules of the present invention according to the first, second, third, and fourth alternative as outlined herein. This kind of nucleic acid molecule refers to the fact that preferably the nucleic acids according to the present invention code for the antigen, or fragments or variants thereof, according to the present invention. This kind of nucleic acid molecule is particularly useful in the detection of a nucleic acid molecule according to the present invention and thus the diagnosis of the respective microorganisms such as *C*. *pneumoniae* or any pathogenic *Chlamydia* species, particularly those pathogenic *Chlamydia* species disclosed herein, and any disease or diseased condition where these kinds of microorganism are involved. Preferably, such microorganism, especially an opportunistic microorganism, is causing such disease directly or indirectly. Preferably, the hybridisation could occur or be preformed under stringent conditions as described in connection with the fourth alternative described herein.

Nucleic acid molecule as used herein generally refers to any ribonucleic acid molecule, such as mRNA or cRNA, or deoxyribonucleic acid molecule, including, for instance, cDNA and genomic DNA obtained by cloning or produced by chemical synthetic techniques or by a combination thereof. The nucleic acid molecule may be unmodified RNA or DNA or modified RNA or DNA. Thus, for instance, nucleic acid molecule as used herein refers to, among others, single- and double-stranded DNA, DNA that is a mixture of single- and double-stranded DNA, and RNA that is a mixture of single- and double-stranded regions, hybrid molecules comprising DNA and RNA that may be single-stranded or, more typically, double-stranded, or triple-stranded, or a mixture of single- and double-stranded regions. In addition, nucleic acid molecule as used herein refers to triple-stranded regions comprising RNA or DNA or both RNA and DNA. The strands in such regions may be from the same molecule or from different molecules. The regions may be derived from one or more of the molecules, but more typically involve only a region of some of the molecules. One of the molecules of a triple-helical region often is an oligonucleotide. Single-stranded DNA may be the coding strand, also known as the sense strand, or it may be the non-coding strand, also referred to as the anti-sense strand. As used herein, the term nucleic acid molecule includes DNAs or RNAs as described above that contain one or more modified bases. Thus, DNAs or RNAs with backbones modified for stability or for other reasons are nucleic acid molecules as that term is intended herein. Moreover, DNAs or RNAs comprising unusual bases, such as inosine, or modified bases, such as tritylated bases, to name just two examples, are nucleic acid molecules as the term is used herein. It will be appreciated that a great variety of modifications can be made to DNA and RNA that serve many useful purposes known to those of skill in the art. The term nucleic acid molecule as it is employed herein embraces such chemically, enzymatically or metabolically modified forms of nucleic acid molecule, as well as the chemical forms of DNA and RNA characteristic of viruses and cells, including simple and complex cells, *inter alia.* The term nucleic acid molecule also embraces short nucleic acid molecules often referred to as oligonucleotide(s). "Polynucleotide" and "nucleic acid" or "nucleic acid molecule" are often used interchangeably herein.

Nucleic acid molecules provided in the present invention also encompass numerous unique fragments, both longer and shorter than the nucleic acid molecule sequences set forth in the sequencing listing of the present application, more specifically of the *C*. *pneumoniae* coding regions, which can be generated by standard cloning methods. To be unique, a fragment must be of sufficient size to distinguish it from other known nucleic acid sequences, most readily determined by comparing any selected *C*. *pneumoniae* fragment to the nucleotide sequences in biosequence databases such as GenBank. It will be appreciated by the one skilled in the art that what is said herein in any aspect in relation to *C*. *pneumoniae* applies equally to any of the other *Chlamydia* species described herein, more preferably any pathogenic *Chlamydia* species described herein.

Additionally, modifications can be made to the nucleic acid molecules and polypeptides that are encompassed by the present invention. For example, the nucleic acid also includes sequences that are a result of the degeneration of the genetic code. There are 20 natural amino acids, most of which are specified by more than one codon. Thus, nucleotide substitutions can be made which do not affect the polypeptide encoded by the nucleic acid. Accordingly, any nucleic acid molecule which encodes an antigen or fragments thereof is encompassed by the present invention.

Furthermore, any of the nucleic acid molecules encoding antigens or fragments thereof provided by the present invention can be functionally linked, using standard techniques such as standard cloning techniques, to any desired regulatory sequences, whether an *C*. *pneumoniae* regulatory sequence or a heterologous regulatory sequence, heterologous leader sequence, heterologous marker sequence or a heterologous coding sequence to create a fusion protein.

The present invention further relates to variants of the nucleic acid molecules described herein which encode fragments, analogs and derivatives of the antigens and fragments thereof having a deducted *C*. *pneumoniae* amino acid sequence set forth in the Sequence Listing. A variant of the nucleic acid molecule may be a naturally occurring variant such as a naturally occurring allelic variant, or it may be a variant that is not known to occur naturally. Such non-naturally occurring variants of the nucleic acid molecule may be made by mutagenesis techniques, including those applied to nucleic acid molecules, cells or organisms.

Among variants in this regard are variants that differ from the aforementioned nucleic acid molecules by nucleotide substitutions, deletions or additions. The substitutions, deletions or additions may involve one or more nucleotides. The variants may be altered in coding or non-coding regions or both. Alterations in the coding regions may produce conservative or non-conservative amino acid substitutions, deletions or additions. Preferred are nucleic acid molecules encoding a variant, analog, derivative or fragment, or a variant, analogue or derivative of a fragment, which have an *C*. *pneumoniae* sequence as set forth in the Sequence Listing, in which several, a few, 5 to 10, 1 to 5, 1 to 4, 3, 2, 1 or no amino acid(s) is substituted, deleted or added, in any combination. Especially preferred among these are silent substitutions, additions and deletions, which do not alter the properties and activities of the *C*. *pneumoniae* polypeptides set forth in the Sequence Listing. Also especially preferred in this regard are conservative substitutions.

The nucleic acid molecules of the present invention may also be used as a hybridisation probe for, e.g., RNA, cDNA and genomic DNA to isolate full-length cDNAs and genomic clones encoding polypeptides of the present invention and to isolate cDNA and genomic clones of other genes that have a high sequence similarity to the nucleic acid molecules of the present invention. Such probes generally will comprise at least 15 bases. Preferably, such probes will have at least 20, at least 25 or at least 30 bases, and may have at least 50 bases. Particularly preferred probes will have at least 30 bases, and will have 50 bases or less, such as 30, 35, 40, 45, or 50 bases.

For example, the coding region of a nucleic acid molecule of the present invention may be isolated by screening a relevant library using the known DNA sequence to synthesize an oligonucleotide probe. A labelled oligonucleotide having a sequence complementary to that of a gene of the present invention is then used to screen a library of cDNA, genomic DNA or mRNA to determine to which members of the library the probe hybridizes.

The nucleic acid molecules and polypeptides of the present invention may be employed as reagents and materials for the development or preparation of pharmaceutical compositions and/or diagnostics for diseases, particularly human disease, as further discussed herein.

The nucleic acid molecules of the present invention that are oligonucleotides can be used in the processes herein as described, but preferably for PCR, to determine whether or not the *C*. *pneumoniae* genes identified herein in whole or in part are present and/or transcribed in infected tissue such as skin, synovia or blood. It is recognized that such sequences will also have utility in diagnosis of the stage of infection and type of infection the pathogen has attained. For this and other purposes arrays which are known as such in the art, comprising at least one of the nucleic acids or polypeptides according to the present invention as described herein, may be used.

The nucleic acid molecules according to the present invention may be used for the detection of nucleic acid molecules and organisms or samples containing these nucleic acids. Preferably such detection is for diagnosis, more preferably for the diagnosis of a disease related or linked to the presence or abundance of *Chlamydiae* or any other pathogen species of *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

Eukaryotes (herein also "individual(s)"), particularly mammals, and especially humans, infected with *Chlamydiae* or any other pathogen species of *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci* can be identified by detecting any of the nucleic acid molecules according to the present invention detected at the DNA level by a variety of techniques. Preferred nucleic acid molecule candidates for distinguishing *Chlamydiae* or said other pathogenic *Chlamydia* from other organisms can be obtained.

The invention provides a process for diagnosing disease, arising from infection with *Chlamydiae* or any other pathogen species of *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci,* comprising determining from a sample isolated or derived from an individual an increased level of expression of a nucleic acid molecule having the sequence of a nucleic acid molecule as disclosed herein and more preferably set forth in the Sequence Listing. Expression of nucleic acid molecules can be measured using any one of the methods well known in the art for the quantification of nucleic acid molecules, such as, for example, PCR, RT-PCR, RNase protection, Northern blotting, other hybridisation methods and the arrays described herein.

Isolated as used herein means separated "by the hand of man" from its natural state; i.e., that, if it occurs in nature, it has been changed or removed from its original environment, or both. For example, a naturally occurring nucleic acid molecule or a polypeptide naturally present in a living organism in its natural state is not "isolated", but the same nucleic acid molecule or polypeptide separated from the coexisting materials of its natural state is "isolated", as the term is employed herein. As part of or following isolation, such nucleic acid molecules can be joined to other nucleic acid molecules, such as DNAs, for mutagenesis, to form fusion genes, and for propagation or expression in a host, for instance. The isolated nucleic acid molecules, alone or joined to other nucleic acid molecules such as vectors, can be introduced into host cells, in culture or in whole organisms. Introduced into host cells in culture or in whole organisms, such DNAs still would be isolated, as the term is used herein, because they would not be in their naturally occurring form or environment. Similarly, the nucleic acid molecules and polypeptides may occur in a composition, such as a media formulations, solutions for introduction of nucleic acid molecules or polypeptides, for example, into cells, compositions or solutions for chemical or enzymatic reactions, for instance, which are not naturally occurring compositions, and, therein remain isolated nucleic acid molecules or polypeptides within the meaning of that term as it is employed herein.

The nucleic acids can, for example, be isolated from *Chlamydiae* or any other pathogen species of *Chlamydia,* preferably *C. pneumoniae, C. trachomatis, C. psittaci, C. pecorum, C. muridarum,* or *C. suis,* and more preferably *C. pneumoniae, C. trachomatis* or *C. psittaci* by methods known to the one skilled in the art. The same applies to the polypeptides according to the present invention.

Preferably, the nucleic acid molecules of the present invention may be originally formed *in vitro,* e.g. by chemical synthesis, or in a cell culture and subsequent isolation or purification. In general, the nucleic acids may be obtained by the manipulation of nucleic acids by endonucleases and/or exonucleases and/or polymerases and/or ligases and/or recombinases or other methods known to the skilled practitioner to produce the nucleic acids.

The nucleic acid sequences as defined by Seq ID Nos 1 to 98 start with the first complete codon comprised by the fragment as inserted into the vector and encodes the first amino acid as defined by Seq ID Nos 99 to 196. However, for the recombinant production additional nucleic acids might be useful or necessary to facilitate the cloning and expression.

The present invention also relates to vectors, which comprise a nucleic acid molecule or nucleic acid molecules of the present invention. A vector may additionally include nucleic acid sequences that permit it to replicate in the host cell, such as an origin of replication, one or more therapeutic genes and/or selectable marker genes and other genetic elements known in the art such as regulatory elements directing transcription, translation and/or secretion of the encoded protein. The vector may be used to transduce, transform or infect a cell, thereby causing the cell to express nucleic acids and/or proteins other than those native to the cell. The vector optionally includes materials to aid in achieving entry of the nucleic acid into the cell, such as a viral particle, liposome, protein coating or the like.

The present invention also relates to host cells, which are genetically engineered with vectors of the invention and to the production of the polypeptides according to the present invention by recombinant techniques.

A great variety of expression vectors can be used to express the polypeptides according to the present invention. Generally, any vector suitable to maintain, propagate or express nucleic acids to express a polypeptide in a host may be used for expression in this regard. In accordance with this aspect of the invention the vector may be, for example, a plasmid vector, a single or double-stranded phage vector, a single or double-stranded RNA or DNA viral vector. Starting plasmids disclosed herein are either commercially available, publicly available, or can be constructed from available plasmids by routine application of well-known, published procedures. Preferred among vectors, in certain respects, are those for expression of nucleic acid molecules and the polypeptides according to the present invention. Nucleic acid constructs in host cells can be used in a conventional manner to produce the gene product encoded by the recombinant sequence. Alternatively, the polypeptides according to the preset invention can be synthetically produced by conventional peptide synthesizers. Mature proteins can be expressed in mammalian cells, yeast, bacteria, or other cells under the control of appropriate promoters. Cell-free translation systems can also be employed to produce such proteins using RNAs derived from the DNA construct of the present invention.

Host cells can be genetically engineered to incorporate nucleic acid molecules and express nucleic acid molecules of the present invention. Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, 293, Vero, PER.C6^{®} and Bowes melanoma cells; and plant cells.

The host cells can be transfected, e.g. by conventional means such as electroporation with at least one expression vector containing a nucleic acid of the invention under the control of a transcriptional regulatory sequence.

According to another aspect of the present invention, a comprehensive set of novel polypeptides is provided. Such polypeptide, as mentioned previously herein, are antigens as disclosed herein, and the fragments thereof, preferably the active fragments thereof, and the variants thereof, preferably the active variants thereof. Preferably, the polypeptides according to the present invention are antigens and fragments thereof. In a preferred embodiment of the invention, an antigen comprising an amino acid sequence being preferably encoded by any one of the nucleic acids molecules and fragments thereof as described herein, are provided. In another preferred embodiment of the invention a novel set of proteins and antigens and active fragments as well as active variants thereof is provided which comprise amino acid sequences selected from the group consisting of Seq ID Nos 99 to 196.

The polypeptides according to the present invention, i.e. the antigens, as provided by the present invention preferably include any polypeptide or molecule set forth in the Sequence Listing as well as polypeptides which have at least 70% identity to such polypeptide according to the present invention, preferably at least 80% or 85% identity to such polypeptide according to the present invention, and more preferably at least 90% similarity (more preferably at least 90% identity) to such polypeptide according to the present invention and more preferably as set forth in the Sequence Listing and still more preferably at least 95%, 96%, 97%, 98%, 99% or 99.5% similarity (still more preferably at least 95%, 96%, 97%, 98%, 99%, or 99.5% identity) to such polypeptide according to the present invention and also include portions of such polypeptides with such portion of the polypeptide generally containing at least 4 amino acids and more preferably at least 8, still more preferably at least 30, still more preferably at most 50 amino acids, such as 4, 8, 10, 20, 30, 35, 40, 45 or 50 amino acids. In a preferred embodiment such portions are active fragments of the polypeptides according to the present invention.

The invention also relates to fragments, analogs, and derivatives of the polypeptides according to the present invention. The terms "fragment", "derivative" and "analog" when referring to such polypeptide whose amino acid sequence is preferably set forth in the Sequence Listing, means a polypeptide which retains essentially the same or a similar biological activity as such polypeptide. It will be acknowledged by the ones skilled in the art that the meaning of the term "similar biological activity" as used herein preferably depends on the polypeptide under consideration and more specifically its function. The term "biological activity" as used herein is further defined below. More preferably, a similar biological function or activity differs from the function of the non-fragment or the non-derivative in terms of extent of activity, affinity, immunogenicity, stability and/or specificity. In a preferred embodiment the difference is less than 50%, less than 75% or less than 90%.

In an embodiment the fragment, derivative, variant or analog of a polypeptide according to the present invention is 1) one in which one or more of the amino acid residues are substituted with a conserved or non-conserved amino acid residue (preferably a conserved amino acid residue) and such substituted amino acid residue may or may not be one encoded by the genetic code, or 2) one in which one or more of the amino acid residues includes a substituent group, or 3) one in which the polypeptide according to the present invention or a fragment thereof is fused with another compound, such as a compound to increase the half-life of the polypeptide according to the present invention or a fragment thereof such as, for example, polyethylene glycol, or 4) one in which the additional amino acids are fused to the polypeptide according to the present invention or a fragment thereof, such as a leader or secretory sequence or a sequence which is employed for purification of said polypeptide according to the present invention or fragment thereof or a proprotein sequence. Such fragments, derivatives, variants and analogs are deemed to be within the scope of those skilled in the art from the teachings herein.

The present invention also relates to proteins and antigens of different *Chlamydia* species, preferably pathogenic *Chlamydia* species, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci* which are preferably homologues. Such homologues may easily be isolated based on the nucleic acid and amino acid sequences disclosed herein.

There are multiple serotypes, genotypes or clinical strains distinguished to date for each of the pathogens and the typing is based on serotype specific antisera or molecular approaches. The presence of any antigen can accordingly be determined for every serotype, genotype or strain. The contribution of the various serotypes to the different *Chlamydia* infections varies in different age groups and especially geographical regions. It is an important aspect that the most valuable protective antigens need to be conserved among various clinical strains.

Additionally, fusion polypeptides comprising such antigens, variants, analogs, derivatives and fragments thereof, and variants, analogs and derivatives of the fragments are also encompassed by the present invention. Such fusion polypeptides and proteins, as well as nucleic acid molecules encoding them, can readily be made using standard techniques, including standard recombinant techniques for producing and expression of a recombinant polynucleic acid encoding a fusion protein.

In another embodiment of the invention the peptide as defined above may be modified by a variety of chemical techniques to produce derivatives having essentially the same activity (as defined above for fragments and variants) as the un-modified peptides, and optionally having other desirable properties. For example, carboxylic acid groups of the protein, whether C-terminal or side chain, may be provided in the form of a salt of a pharmaceutically acceptable cation or esterified to form an ester, or converted to an amide. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be converted to an amide. Hydroxyl groups of the peptide side chains may be converted to alkoxy or to an ester using well recognized techniques. Phenyl and phenolic rings of the peptide side chains may be substituted with one or more halogen atoms, such as fluorine, chlorine, bromine or iodine, or with alkyl, alkoxy, carboxylic acids and esters thereof, or amides of such carboxylic acids. Thiols can be protected with any one of a number of well recognized protecting groups, such as acetamide groups.

Further particularly preferred in this regard are variants, analogs, derivatives and fragments, and variants, analogs and derivatives of the fragments, having the amino acid sequence of any polypeptide according to the present invention as disclosed herein and preferably set forth in the Sequence Listing, in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the peptide of the present invention. Also especially preferred in this regard are conservative substitutions. Most highly preferred are peptides having an amino acid sequence set forth in the Sequence Listing without substitutions.

Variants of any of the antigens in their various embodiments disclosed herein and in particular the antigens and peptides specified herein by Seq ID Nos 99 to 196, can typically also be characterized by means of bioinformatics. Respective tools such as the NCBI Basic Local Alignment Search Tool (BLAST) (Altschul, S. et al., 1990) are available from several sources, including the National Center for Biotechnology Information (NCBI, Bethesda, MD) and on the Internet, for use in connection with the sequence analysis programs blastp, blastn, blastx, tblastn and tblastx. For comparisons of amino acid sequences of at least 35 amino acids, the Blast 2 sequences function of NCBI Blast 2.0 was employed using the default BLOSUM62 matrix set to default parameters (gapped blastp; gap existence cost of 11, and a per residue gap cost of 1). When aligning short peptides (fewer than around 35 amino acids), the alignment is performed using the Blast 2 sequences function, employing the PAM30 matrix set to default parameters (open gap 9, extension gap 1 penalties). Methods for determining sequence identity over such short windows such as 15 amino acids or less are described at the website that is maintained by the National Center for Biotechnology Information in Bethesda, Maryland (http://www.ncbi.nlm.nih.gov/BLAST/).

The active variant of an antigen is obtained by sequence alterations in the antigen, including each and any variant, fragment, analogue or derivative thereof, if not explicitly indicated to the contrary, wherein the polypeptide according to the present invention with the sequence alterations retains a function of the unaltered polypeptide according to the present invention, e.g. having a biological activity similar to that displayed by the complete antigen, including the ability to induce an immune response and/or to show protection against a *Chlamydia* organism e.g. in a mouse model of *Chlamydia* infection. Suitable mouse models are, for example, the mouse model of pneumonia as described in Yang, Z.P. et al. (1993); the mouse model of Alzheimer's disease as described by Little, C.S. et al. (2004); the mouse model of primary biliary cirrhosis as described by Marangoni, A. et al. (2006), and/or the mouse model of atherosclerosis as described in Hauer, A.D. et al. (2006).

A further example of retaining the function of the unaltered polypeptide according to the present invention is that the active variant of the antigen specifically binds a polypeptide specific antibody that binds an unaltered form of the polypeptide according to the present invention. By "biological function" or "biological activity" is preferably meant a function of the polypeptide in cells or organisms in which it naturally occurs, even if the function is not necessary for the growth or survival of the cells and organisms, respectively. For example, the biological function of a porin is to allow the entry into cell of compounds present in the extracellular medium. The biological function is distinct from the antigenic function. A polypeptide according to the present invention can have more than one biological function.

The sequence alterations of such variants can include, but are not limited to, conservative substitutions, deletions, mutations and insertions. Among preferred variants are those that vary from a reference by conservative amino acid substitutions. Conservative substitutions are those that substitute a given amino acid in a polypeptide according to the present invention by another amino acid of like characteristics, i.e. those substitutions that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains, etc.. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe and Tyr.

In one embodiment, one conservative substitution is included in the peptide. In another embodiment, two conservative substitutions or less are included in the peptide. In a further embodiment, three conservative substitutions or less are included in the peptide.

Examples of conservative amino acid substitutions include, but are not limited to, those listed below:

| Original Residue | Conservative Substitutions |
|---|---|
| Ala | Ser |
| Arg | Lys |
| Asn | Gln; His |
| Asp | Glu |
| Cys | Ser |
| Gln | Asn |
| Glu | Asp |
| His | Asn; Gln |
| Ile | Leu; Val |
| Leu | Ile; Val |
| Lys | Arg; Gln; Asn |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Preferably, the active variant exhibits reactivity with human sera of patients with *Chlamydia* infections, more preferably mediates seroconversion and most preferably shows bactericidal activity. These characteristics of the active variant can be assessed e.g. as detailed in the Examples. In the context of the present invention a variant specifically binds a specific antibody (preferably being polyclonal antibodies raised against recombinant proteins in animals such as mouse, rabbit or monoclonal antibodies generated in mouse), exhibits reactivity with human sera from patients with *Chlamydia* infections, mediates seroconversion or shows bactericidal activity, if the activity of the variant amounts to at least 10%, preferably at least 25%, more preferably at least 50%, even more preferably at least 70%, still more preferably at least 80%, especially at least 90%, particularly at least 95%, most preferably at least 99% of the activity of the antigen without sequence alterations.

Said active variants include naturally-occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide, as it is described above.

Within any species of the living world, allelic variation is the rule. For example, any bacterial species, e.g. *C*. *pneumoniae,* is usually represented by a variety of strains (characterized by clonal reproduction) that differ from each other by minor allelic variations. Indeed, a polypeptide that fulfils the same biological function in different strains can have an amino acid sequence that is not identical in each of the strains. Such an allelic variation is equally reflected at the nucleotide level.

Allelic variation is very common within the *Chlamydia* species as described for outer membrane protein A (Brunelle, B.W. et al., 2006).

In a preferred embodiment, the active variant or the active fragment derived from the polypeptide according to the present invention by amino acid exchanges, deletions or insertions may also conserve, or more preferably improve, the activity (reactivity, seroconversion and/or bactericidal activity as defined herein). Furthermore, these polypeptides may also cover epitopes, which trigger the same or preferably an improved T cell response. These epitopes are referred to as "heteroclitic" as further defined herein. They have a similar or preferably greater affinity to MHC/HLA molecules, and the ability to stimulate the T cell receptors (TCR) directed to the original epitope in a similar or preferably stronger manner. Heteroclitic epitopes can be obtained by rational design i.e. taking into account the contribution of individual residues to binding to MHC/HLA as for instance described by Rammensee H. et al. (1999), combined with a systematic exchange of residues potentially interacting with the TCR and testing the resulting sequences with T cells directed against the original epitope. Such a design is possible for a skilled person in the art without undue experimentation.

In a still more preferred embodiment of the invention the active variant of a polypeptide according to the present invention is any of the polypeptides disclosed herein and more specifically any of the polypeptides defined by the Seq ID Nos 99 to 196, having at least 50% sequence identity to the polypeptides of any of said Seq ID Nos 99 to 196, especially at least 60%, preferably at least 70%, more preferably at least 80%, still more preferably at least 90%, even more preferably at least 95%, 96%, 97%, 98%, most preferably 99% sequence identity to the polypeptides of any of said Seq ID Nos 99 to 196 and/or is derived from said polypeptides of any of the sequences of Seq ID Nos 99 to 196 by conservative substitutions as defined above.

The polypeptides according to the present invention, and fragments and variants thereof, also include or consist of modified epitopes wherein preferably one or two of the amino acids of a given epitope are modified or replaced according to the rules disclosed in, e.g., Tourdot, S. et al., (2000), as well as the nucleic acid sequences encoding such modified epitopes. The epitopes as presented by the polypeptides according to the present invention are also referred to herein as the present epitopes.

It is clear that also epitopes derived from the present epitopes by amino acid exchanges improving, conserving or at least not significantly impeding the T cell activating capability of the epitopes are covered by the epitopes according to the present invention. Therefore the present epitopes also cover epitopes, which do not contain the original sequence as derived from *C*. *pneumoniae,* but trigger the same or preferably an improved T cell response. These epitope are referred to as "heteroclitic"; they need to have a similar or preferably greater affinity to MHC/HLA molecules, and the need the ability to stimulate the T cell receptors (TCR) directed to the original epitope in a similar or preferably stronger manner.

Another possibility for identifying epitopes and more specifically heteroclitic epitopes includes the screening of peptide libraries with T cells directed against one or several of the present epitopes. A preferred way is the positional scanning of synthetic peptide libraries. Such approaches have been described in detail for instance by Hemmer, B. et al., (1999) and the references given therein.

As an alternative to epitopes represented by the present derived amino acid sequences or heteroclitic epitopes as disclosed herein, also substances or compounds mimicking these epitopes which are also referred to herein as "peptidemimetica" or "retro-inverse-peptides" can be applied and are thus within the present invention.

Another aspect of the design of improved epitopes is their formulation or modification with substances increasing their capacity to stimulate T cells. These include T helper cell epitopes, lipids or liposomes or preferred modifications as described in WO 01/78767.

Another way to increase the T cell stimulating capacity of epitopes is their formulation with immune stimulating substances for instance cytokines or chemokines like interleukin-2, -7, -12, -18, class I and II interferons (IFN), especially IFN-γ, GM-CSF, TNF-alpha, flt3-ligand and others.

The polypeptides according to the present invention are preferably provided in an isolated form, and preferably are purified to homogeneity.

In another embodiment of the present invention the variant is a fragment. The fragment is characterized by being derived from the antigen as defined above by one or more amino acid deletions. The deletion(s) may be, C-terminally, N-terminally and/or internally. Preferably the fragment is obtained by at most 10, 20, 30, 40, 50, 60, 80, 100, 150 or 200, more preferably by at most 10, 20, 30, 40 or 50, even more preferably at most 5, 10 or 15, still more preferably at most 5 or 10, most preferably 1, 2, 3, 4 or 5 deletion(s). The active fragment of the invention is characterized by having a biological activity similar to that displayed by the complete antigen, including the ability to induce an immune response and/or to show protection against *Chlamydia* e.g. in a mouse model of *Chlamydia* infection, such as described above. The fragment of an antigen is active in the context of the present invention, if the activity of the fragment amounts to at least 10%, preferably at least 25%, more preferably at least 50%, even more preferably at least 70%, still more preferably at least 80%, especially at least 90%, particularly at least 95%, most preferably at least 99% of the activity of the antigen without sequence alteration. These fragments may be designed or obtained in any desired length, including as small as about 50 to 80 amino acids in length.

In a further embodiment a fragment, and more preferably an active fragment, of the polypeptide according to the present invention are characterised by structural or functional attributes, i.e. fragments that comprise alpha-helix and alpha-helix forming regions, beta-sheet and beta-sheet forming regions, turn and turn-forming regions, coil and coil-forming regions, hydrophilic regions, hydrophobic regions, alpha-amphipathic regions, beta-amphipathic regions, flexible regions, surface-forming regions, substrate binding regions, and high antigenic index regions of the polypeptide according to the present invention, and combinations of such fragments. Preferred regions are those that mediate antigenicity and antibody binding activities of the polypeptides according to the present invention. Most highly preferred in this regard are fragments that have a chemical, biological or other activity of the antigen and fragments thereof of the present invention, including those with a similar activity or an improved activity, whereby such improved activities are immunogenicity and stability, or with a decreased undesirable activity, whereby such decreased undesirable activity is enzymatic and toxic function and generation of human cross-reactive antibodies. Particularly preferred are fragments comprising receptors or domains of enzymes that confer a function essential for viability of *Chlamydiae* or any other pathogenic *Chlamydia* species, or the ability to cause disease in humans. Further preferred fragments of the polypeptides according to the present invention are those that comprise or contain antigenic or immunogenic determinants in an animal, especially in a human. Such fragments are also referred to as antigenic fragment.

An antigenic fragment is preferably defined as a fragment, which is antigenic by itself or may be made antigenic when provided as a hapten. Therefore, also antigens or antigenic fragments showing one or, particularly for longer fragments, only a few amino acid exchanges are enabled by the present invention, provided that the antigenicity or antigenic capacities of such fragments with amino acid exchanges are not severely deteriorated on the exchange(s), i.e., suited for eliciting an appropriate immune response in an individual vaccinated with this antigen and identified by individual antibody preparations from individual sera.

Preferred examples of such fragments of the polypeptides according to the present invention are the core amino acid sequence as indicated in column "Predicted immunogenic aa" or "Predicted class 11-restricted T cell epitope/regions" or "Location of identified immunogenic region (aa)" of Table 1.

All these fragments listed in Table 1 individually and each independently form a preferred selected aspect of the present invention.

It will be appreciated that the invention also relates to, among others, nucleic acid molecules encoding the aforementioned fragments, variants, active variants, and active fragments, nucleic acid molecules that hybridise to nucleic acid molecules encoding the fragments, variants, active variants, and active fragments, particularly those that hybridise under stringent conditions, and nucleic acid molecules, such as PCR primers, for amplifying nucleic acid molecules that encode the fragments. In these regards, preferred nucleic acid molecules are those that correspond to the preferred fragments, as discussed above.

The polypeptides according to the present invention may be expressed in a modified form, such as a fusion protein, and may include not only secretion signals but also additional heterologous functional regions. Thus, for instance, a region of additional amino acids, particularly charged amino acids, may be added to the N- or C-terminus of the polypeptide to improve stability and persistence in the host cell, during purification or during subsequent handling and storage. Also, regions may be added to the polypeptide to facilitate purification or to enhance expression. Such regions may be removed prior to final preparation of the polypeptide. The addition of peptide moieties to polypeptides to engender secretion or excretion, to improve stability, to enhance expression or to facilitate purification, among others, are familiar and routine techniques in the art. A preferred fusion protein comprises a heterologous region from immunoglobulin that is useful to solubilize or purify polypeptides. For example, EP 0 464 533 discloses fusion proteins comprising various portions of constant region of immunoglobin molecules together with another protein or part thereof. In drug discovery, for example, proteins have been fused with antibody Fc portions for the purpose of high-throughout screening assays to identify antagonists. See for example, (Bennett, D. et al., 1995) and (Johanson, K. et al., 1995). Fusions also may include the polypeptides according to the present invention fused or coupled to moieties other than amino acids, including lipids and carbohydrates. Further, antigens of this invention may be employed in combination with other vaccinal agents described by the prior art, as well as with other species of vaccinal agents derived from other microorganisms. Such proteins are useful in the prevention, treatment and diagnosis of diseases caused by a wide spectrum of *Chlamydia* isolates.

In a further embodiment the peptide of the invention is fused to an epitope tag which provides an epitope to which an anti-tag substance can selectively bind. The epitope tag is generally placed at the amino- or carboxyl-terminus of the peptide but may be incorporated as an internal insertion or substitution as the biological activity permits. The presence of such epitope-tagged forms of a peptide can be detected using a substance such as an antibody against the tagged peptide. Also, provision of the epitope tag enables the peptide to be readily purified by affinity purification using an anti-tag antibody or another type of affinity matrix that binds to the epitope tag. Various tag polypeptides and their respective antibodies are well known in the art. Examples include poly-histidine (poly-his), poly-histidine-glycine (poly-his-gly) tags, the HA tag polypeptide, the c-myc tag, the Strep tag and the FLAG tag.

The polypeptides of the invention may be prepared by any of a number of conventional techniques. For example, they can be produced by chemical synthesis as well as by biotechnological means. The latter comprise the transfection or transformation of a host cell with a vector containing a nucleic acid according to the present invention. In a preferred embodiment the vector is a vector according to the present invention. The biotechnological production of the polypeptides according to the present invention further comprises the cultivation of the transfected or transformed host cell under conditions, that allow expression of the protein and which are known to the one skilled in the art. The expressed protein is recovered, isolated, and optionally purified from the cell (or from the culture medium, if expressed extracellularly) by appropriate means known to one of skill in the art. For example, the proteins are isolated in soluble form following cell lysis, or extracted using known techniques, e.g. in guanidine chloride. The molecules comprising the polypeptides and antigens of this invention may be further purified using any of a variety of conventional methods including, but not limited to: ammonium sulfate or ethanol precipitation, acid extraction, liquid chromatography such as normal or reversed phase, using HPLC, FPLC and the like; affinity chromatography (such as with inorganic ligands or monoclonal antibodies), size exclusion chromatography, anion or cation exchange chromatography, phosphocellulose chromatography, hydrophobic interaction chromatography, hydroxylapatite chromatography, lectin chromatography, immobilized metal chelate chromatography, gel electrophoresis, and the like. One of skill in the art may select the most appropriate isolation and purification techniques without departing from the scope of this invention. Such purification provides the antigen in a form substantially free from other proteinaceous and non-proteinaceous materials of the microorganism.

An alternative approach to prepare polypeptides according to the invention involves generating the fragments of known peptides by enzymatic digestion, e.g., by treating the protein with an enzyme known to cleave proteins at sites defined by particular amino acid residues, or by digesting the DNA with suitable restriction enzymes, expressing the digested DNA and isolating the desired fragment. Yet another suitable technique involves isolating and amplifying a DNA fragment encoding a desired peptide fragment, by polymerase chain reaction (PCR). Oligonucleotides that define the desired termini of the DNA fragment are employed as the 5' and 3' primers in the PCR. Techniques for making mutations, such as deletions, insertions and substitutions, at predetermined sites in DNA, and therefore in proteins, having a known sequence are well known. One of skill in the art using conventional techniques, such as PCR, may readily use the antigens and peptides provided herein to identify and isolate other similar proteins. Such methods are routine and not considered to require undue experimentation, given the information provided herein. For example, variations can be made using oligonucleotide-mediated site-directed mutagenesis (Carter, P. et al., 1985; Zoller, M.J. et al., 1987), cassette mutagenesis (Wells, J.A. et al., 1985), restriction selection mutagenesis (Wells, J.A. et al., 1986), PCR mutagenesis, or other known techniques can be performed on the cloned DNA to produce the peptide of the invention.

The polypeptide according to the present invention may be used for the detection of the organism or organisms in a sample containing these organisms or proteins or antigens, including fragments thereof. Preferably such detection is for diagnosis, more preferable for the diagnosis of a disease, most preferably for the diagnosis of a disease related or linked to the presence or abundance of Gram-negative bacteria, especially pathogenic *Chlamydia* species, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

The nucleic acids according to the present invention can also be used for the diagnosis or detection of organisms in a sample, whereby the organisms are preferably the same ones as disclosed in connection with the use of the polypeptides according to the present invention and the antibody according to the present invention, respectively. Basically, it is within the skills of the person of the art to design and practice such diagnosis and detection assays and methods, respectively, in the light of the present disclosure. More preferably such diagnosis or detection uses primers or probes to specifically interact with the nucleic acid molecules according to the present invention. The length and design of such primers and probes, respectively, varies depending on the particular method or diagnosis practiced. Using, in a preferred embodiment, a primer for, e.g., a PCR based detection or diagnosis system, i.e. method or assay, the length of the primer will range from about 10 nucleotides to about 30 nucleotides and more preferably from about 16 to 25 nucleotides. In case of a probe based detection or diagnosis system the length of the probe is preferably about the same as specified for the primer based system. Additionally, in case of a probe based system, the probe will comprise a moiety which allows its detection, either directly or indirectly. Such moiety for direct detection can be a radioactive label or a fluorescence label as known to the ones skilled in the art. Such moiety for indirect detection can be a biotin or any other moiety which mediates interaction with a further compound which in turn is labelled so as to allow its detection.

The present invention also relates to diagnostic assays, such as quantitative diagnostic assays for detecting levels of the polypeptides according to the present invention, and more preferably antigens and fragments thereof of the present invention, in cells and tissues, including determination of normal and abnormal levels. Thus, for instance, a diagnostic assay in accordance with the invention for detecting over-expression of the polypeptides according to the present invention compared to normal control tissue samples may be used to detect the presence of an infection, for example, and to identify the infecting organism. Assay techniques that can be used to determine levels of such polypeptides in a sample derived from a host are well known to those of skill in the art. Such assay methods include radioimmunoassays, competitive-binding assays, Western Blot analysis and ELISA assays. Among these, ELISA and Western Blot analysis frequently are preferred. An ELISA assay initially comprises preparing an antibody specific to one of the polypeptides according to the present invention, preferably a monoclonal antibody. In addition, a reporter antibody generally is prepared which binds to the monoclonal antibody. The reporter antibody is attached to a detectable reagent such as radioactive, fluorescent or enzymatic reagent, such as horseradish peroxidase enzyme. One or several of the polypeptides according to the present invention and more preferably an antigen and fragment thereof according to the present invention may be immobilised on ELISA plates for detection of reactive antibodies in sera of patients or subjects to be tested.

A Western blot assay initially separates the polypeptides according to the present invention individually or in combination by SDS-polyacrylamide gelelectrophoresis and which subsequently are transferred and immobilised onto a solid support matrix, such as nitrocellulose, nylon or combinations thereof. Together with a reporter antibody reactive antibodies can be detected. The reporter antibody is attached to a detectable reagent such as radioactive, fluorescent or enzymatic reagent, such as horseradish peroxidase enzyme.

The polypeptides according to the present invention or the nucleic acid molecules according to the present invention or primers or probes directed thereto as described herein, may also be used for the purpose of or in connection with an array. In case of the nucleic acid molecule according to the present invention and the primers and probes directed there against, the length of the probes and the primer, can also preferably be in the range from about 25 to about 75 nucleotides, more preferably from about 35 to about 50 nucleotides. More particularly, at least one of the polypeptides according to the present invention may be immobilized on a support. Said support typically comprises a variety of the polypeptides according to the present invention and/or antigens and fragments thereof whereby the variety may be created by using one or several of the antigens and fragments thereof according to the present invention and/or antigens and fragments thereof being different. The characterizing feature of such array as well as of any array in general is the fact that at a distinct or predefined region or position on said support or a surface thereof, a distinct polypeptide is immobilized. Because of this any activity at a distinct position or region of an array can be correlated with a specific polypeptide. The number of different polypeptides and more preferably different antigens and fragments thereof immobilized on a support may range from as little as 10 to several 1,000 different polypeptides and antigens and fragments thereof, respectively. The density of said molecules per cm² is in a preferred embodiment as little as 10 per cm² to at least 400 different of such polypeptides per cm² and more particularly at least 1,000 different of such polypeptides and more preferably different antigens and fragments thereof per cm². What is said herein about the immobilization of the polypeptides according to the present invention and their use, is also applicable to the nucleic acid molecules and the primers and probes, respectively, directed there against, as will be acknowledged by the ones skilled in the art.

The manufacture of such arrays is known to the one skilled in the art and, for example, described in US patent 5,744,309. The array preferably comprises a planar, porous or non-porous solid support having at least a first surface. The polypeptides according to the present invention are immobilized on said surface. Preferred support materials are, among others, glass or cellulose. It is also within the present invention that the array is used for any of the diagnostic applications described herein. Apart from the polypeptides according to the present invention also the nucleic acid molecules according to the present invention may be used for the generation of an array as described above which, in principle, can be used for any of the purposes disclosed for the array containing polypeptides. This applies as well to an array made of antibodies, preferably monoclonal antibodies as, among others, described herein.

In a further aspect the present invention relates to an antibody directed to any of polypeptides according to the present invention, derivatives, fragments, variants, active fragments and active variants thereof according to the present invention. The present invention includes, for example, monoclonal and polyclonal antibodies, chimeric, single chain, and humanized antibodies, as well as Fab fragments, or the product of a Fab expression library. It is within the present invention that the antibody may be chimeric, i.e. that different parts thereof stem from different species or at least the respective sequences are taken from different species.

Such antibodies in general and in particular directed against the antigens and fragments thereof corresponding to a sequence of the present invention can be obtained by direct injection of a polypeptide according to the present invention into an animal or by administering said polypeptide to an animal, preferably a non-human. The antibody so obtained will then bind said polypeptide itself. In this manner, even a sequence encoding only a fragment said polypeptide can be used to generate antibodies binding the whole native polypeptides according to the present invention. Such antibodies can then be used to isolate the polypeptide according to the present invention from tissue expressing antigens and fragments thereof. It will be understood by the ones skilled in the art that this procedure is also applicable to the fragments, variants, active fragments and active variants thereof of said polypeptides.

Another aspect of the present invention relates to methods for producing antibodies according to the invention. This includes, for example, monoclonal and polyclonal antibodies, chimeric, single chain, and humanized antibodies, as well as Fab fragments, or the product of a Fab expression library, which are able to specifically bind to the peptide or composition according to the invention.

In a preferred embodiment the antibody is a monoclonal, polyclonal, chimeric or humanized antibody or functionally active fragment thereof. In another preferred embodiment the functionally active fragment comprises a Fab fragment.

Antibodies generated against the peptide or antigen or composition according to the invention can be obtained by direct injection of the peptide or antigen or composition according to the invention into an animal or administering of the peptide or antigen or composition according to the invention to an animal, preferably a non-human. The antibody so obtained will then bind the peptide or antigen or composition according to the invention. Such antibodies can then be used to isolate reactive antigens, peptide or proteins from a tissue expressing those.

For preparation of monoclonal antibodies, any technique known in the art, which provides antibodies produced by continuous cell line cultures, can be used (as described originally in Köhler, G. et al., 1975).

Techniques described for the production of single chain antibodies (U.S. Patent No. 4,946,778) can be adapted to produce single chain antibodies to immunogenic antigens and fragments thereof in their diverse embodiments according to this invention. Also, transgenic mice, or other organisms such as other mammals, may be used to express humanized antibodies to the polypeptides according to the present invention.

Antibodies may also be produced using a hybridoma cell line.

Still another aspect of the invention relates to a hybridoma cell line which produces the antibody of the invention.

Hybridoma cell lines expressing desirable monoclonal antibodies are generated by well-known conventional techniques. The hybridoma cell can be generated by fusing a normal-activated, antibody-producing B cell with a myeloma cell. In the context of the present invention the hybridoma cell is able to produce an antibody specifically binding to the antigen of the invention.

Similarly, desirable high titre antibodies are generated by applying known recombinant techniques to the monoclonal or polyclonal antibodies developed to these antigens (see, e.g., PCT Patent Application No. PCT/GB85/00392; British Patent Application Publication No. GB2188638A; Amit, A.G. et al., 1986; Queen, C. et al., 1989; PCT Patent Application No. WO90/07861; Riechmann, L. et al., 1988; Huse, W.D. et al., 1988).

Alternatively, the antibody may be produced employing display libraries. For example, phage display technology or ribosomal display could be utilized to select antibody genes with binding activities towards the polypeptides according to the present invention either from repertoires of PCR amplified v-genes of lymphocytes from humans screened for possessing respective target antigen binding antibodies or from naive libraries (McCafferty, J. et al., 1990; Marks, J. et al., 1992). The affinity of these antibodies can also be improved by chain shuffling (Clackson, T. et al., 1991).

If two antigen binding domains are present, each domain may be directed against a different epitope - termed 'bispecific' antibodies.

The above-described antibodies may be employed to isolate or to identify clones expressing the polypeptides according to the present invention by attachment of the antibody to a solid support for isolation and/or purification by affinity chromatography.

Thus, among others, antibodies against the polypeptides according to the present invention may be employed to inhibit and/or treat infections, particularly bacterial infections and especially infections arising from pathogenic *Chlamydia* species, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

The polypeptides according to the present invention and more specifically antigens and fragments thereof in their diverse embodiments include antigenically, epitopically or immunologically equivalent derivatives, which form a particular aspect of this invention. The term "antigenically equivalent derivative" as used herein encompasses such polypeptide according to the present invention or its equivalent which will be specifically recognized by certain antibodies which, when raised to said polypeptide, interfere with the interaction between pathogen and mammalian host. The term "immunologically equivalent derivative" as used herein encompasses a peptide or its equivalent which when used in a suitable formulation to raise antibodies in a vertebrate, the antibodies act to interfere with the interaction between pathogen and mammalian host.

The polypeptides according to the present invention and more specifically the antigens and fragments thereof in their diverse embodiments, such as an antigenically or immunologically equivalent derivative or a fusion protein thereof can be used as an antigen to immunize a mouse or other animal such as a rat or chicken. The fusion protein may provide stability to the polypeptide according to the present invention. Such polypeptide may be associated, for example by conjugation, with an immunogenic carrier protein, for example bovine serum albumin (BSA) or keyhole limpet haemocyanin (KLH). Alternatively, an antigenic peptide comprising multiple copies of the polypeptide according to the present invention and more preferably an antigen and fragments thereof, or an antigenically or immunologically equivalent antigen and fragments thereof, may be sufficiently antigenic to improve immunogenicity so as to obviate the use of a carrier.

Preferably the antibody or derivative thereof is modified to make it less immunogenic in the individual. For example, if the individual is human the antibody may most preferably be "humanized", wherein the complementarity determining region(s) of the hybridoma-derived antibody has been transplanted into a human monoclonal antibody, for example as described in (Jones, P. et al., 1986) or (Tempest, P. et al., 1991).

The use of a nucleic acid molecule according to the present invention in genetic immunization will preferably employ a suitable delivery method such as direct injection of plasmid DNA into muscle, delivery of DNA complexed with specific protein carriers, coprecipitation of DNA with calcium phosphate, encapsulation of DNA in various forms of liposomes, particle bombardment (Tang, D. et al., 1992; Eisenbraun, M. et al., 1993) and *in vivo* infection using cloned retroviral vectors (Seeger, C. et al., 1984).

In a further aspect the present invention relates to a peptide binding to any of the polypeptides according to the present invention, and a method for the preparation of such peptides whereby the method is characterized by the use of said polypeptide and the basic steps are known to the one skilled in the art.

Such peptides may be generated by using methods according to the state of the art such as phage display or ribosome display. In case of phage display, basically a library of peptides is generated, in form of phages, and this kind of library is contacted with the target molecule, in the present case a polypeptide according to the present invention. Those peptides binding to the target molecule are subsequently removed, preferably as a complex with the target molecule, from the respective reaction. It is known to the one skilled in the art that the binding characteristics, at least to a certain extent, depend on the particularly realized experimental set-up such as the salt concentration and the like. After separating those peptides binding to the target molecule with a higher affinity or a bigger force, from the non-binding members of the library, and optionally also after removal of the target molecule from the complex of target molecule and peptide, the respective peptide(s) may subsequently be characterised. Prior to the characterisation optionally an amplification step is realized such as, e.g. by propagating the peptide encoding phages. The characterisation preferably comprises the sequencing of the target binding peptides. Basically, the peptides are not limited in their lengths, however preferably peptides having a length from about 8 to 20 amino acids are preferably obtained in the respective methods. The size of the libraries may be about 10² to 10¹⁸, preferably 10⁸ to 10¹⁵ different peptides, however, is not limited thereto. In a preferred embodiment such peptides are high-affinity binding peptides. In an even more preferred embodiment the peptides are peptide aptamers.

Peptide aptamers as used herein refer to peptide molecules that bind a specific target molecule. Peptide aptamers are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which have good solubility and compacity properties. Currently, the bacterial protein Thioredoxin-A is the most used scaffold protein, the variable loop being inserted within the reducing active site, which is a -Cys-Gly-Pro-Cys- loop in the wild protein, the two Cysteines lateral chains being able to form a disulfide bridge. Peptide aptamer selection can be made using different systems, but the most used is currently the yeast two-hybrid system. Selection of Ligand Regulated Peptide Aptamers (LiRPAs) has been demonstrated. By displaying 7 amino acid peptides from a novel scaffold protein based on the trimeric FKBP-rapamycin-FRB structure, interaction between the randomized peptide and target molecule can be controlled by the small molecule Rapamycin or non-immunosuppressive analogs.

A particular form of target binding peptides as described above, are the so-called "anticalines" which are, among others, described in German patent application DE 19742706. In so far, the present invention is also related to peptides specifically binding to the polypeptides according to the present invention and the use thereof for any of the therapeutic and diagnostic applications described herein, preferably for antibodies.

In a further aspect the present invention relates to functional nucleic acids interacting with any of the polypeptides according to the present invention, and a method for the preparation of such functional nucleic acids whereby the method is characterized by the use of the polypeptides according to the present invention and the basic steps are known to the one skilled in the art. The functional nucleic acids are preferably nucleic acid aptamers and spiegelmers. In so far, the present invention is also related to nucleic acid aptamers and spiegelmers specifically binding to the polypeptides according to the present invention and the use thereof for any of the therapeutic and diagnostic applications described herein, preferably for antibodies.

Nucleic acid aptamers are D-nucleic acids, which are either single stranded or double stranded and which specifically interact with a target molecule. The preparation or selection of aptamers is, e.g. described in European patent EP 0 533 838. Basically the following steps are realized. First, a mixture of nucleic acids, i.e. potential aptamers, is provided whereby each nucleic acid typically comprises a segment of several, preferably at least eight subsequent randomised nucleotides. This mixture is subsequently contacted with the target molecule whereby the nucleic acid(s) bind to the target molecule, such as based on an increased affinity towards the target or with a bigger force thereto, compared to the candidate mixture. The binding nucleic acid(s) are/is subsequently separated from the remainder of the mixture. Optionally, the thus obtained nucleic acid(s) is amplified using, e.g. polymerase chain reaction. These steps may be repeated several times giving at the end a mixture having an increased ratio of nucleic acids specifically binding to the target from which the final binding nucleic acid is then optionally selected. These specifically binding nucleic acid(s) are referred to as aptamers. It is obvious that at any stage of the method for the generation or identification of the aptamers samples of the mixture of individual nucleic acids may be taken to determine the sequence thereof using standard techniques. It is within the present invention that the aptamers may be stabilized such as, e.g., by introducing defined chemical groups which are known to the one skilled in the art of generating aptamers. Such modification may for example reside in the introduction of an amino group at the 2'-position of the sugar moiety of the nucleotides. Aptamers are currently used as therapeutic agents. However, it is also within the present invention that the thus selected or generated aptamers may be used for target validation and/or as lead substance for the development of pharmaceutical compositions, preferably of pharmaceutical compositions based on small molecules. This is actually done by a competition assay whereby the specific interaction between the target molecule and the aptamer is inhibited by a candidate drug whereby upon replacement of the aptamer from the complex of target and aptamer it may be assumed that the respective drug candidate allows a specific inhibition of the interaction between target and aptamer, and if the interaction is specific, said candidate drug will, at least in principle, be suitable to block the target and thus decrease its biological availability or activity in a respective system comprising such target. The thus obtained small molecule may then be subject to further derivatisation and modification to optimise its physical, chemical, biological and/or medical characteristics such as toxicity, specificity, biodegradability and bioavailability.

Spiegelmers and their generation or preparation is based on a similar principle. The preparation of spiegelmers is described in international patent application WO 98/08856. Spiegelmers are L-nucleic acids, which means that they are composed of L-nucleotides rather than D-nucleotides, as aptamers are. Spiegelmers are characterized by the fact that they have a very high stability in biological systems and, comparable to aptamers, specifically interact with the target molecule against which they are directed. In the process of generating spiegelmers, a heterogeneous population of D-nucleic acids is created and this population is contacted with the optical antipode of the target molecule, in the present case for example with the D-enantiomer of the naturally occurring L-enantiomer of the antigens and fragments thereof according to the present invention. Subsequently, those D-nucleic acids are separated which do not interact with the optical antipode of the target molecule. But those D-nucleic acids interacting with the optical antipode of the target molecule are separated, optionally identified and/or sequenced and subsequently the corresponding L-nucleic acids are synthesized based on the nucleic acid sequence information obtained from the D-nucleic acids. These L-nucleic acids, which are identical in terms of sequence with the aforementioned D-nucleic acids interacting with the optical antipode of the target molecule, will specifically interact with the naturally occurring target molecule rather than with the optical antipode thereof. Similar to the method for the generation of aptamers it is also possible to repeat the various steps several times and thus to enrich those nucleic acids specifically interacting with the optical antipode of the target molecule.

In a further aspect the present invention relates to functional nucleic acids interacting with any of the nucleic acid molecules according to the present invention, and a method for the preparation of such functional nucleic acids whereby the method is characterized by the use of the nucleic acid molecules and their respective sequences according to the present invention and the basic steps are known to the one skilled in the art. The functional nucleic acids are preferably ribozymes, antisense oligonucleotides and siRNA. In so far, the present invention is also related to this kind of functional nucleic acid specifically binding to the polypeptides according to the present invention and the use thereof for any of the therapeutic and diagnostic applications described herein, preferably for antibodies.

Ribozymes are catalytically active nucleic acids, which preferably consist of RNA, which basically comprises two moieties. The first moiety shows a catalytic activity whereas the second moiety is responsible for the specific interaction with the target nucleic acid, in the present case the nucleic acid coding for the polypeptides according to the present invention. Upon interaction between the target nucleic acid and the second moiety of the ribozyme, typically by hybridisation and Watson-Crick base pairing of essentially complementary stretches of bases on the two hybridising strands, the catalytically active moiety may become active which means that it catalyses, either intramolecularly or intermolecularly, the target nucleic acid in case the catalytic activity of the ribozyme is a phosphodiesterase activity. Subsequently, there may be a further degradation of the target nucleic acid, which in the end results in the degradation of the target nucleic acid as well as the protein derived from the said target nucleic acid. Ribozymes, their use and design principles are known to the one skilled in the art, and, for example described in (Doherty, E. et al., 2001) and (Lewin, A. et al., 2001).

The activity and design of antisense oligonucleotides for the preparation of a pharmaceutical composition and as a diagnostic agent, respectively, is based on a similar mode of action. Basically, antisense oligonucleotides hybridise based on base complementarity, with a target RNA, preferably with a mRNA, thereby activating RNase H. RNase H is activated by both phosphodiester and phosphorothioate-coupled DNA. Phosphodiester-coupled DNA, however, is rapidly degraded by cellular nucleases with the exception of phosphorothioate-coupled DNA. These resistant, non-naturally occurring DNA derivatives do not inhibit RNase H upon hybridisation with RNA. In other words, antisense polynucleotides are only effective as DNA RNA hybrid complexes. Examples for this kind of antisense oligonucleotides are described, among others, in US patents US 5,849,902 and US 5,989,912. In other words, based on the nucleic acid sequence of the target molecule which in the present case are the nucleic acid molecules for the antigens and fragments thereof according to the present invention, either from the target protein from which a respective nucleic acid sequence may in principle be deduced, or by knowing the nucleic acid sequence as such, particularly the mRNA, suitable antisense oligonucleotides may be designed base on the principle of base complementarity.

Particularly preferred are antisense-oligonucleotides, which have a short stretch of phosphorothioate DNA (3 to 9 bases). A minimum of 3 DNA bases is required for activation of bacterial RNase H and a minimum of 5 bases is required for mammalian RNase H activation. In these chimeric oligonucleotides there is a central region that forms a substrate for RNase H that is flanked by hybridising "arms" comprised of modified nucleotides that do not form substrates for RNase H. The hybridising arms of the chimeric oligonucleotides may be modified such as by 2'-O-methyl or 2'-fluoro. Alternative approaches used methylphosphonate or phosphoramidate linkages in said arms. Further embodiments of the antisense oligonucleotide useful in the practice of the present invention are P-methoxyoligonucleotides, partial P-methoxyoligodeoxyribonucleotides or P-methoxyoligodeoxy-ribonucleotides.

Of particular relevance and usefulness for the present invention are those antisense oligonucleotides as more particularly described in the above two mentioned US patents. These oligonucleotides contain no naturally occurring 5'=>3' -linked nucleotides. Rather the oligonucleotides have two types of nucleotides: 2'-deoxyphosphorothioate, which activate RNase H, and 2'-modified nucleotides, which do not. The linkages between the 2'-modified nucleotides can be phosphodiesters, phosphorothioate or P-ethoxyphosphodiester. Activation of RNase H is accomplished by a contiguous RNase H-activating region, which contains between 3 and 5 2'-deoxyphosphorothioate nucleotides to activate bacterial RNase H and between 5 and 10 2'-deoxyphosphorothioate nucleotides to activate eukaryotic and, particularly, mammalian RNase H. Protection from degradation is accomplished by making the 5' and 3' terminal bases highly nuclease resistant and, optionally, by placing a 3' terminal blocking group.

More particularly, the antisense oligonucleotide comprises a 5' terminus and a 3' terminus; and from position 11 to 59 5'=>3' -linked nucleotides independently selected from the group consisting of 2'-modified phosphodiester nucleotides and 2'-modified P-alkyloxyphosphotriester nucleotides; and wherein the 5'-terminal nucleoside is attached to an RNase H-activating region of between three and ten contiguous phosphorothioate-linked deoxyribonucleotides, and wherein the 3'-terminus of said oligonucleotide is selected from the group consisting of an inverted deoxyribonucleotide, a contiguous stretch of one to three phosphorothioate 2'-modified ribonucleotides, a biotin group and a P-alkyloxyphosphotriester nucleotide.

Also an antisense oligonucleotide may be used wherein not the 5' terminal nucleoside is attached to an RNase H-activating region but the 3' terminal nucleoside as specified above. Also, the 5' terminus is selected from the particular group rather than the 3' terminus of said oligonucleotide.

The nucleic acids as well as the polypeptides according to the present invention in their diverse embodiments may be used as or for the preparation of pharmaceutical compositions, especially vaccines. Preferably such pharmaceutical composition, preferably vaccine is, for the prevention or treatment of diseases caused by, related to or associated with *Chlamydia* species, preferably pathogenic *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.* In so far another aspect of the invention relates to a method for inducing an immunological response in an individual, particularly a mammal, which comprises inoculating the individual with the polypeptides according to the present invention in their diverse embodiments, or a fragment or variant thereof, adequate to produce antibodies to protect said individual from infection by the above microorganisms.

Yet another aspect of the invention relates to a method of inducing an immunological response in an individual which comprises, through gene therapy or otherwise, delivering a nucleic acid molecule according to the present invention, preferably functionally encoding antigens and fragments thereof in their diverse embodiments, for expressing the polypeptide according to the present invention *in vivo* in order to induce an immunological response to produce antibodies or a cell mediated T cell response, either cytokine-producing T cells or cytotoxic T cells, to protect said individual from disease, whether that disease is already established within the individual or not. One-way of administering the gene is by accelerating it into the desired cells as a coating on particles or otherwise.

A further aspect of the invention relates to an immunological composition which, when introduced into a host capable of having induced within it an immunological response, induces an immunological response in such host, wherein the composition comprises recombinant DNA which codes for and expresses at least one of the polypeptides according to the present invention in their diverse embodiments. The immunological response may be used therapeutically or prophylactically and may take the form of antibody immunity or cellular immunity such as that arising from CTL or CD4+ T cells.

The polypeptides according to the present invention in their diverse embodiments may be fused with a co-protein which may not by itself produce antibodies, but is capable of stabilizing the first protein and producing a fused protein which will have immunogenic and protective properties. This fused recombinant protein preferably further comprises an antigenic co-protein, such as Glutathione-S-transferase (GST) or beta-galactosidase, relatively large co-proteins which solubilise the protein and facilitate production and purification thereof. Moreover, the co-protein may act as an adjuvant in the sense of providing a generalized stimulation of the immune system. The co-protein may be attached to either the amino or carboxy terminus of the first protein.

Also provided by this invention are methods using the nucleic acid molecule according to the present invention in their diverse embodiments in such genetic immunization experiments in animal models of infection with any of the *Chlamydia* species described herein, preferably *C. pneumoniae, C. trachomatis, C. psittaci, C. pecorum, C. muridarum,* or *C*. *suis,* and more preferably *C*. *pneumoniae, C. trachomatis* or *C. psittaci.* Such molecules will be particularly useful for identifying protein epitopes able to provoke a prophylactic or therapeutic immune response. This approach can allow for the subsequent preparation of monoclonal antibodies of particular value from the requisite organ of the animal successfully resisting or clearing infection for the development of prophylactic agents or therapeutic treatments of the *Chlamydia* species described herein, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci* infection in mammals, particularly humans.

The polypeptides according to the present invention in their diverse embodiments may be used as an antigen for vaccination of a host to produce specific antibodies which protect against invasion of bacteria, for example by blocking adherence of bacteria to damaged tissue. Examples of tissue damage and thus damaged tissue include wounds in skin or connective tissue and mucosal tissues caused e.g. by viral infection (esp. respiratory, such as the flu) mechanical, chemical or thermal damage or by implantation of indwelling devices, or wounds in the mucous membranes, such as the mouth, mammary glands, urethra or vagina.

The present invention also includes a vaccine formulation, which comprises one or several of polypeptides according to the present invention in their diverse embodiments together with one or more suitable carriers and/or excipients. The pharmaceutically acceptable carriers and/or excipients useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the (poly)peptides herein disclosed. In general, the nature of the carrier or excipients will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

Since said polypeptides according to the present invention may be broken down in the stomach, they are preferably administered parenterally, including, for example, administration that is subcutaneous, intramuscular, intravenous, intradermal, intranasal or transdermal. Formulations suitable for parenteral administration include aqueous and non-aqueous sterile injection solutions which may contain anti-oxidants, buffers, bacteriostats and solutes which render the formulation isotonic with the body fluid, preferably the blood, of the individual; and aqueous and non-aqueous sterile suspensions which may include suspending agents or thickening agents. The formulations may be presented in unit-dose or multi-dose containers, for example, sealed ampoules and vials, and may be stored in a freeze-dried condition requiring only the addition of the sterile liquid carrier immediately prior to use. The vaccine formulation may also include adjuvant systems for enhancing the immunogenicity of the formulation, such as oil-in-water systems and other systems known in the art. The dosage will depend on the specific activity of the vaccine and can be readily determined by routine experimentation.

According to another aspect, the present invention relates to a pharmaceutical composition comprising one or several of the polypeptides according to the present invention in their diverse embodiments for the various *Chlamydia* species described herein, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.* Such a pharmaceutical composition may comprise one, preferably at least two or more of said polypeptides against said *Chlamydia* species. Optionally, such polypeptides may also be combined with antigens against even further pathogens in a combination pharmaceutical composition. Preferably, said pharmaceutical composition is a vaccine for preventing or treating an infection caused by a *Chlamydia* species, more preferably a pathogenic *Chlamydia* species such as *C*. *pneumoniae, C. trachomatis, C. psittaci, C. pecorum, C. muridarum,* or *C*. *suis,* and more preferably *C*. *pneumoniae, C. trachomatis* or *C*. *psittaci,* and/or other pathogens against which the antigens have been included in the vaccine.

According to a further aspect, the present invention relates to a pharmaceutical composition comprising a nucleic acid molecule according to the present invention. Such a pharmaceutical composition may comprise one or more nucleic acid molecules according to the present invention encoding a polypeptide according to the present invention. Optionally, such nucleic acid molecules encoding the polypeptides according to the present invention are combined with nucleic acid molecules encoding antigens against other pathogens in a combination pharmaceutical composition. Preferably, said pharmaceutical composition is a vaccine for preventing or treating an infection caused by *Chlamydia* species, more preferably pathogenic *Chlamydia* species as disclosed herein, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci* and/or other pathogens against which the antigens have been included in the vaccine.

The pharmaceutical composition may contain any suitable auxiliary substances, such as buffer substances, stabilisers or further active ingredients, especially ingredients known in connection of pharmaceutical composition and/or vaccine production.

In a preferred embodiment the pharmaceutical composition further comprises an immunostimulatory substance such as an adjuvant. The adjuvant can be selected based on the method of administration and may include polycationic substances, especially polycationic peptides, immunostimulatory nucleic acids molecules, preferably immunostimulatory oligo-deoxynucleotides (ODNs), especially Oligo(dIdC)₁₃, peptides containing at least two LysLeuLys motifs, especially peptide KLKLLLLLKLK, alum, mineral oil-based adjuvants such as Freund's complete adjuvant, Freund's incomplete adjuvant, neuroactive compounds, especially human growth hormone, or any combination of one or more of the above mentioned adjuvants. Other suitable adjuvants may be selected from the group consisting of Montanide incomplete Seppic adjuvant such as ISA, oil in water emulsion adjuvants such as the Ribi adjuvant system, syntax adjuvant formulation containing muramyl dipeptide, or aluminum salt adjuvants or combinations thereof.

The term "Oligo(dIdC)₁₃" as used in the present invention means a phosphodiester backboned single-stranded DNA molecule containing 13 deoxy (inosine-cytosine) motifs, also defined by the term [oligo-d(IC)₁₃]. The exact sequence is 5'-dIdCdIdCdIdCdIdCdIdCdIdCdIdCdIdCdIdCdIdCdIdCdIdCdIdC-3'. Oligo(dIdC)₁₃ can also be defined by the terms (oligo-dIC₂₆); oligo-dIC₂₆₋ₘₑᵣ; oligo-deoxy IC, 26-mer; or oligo-dIC, 26-mer, as specified for example in WO 01/93903 and WO 01/93905.

It is also within the scope of the present invention that the pharmaceutical composition, especially a vaccine, comprises apart from one or several of the polypeptides according to the present invention in their diverse embodiments, and/or nucleic acid molecules in accordance with the present invention other compounds which are biologically or pharmaceutically active. Preferably, the vaccine composition comprises at least one polycationic peptide. The polycationic compound(s) to be used according to the present invention may be any polycationic compound, which shows the characteristic effects according to the WO 97/30721. Preferred polycationic compounds are selected from basic polypeptides, organic polycations, basic polyamino acids or mixtures thereof. These polyamino acids should have a chain length of at least 4 amino acid residues (WO 97/30721). Especially preferred are substances like polylysine, polyarginine and polypeptides containing more than 20%, especially more than 50% of basic amino acids in a range of more than 8, especially more than 20, amino acid residues or mixtures thereof. Other preferred polycations and their pharmaceutical compositions are described in WO 97/30721 (e.g. polyethyleneimine) and WO 99/38528. Preferably these polypeptides contain between 20 and 500 amino acid residues, especially between 30 and 200 residues.

These polycationic compounds may be produced chemically or recombinantly or may be derived from natural sources.

Cationic (poly)peptides may also be anti-microbial with properties as reviewed in (Ganz, T., 1999). These (poly)peptides may be of prokaryotic or animal or plant origin or may be produced chemically or recombinantly (WO 02/13857). Peptides may also belong to the class of defensins (WO 02/13857). Sequences of such peptides can be, for example, found in the Antimicrobial Sequences Database under the following internet address:
http://www.bbcm.univ.trieste.it/~tossi/pag2.html.

Such host defence peptides or defensives are also a preferred form of the polycationic polymer according to the present invention. Generally, a compound allowing as an end product activation (or down-regulation) of the adaptive immune system, preferably mediated by APCs (including dendritic cells) is used as polycationic polymer.

Especially preferred for use as polycationic substances in the present invention are cathelicidin derived antimicrobial peptides or derivatives thereof (International patent application WO 02/13857, incorporated herein by reference), especially antimicrobial peptides derived from mammalian cathelicidin, preferably from human, bovine or mouse.

Polycationic compounds derived from natural sources include HIV-REV or HIV-TAT (derived cationic peptides, antennapedia peptides, chitosan or other derivatives of chitin) or other peptides derived from these peptides or proteins by biochemical or recombinant production. Other preferred polycationic compounds are cathelin or related or derived substances from cathelin. For example, mouse cathelin is a peptide, which has the amino acid sequence NH₂-RLAGLLRKGGEKIGEKLKKIGOKIKNFFQKLVPQPE-COOH. Related or derived cathelin substances contain the whole or parts of the cathelin sequence with at least 15-20 amino acid residues. Derivations may include the substitution or modification of the natural amino acids by amino acids, which are not among the 20 standard amino acids. Moreover, further cationic residues may be introduced into such cathelin molecules. These cathelin molecules are preferred to be combined with the antigen. These cathelin molecules surprisingly have turned out to be also effective as an adjuvant for an antigen without the addition of further adjuvants. It is therefore possible to use such cathelin molecules as efficient adjuvants in vaccine formulations with or without further immunactivating substances.

Another preferred polycationic substance to be used in accordance with the present invention is a synthetic peptide containing at least 2 KLK-motifs separated by a linker of 3 to 7 hydrophobic amino acids (International patent application WO 02/32451, incorporated herein by reference).

The pharmaceutical composition of the present invention may further comprise immunostimulatory nucleic acid(s). Immunostimulatory nucleic acids are e.g. neutral or artificial CpG containing nucleic acids, short stretches of nucleic acids derived from non-vertebrates or in form of short oligonucleotides (ODNs) containing non-methylated cytosine-guanine di-nucleotides (CpG) in a certain base context (e.g. described in WO 96/02555). Alternatively, also nucleic acids based on inosine and cytidine as e.g. described in the WO 01/93903, or deoxynucleic acids containing deoxy-inosine and/or deoxyuridine residues (described in WO 01/93905 and WO 02/095027, incorporated herein by reference) may preferably be used as immunostimulatory nucleic acids in connection with the present invention. Preferably, the mixtures of different immunostimulatory nucleic acids may be used according to the present invention.

It is also within the present invention that any of the aforementioned polycationic compounds is combined with any of the immunostimulatory nucleic acids as aforementioned. Preferably, such combinations are according to the ones as described in WO 01/93905, WO 02/32451, WO 01/54720, WO 01/93903, WO 02/13857 and WO 02/095027 and WO 03/047602, incorporated herein by reference.

In addition or alternatively such vaccine composition may comprise apart from the polypeptides according to the present invention and the nucleic acid molecules according to the present invention, preferably the coding nucleic acid molecules according to the present invention, a neuroactive compound. Preferably, the neuroactive compound is human growth factor as, e.g. described in WO 01/24822. Also preferably, the neuroactive compound is combined with any of the polycationic compounds and/or immunostimulatory nucleic acids as afore-mentioned.

Also, the pharmaceutical composition in accordance with the present invention is a pharmaceutical composition which comprises at least any of the following compounds or combinations thereof: the nucleic acid molecules according to the present invention, the polypeptides according to the present invention in their diverse embodiments, the vector according to the present invention, the cells according to the present invention, the antibody according to the present invention, the functional nucleic acids according to the present invention and the binding peptides such as the anticalines and high-affinity binding peptides and peptide aptamers, respectively, according to the present invention, any agonists and antagonists according to the present invention, preferably screened as described herein. In connection therewith any of these compounds may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of an antigen and fragments thereof of the invention and a pharmaceutically acceptable carrier or excipient. Such carriers may include, but are not limited to, saline, buffered saline, dextrose, water, glycerol, ethanol and combinations thereof. The formulation should suit the mode of administration.

The composition may be used e.g. for immunization or treatment of a subject. The pharmaceutical composition encompasses at least one peptide of the invention; however, it may also contain a cocktail (i.e., a simple mixture) containing different peptides (including fragments and other variants) of the invention, optionally mixed with different antigenic proteins or peptides of other pathogens. Such mixtures of these peptides, polypeptides, proteins or fragments or variants thereof are useful e.g. in the generation of desired antibodies to a wide spectrum of *Chlamydia* isolates. The peptide(s) of the present invention may also be used in the form of a pharmaceutically acceptable salt. Suitable acids and bases which are capable of forming salts with the peptides of the present invention are well known to those of skill in the art, and include inorganic and organic acids and bases.

Still another aspect of the present invention is a pharmaceutical composition containing a nucleic acid selected from the group consisting of:
(i) a nucleic acid of the invention and/or a nucleic acid complementary thereto, and
(ii) optionally a pharmaceutically acceptable carrier or excipient.

The nucleic acid sequences, alone or in combination with other nucleic acid sequences encoding antigens or antibodies or directed to other pathogenic microorganisms, may further be used as components of a pharmaceutical composition. The composition may be used for immunizing or treating humans and/or animals with the disease caused by infection with *Chlamydia,* preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.* The pharmaceutically acceptable carrier or excipient may be as defined above.

In another embodiment, the nucleic acid sequences of this invention, alone or in combination with nucleic acid sequences encoding other antigens or antibodies from other pathogenic microorganisms, may further be used in compositions directed to actively induce a protective immune response to the pathogen in a subject. These components of the present invention are useful in methods for inducing a protective immune response in humans and/or animals against infection with *Chlamydia,* preferably *C. pneumoniae, C. trachomatis, C. psittaci, C. pecorum, C. muridarum,* or *C*. *suis,* and more preferably *C. pneumoniae, C. trachomatis* or *C. psittaci.*

For use in the preparation of the therapeutic or vaccine compositions, nucleic acid delivery compositions and methods are useful, which are known to those of skill in the art. The nucleic acid of the invention may be employed in the methods of this invention or in the compositions described herein as DNA sequences, either administered as naked DNA, or associated with a pharmaceutically acceptable carrier and provide for *in vivo* expression of the antigen, peptide or polypeptide. So-called "naked DNA" may be used to express the antigen, peptide or polypeptide of the invention *in vivo* in a patient. (See, e.g., Cohen, J., 1993, which describes similar uses of "naked DNA"). For example, "naked DNA" associated with regulatory sequences may be administered therapeutically or as part of the vaccine composition e.g., by injection.

Alternatively, a nucleic acid encoding the antigens or peptides of the invention or a nucleic acid complementary thereto may be used within a pharmaceutical composition, e.g. in order to express the antigens or peptides or polypeptides of the invention *in vivo,* e.g., to induce antibodies.

A preferred embodiment of the invention relates to a pharmaceutical composition, wherein the nucleic acid according to the invention is comprised in a vector and/or a cell. Vectors and cells suitable in the context of the present invention are described above. Vectors are particularly employed for a DNA vaccine. An appropriate vector for delivery may be readily selected by one of skill in the art. Exemplary vectors for *in vivo* gene delivery are readily available from a variety of academic and commercial sources, and include, e.g., adeno-associated virus (International patent application No. PCT/US91/03440), adenovirus vectors (Kay, M. et al., 1994; Ishibashi, S. et al., 1993), or other viral vectors, e.g., various poxviruses, vaccinia, etc.. Recombinant viral vectors, such as retroviruses or adenoviruses, are preferred for integrating the exogenous DNA into the chromosome of the cell.

The pharmaceutical compositions of the present invention may be administered in any effective, convenient manner including, for instance, administration by topical, oral, anal, vaginal, intravenous, intraperitoneal, intramuscular, subcutaneous, intranasal, intratracheal or intradermal routes among others.

In therapy or as a prophylactic, the active agent of the pharmaceutical composition of the present invention may be administered to an individual as an injectable composition, for example as a sterile aqueous dispersion, which is preferably isotonic.

Preferably, the pharmaceutical composition of the present invention may be administered intranasally, e.g. as an aerosol formulation.

In general, intranasal vaccination represents an attractive non-invasive alternative to needle-based injection and provides superior protection at mucosal surfaces. Furthermore, mucosal as well as systemic immunity can be induced after intranasal immunizations.

It has been shown in the studies of the above mentioned publication of Rodriguez A. et al., (2006) that intranasal immunizations with DNA vaccines provided protection against *C*. *pneumoniae* infection in mice. Also, intranasal immunization with a protein vaccine induced a local immune response in the respiratory tract of the mice.

Therefore, intranasal application is particularly suited for *Chlamydiae* vaccines according to the present invention, especially for *C*. *pneumoniae* vaccines.

Alternatively the composition, preferably the pharmaceutical composition may be formulated for topical application, for example in the form of ointments, creams, lotions, eye ointments, eye drops, ear drops, mouthwash, impregnated dressings and sutures and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

In addition to the therapy described above, the compositions of this invention may be used generally as a wound treatment agent to prevent adhesion of bacteria to matrix proteins exposed in wound tissue and for prophylactic use in dental treatment as an alternative to, or in conjunction with, antibiotic prophylaxis.

In a preferred embodiment the pharmaceutical composition is a vaccine composition. Preferably, such vaccine composition is conveniently in injectable form or in an aerosol formulation for intranasal delivery. Conventional adjuvants may be employed to enhance the immune response. A suitable unit dose for vaccination with a protein antigen is for adults between 0.02 to 3 µg antigen / per kg of body weight and for children between 0.2 to 10 µg antigen / per kg body weight, and such dose is preferably administered 1-3 times and with an interval of 2 to 24 weeks.

An "effective amount" or "therapeutically effective amount" of an antigen, nucleic acid, vector, an antibody or a pharmaceutical composition of the invention may be calculated as that amount capable of exhibiting an *in vivo* effect, e.g. preventing or ameliorating a sign or symptom of infection with *Chlamydia,* preferably *C. pneumoniae, C. trachomatis, C. psittaci, C. pecorum, C. muridarum,* or *C*. *suis,* and more preferably *C. pneumoniae, C. trachomatis* or *C. psittaci.* Such amounts may be determined by one of skill in the art.

With the indicated dose range, no adverse toxicological effects are expected with the compounds of the invention, which would preclude their administration to suitable individuals.

In a further embodiment the present invention relates to diagnostic and pharmaceutical packs and kits comprising one or more containers filled with one or more of the ingredients of the aforementioned compositions of the invention. The ingredient(s) can be present in a useful amount, dosage, formulation or combination. Associated with such container(s) can be a notice in the form prescribed by a governmental agency regulating the preparation, use or sale of pharmaceuticals or biological products, reflecting approval by the agency of the preparation, use or sale of the product for human administration.

In connection with the present invention any disease related use as disclosed herein such as, e.g., use of the pharmaceutical composition or vaccine, is particularly a disease or diseased condition which is caused by, linked or associated with *Chlamydia,* more preferably any pathogenic *Chlamydia* species, preferably *C. pneumoniae, C. trachomatis, C. psittaci, C. pecorum, C. muridarum,* or *C*. *suis,* and more preferably *C. pneumoniae, C. trachomatis* or *C. psittaci.* A disease related, caused or associated with the bacterial infection to be prevented and/or treated according to the present invention includes chronic infections. Common sites include the upper and lower respiratory tract. The spectrum of clinical syndromes includes pneumonia, upper respiratory tract disease, bronchitis, sinusitis, asthmatic bronchitis, adult-onset asthma, and chronic obstructive pulmonary disease. In addition, *Chlamydia* infection has also been associated with atherosclerosis and cardiovascular disease as well as Alzheimer's disease.

It is within the present invention that each and any of the symptoms, diseases, disorders or syndromes described herein which are either directly or indirectly linked to or arise from a contact of an organism such as any animal or human with a *Chlamydia* species, preferably a pathogenic *Chlamydia* species, preferably *C. pneumoniae, C. trachomatis, C. psittaci, C. pecorum, C. muridarum,* or *C*. *suis,* and more preferably *C. pneumoniae, C. trachomatis* or *C. psittaci* are separately and independently indications, diseases or disorders in the meaning of the present invention. Accordingly and just by means of illustration, a disease in the sense of the present application is pneumonia as well as bronchitis and chronic obstructive pulmonary disease.

It is within the present invention that the disease for which the various compounds described herein can be used are also those diseases where the polypeptide according to the present invention is expressed or any disease where the compounds described herein such as the polypeptides according to the present invention, the vaccine, the antibody, and any aptamer and spiegelmer, respectively, are suitable for the treatment and/or diagnosis thereof. Such potential use can arise from cross-reactivity and homology, respectively. It understood by the one skilled in the art that any disease described in connection with the pharmaceutical composition according to the present invention can be subject to the use of the pharmaceutical composition described herein, and *vice versa.*

Treatment in the context of the present invention refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

In a still further embodiment the present invention is related to a screening method using any of the polypeptides according to the present invention or any of the nucleic acids according to the present invention. Screening methods as such are known to the one skilled in the art and can be designed such that an agonist or an antagonist is screened. In connection with such screening method preferably an antagonist is screened which in the present case inhibits or prevents the binding of any antigen and fragment thereof according to the present invention to an interaction partner. Such interaction partner can be a naturally occurring interaction partner or a non-naturally occurring interaction partner.

The invention also provides a method for screening compounds to identify those, which enhance (agonist) or block (antagonist) the function of the polypeptides according to the present invention or of the nucleic acid molecules of the present invention, such as its interaction with a binding molecule. The method of screening may involve high-throughput.

For example, to screen for agonists or antagonists, the interaction partner of the polypeptide according to the present invention, maybe a synthetic reaction mix, a cellular compartment, such as a membrane, cell envelope or cell wall, or a preparation of any thereof, may be prepared from a cell that expresses a molecule that binds to the polypeptides according to the present invention. The preparation is incubated with labelled forms of such polypeptides in the absence or the presence of a candidate molecule, which may be an agonist or antagonist. The ability of the candidate molecule to bind the binding molecule is reflected in decreased binding of the labelled ligand. Molecules which bind gratuitously, i.e., without inducing the functional effects of said polypeptides, are most likely to be good antagonists. Molecules that bind well and elicit functional effects that are the same as or closely related to the polypeptides according to the present invention are good agonists.

The functional effects of potential agonists and antagonists may be measured, for instance, by determining the activity of a reporter system following interaction of the candidate molecule with a cell or appropriate cell preparation, and comparing the effect with that of polypeptides according to the present invention or molecules that elicit the same effects as said polypeptides. Reporter systems that may be useful in this regard include but are not limited to colorimetric labelled substrate converted into product, a reporter gene that is responsive to changes in the functional activity of the polypeptides according to the present invention, and binding assays known in the art.

Another example of an assay for antagonists is a competitive assay that combines the polypeptides according to the present invention and a potential antagonist with membrane-bound binding molecules, recombinant binding molecules, natural substrates or ligands, or substrate or ligand mimetics, under appropriate conditions for a competitive inhibition assay. The polypeptides according to the present invention can be labelled such as by radioactivity or a colorimetric compound, such that the molecule number of polypeptides according to the present invention bound to a binding molecule or converted to product can be determined accurately to assess the effectiveness of the potential antagonist.

Potential antagonists include small organic molecules, peptides, polypeptides and antibodies that bind to polypeptides according to the present invention and thereby inhibit or extinguish its activity. Potential antagonists may also be small organic molecules, peptides, polypeptides such as a closely related proteins or antibodies that bind to the same sites on a binding molecule without inducing functional activity of the polypeptides according to the present invention.

Potential antagonists include a small molecule, which binds to and occupies the binding site of the polypeptides according to the present invention thereby preventing binding to cellular binding molecules, such that normal biological activity is prevented. Examples of small molecules include but are not limited to small organic molecules, peptides or peptide-like molecules.

Other potential antagonists include antisense molecules (see (Okano, H. et al., 1991); OLIGODEOXYNUCLEOTIDES AS ANTISENSE INHIBITORS OF GENE EXPRESSION; CRC Press, Boca Raton, FL (1988), for a description of these molecules).

Preferred potential antagonists include derivatives of the antigens and fragments thereof of the invention.

As used herein the activity of a polypeptide according to the present invention is its capability to bind to any of its interaction partner or the extent of such capability to bind to its or any interaction partner.

In a particular aspect, the invention provides the use of the polypeptides according to the present invention antigens and fragments thereof, nucleic acid molecules or inhibitors of the invention to interfere with the initial physical interaction between a pathogen and mammalian host responsible for sequelae of infection. In particular the molecules of the invention may be used: i) in the prevention of adhesion of the *Chlamydia* species as disclosed herein, and more preferably the pathogenic species thereof, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci* to mammalian extracellular matrix proteins; ii) to block bacterial adhesion between mammalian extracellular matrix proteins and bacterial proteins which mediate tissue reaction; iii) or lead to evasion of immune defence; iv) to block the normal progression of pathogenesis in infections initiated other than by the implantation of in-dwelling devices or by other surgical techniques, e.g. through inhibiting nutrient acquisition.

Each of the DNA coding sequences provided herein may be used in the discovery, development and/or preparation of antibacterial compounds. The encoded protein upon expression can be used as a target for the screening of antibacterial drugs. Additionally, the DNA sequences encoding the amino terminal regions of the encoded protein or Shine-Delgarno or other translation facilitating sequences of the respective mRNA can be used to construct antisense sequences to control the expression of the coding sequence of interest.

The antagonists and agonists may be employed, for instance, to inhibit diseases arising from infection with *Chlamydia* species, preferably *C*. *pneumoniae, C. trachomatis, C. psittaci, C. pecorum, C. muridarum,* or *C*. *suis,* and more preferably *C*. *pneumoniae, C. trachomatis* or *C*. *psittaci.*

In a still further aspect the present invention is related to an affinity device. Such affinity device comprises as least a support material and any of the polypeptides according to the present invention, which is attached to the support material. Because of the specificity of said polypeptides for their target cells or target molecules or their interaction partners, said polypeptides allow a selective removal of their interaction partner(s) from any kind of sample applied to the support material provided that the conditions for binding are met. The sample may be a biological or medical sample, including but not limited to, fermentation broth, cell debris, cell preparation, tissue preparation, organ preparation, blood, urine, lymph liquid, liquor and the like.

The polypeptides according to the present invention may be attached to the matrix in a covalent or non-covalent manner. Suitable support material is known to the one skilled in the art and can be selected from the group consisting of cellulose, silicon, glass, aluminum, paramagnetic beads, starch and dextrane.

The present invention is further illustrated by the following Figures, Tables, Examples and the Sequence Listing, from which further features, embodiments and advantages may be taken. It is to be understood that the present examples are given by way of illustration only and not by way of limitation of the disclosure.

In connection with the present invention
**Fig. 1** shows the characterization of human sera as sources of pathogen specific antibodies.
**Fig. 2** shows the characterization of the libraries.
**Fig. 3** shows the selection of bacterial cells by MACS using biotinylated human IgAs.
**Fig. 4** shows the PCR analysis to determine the gene distribution of selected antigens in clinical isolates of the respective bacterial pathogen.
**Table 1** shows the summary of all screens performed with genomic *C*. *pneumoniae* AR39 libraries and human serum.
**Table 2** shows the strains used for gene distribution analysis.
**Table 3** shows the summary of the gene distribution analysis for a selected number of antigens in various strains of the respective bacterial species.

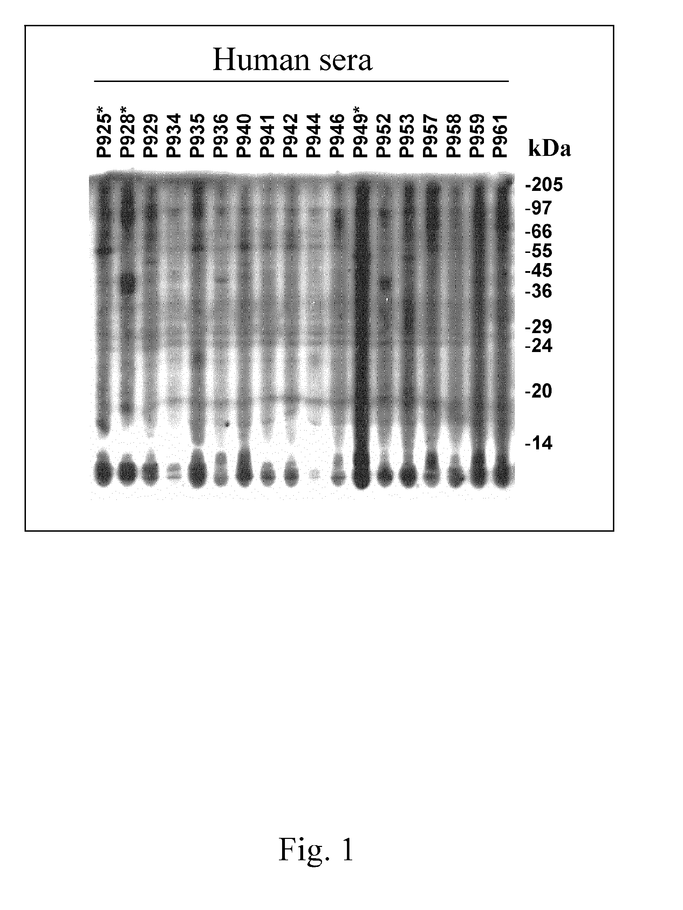

The figures and tables to which it might be referred to in the specification are described in the following in more details. **Figure 1** shows the characterization of human sera for anti-*C. pneumoniae* antibodies as measured by immunoblotting. Sera were preselected for high anti-*C. pneumoniae* IgG antibody levels by IgG-ELISA using *Chlamydia* elementary body (EB) preparations. Proteins of the elementary bodies isolated from *C. pneumoniae* AR39 infected HeLa cells were separated on SDS-PAGE gels and transferred to nitrocellulose membrane. Results of a representative experiment using selected patients' sera at 5,000x dilution are shown. Blots were developed with anti-human IgG secondary antibody reagent. The most reactive samples were selected into screening pools (indicated with *). Mw: molecular weight markers.

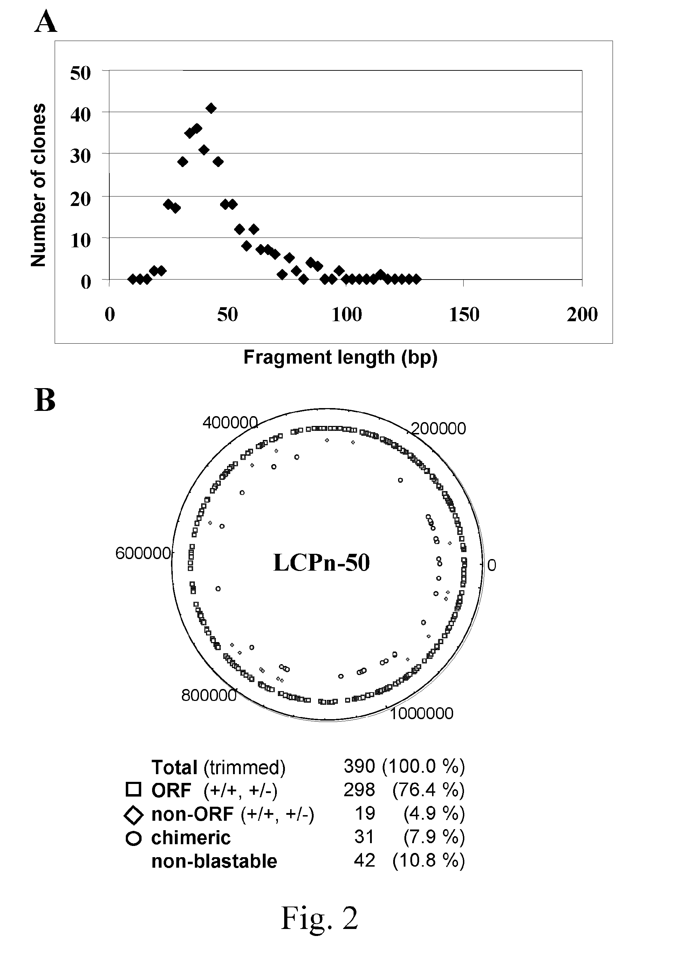

**Figure 2 (A)** shows the fragment size distribution of the *Chlamydia pneumoniae* AR39 large fragment genomic library, LCPn-50. After sequencing 480 randomly selected clones, sequences were trimmed to eliminate vector residues and the numbers of clones with various genomic fragment sizes were plotted. **(B)** shows the graphic illustration of the distribution of the same set of randomly sequenced clones of LCPn-50 over the *C*. *pneumoniae* chromosome (according to the AR39 genome data). Rectangles indicate matching sequences to annotated ORFs and diamonds represent fully matched clones to non-coding chromosomal sequences in +/+ or +/- orientation, respectively. Circles position all clones with chimeric sequences. Numeric distances in base pairs are indicated over the circular genome for orientation. Partitioning of various clone sets within the library is given in numbers and percentage at the bottom of the figure.

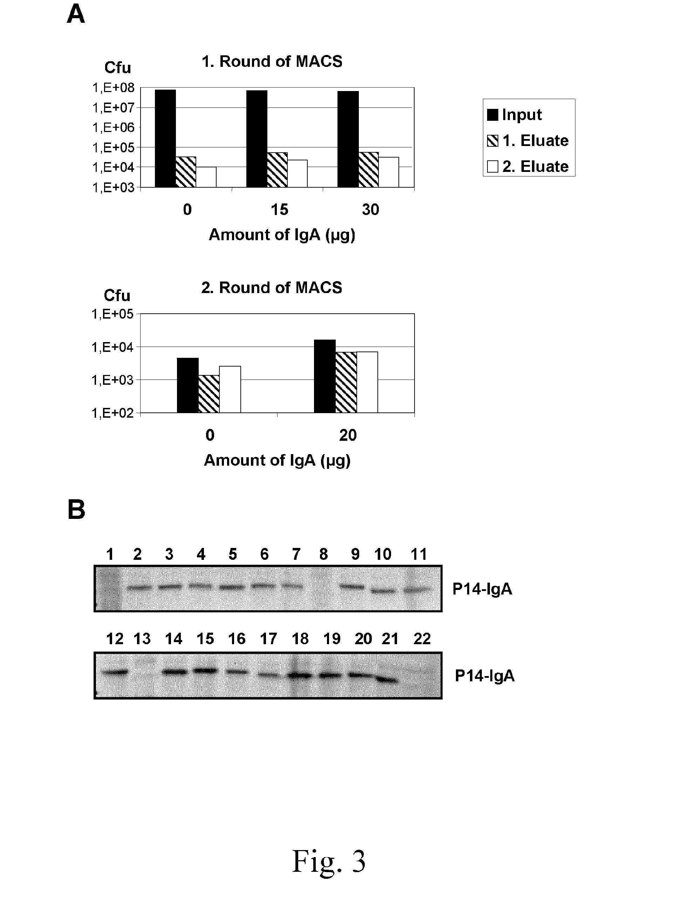

**Figure 3 (A)** shows the MACS selection with human IgAs. The LCPn-50 library in pMAL9.1 was screened with 15 to 30 µg IgA (P14-IgA, purified from human serum) and 10 µg biotinylated anti-human IgA antibodies. As negative control, no serum was added to the library cells for screening. Number of cells selected after the 1^{st} and/or 2^{nd} elution are shown for each of the two selection rounds. **(B)** shows the reactivity of specific clones (1-22) selected by bacterial surface display as analyzed by immunoblot analysis with the human serum IgA pool (P14-IgA) used for selection by MACS at a concentration of 1 µg/ml.

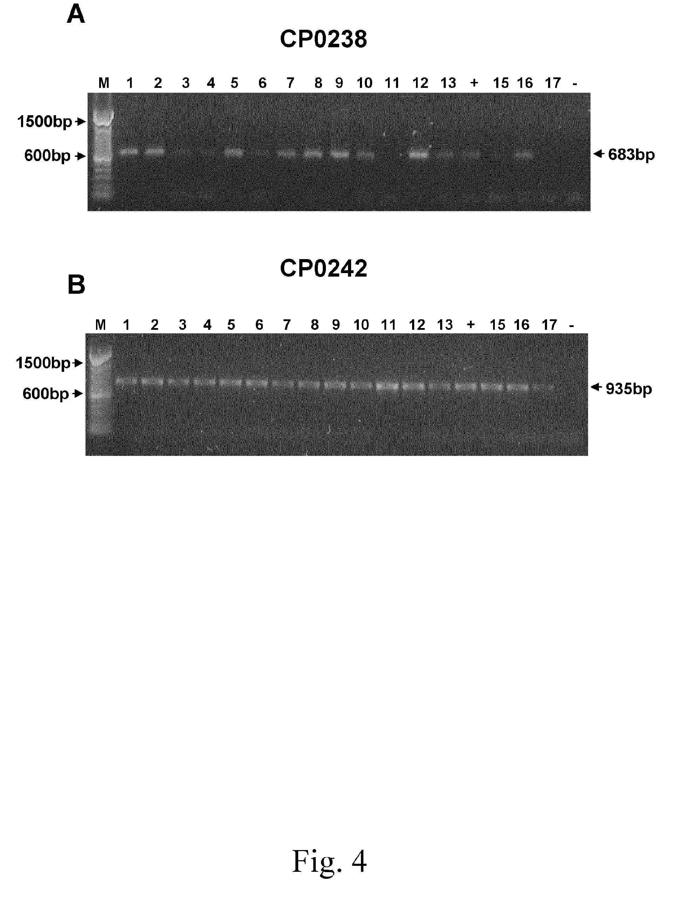

**Figure 4** shows an example for the PCR analysis for the gene distribution analysis of two genes with the respective oligonucleotides and 17 *C*. *pneumoniae* and *C*. *trachomatis* strains. Genomic DNA was used at a dilution of 1:25 to 1:50. Two microliter of each PCR was loaded on a 1% agarose gel. The predicted sizes of the PCR fragments derived from antigens CP0238 and CP0242 from *C*. *pneumoniae* are 683 and 935 bp, respectively. 1-13, 15-17, strains or clinical isolates as shown in Table 2; -, no genomic DNA; +, genomic DNA of *C*. *pneumoniae* strain AR39 (strain 14 in Table 2).

**Table 1: Immunogenic proteins identified by bacterial surface display.**

| | | | | | | | |
|---|---|---|---|---|---|---|---|
| A, 50 bp library (LCPn-50) of *C*. *pneumoniae* AR39 in *lamB* with P14-IgA (number of clones after trimming: 767); B, 300 bp library (LCPn-300) in *fhuA* with P14-IgA (883). The number of selected clones per ORF is listed as well as the immunogenic region delineated by the selected clones. CP0019, annotated reading frame of *C*. *pneumoniae;* ARF0008, predicted novel ORF in alternative reading frame of CP0008; CRF0082, predicted novel ORF on complement reading frame of CP0082. *, prediction of sequences longer than 5 amino acids capable of inducing an antibody response was performed with the program ANTIGENIC (Kolaskar, A.S. and Tongaonkar, P.C., 1990); **, prediction of sequences capable of inducing a class II-restricted T cell response was performed with the program IEDB Analysis resource (Bui, H.H. et al., 2005). The prediction was performed for the twelve MHC II types HLA DRB1*0101, *0301, *0401, *0405, *0701, *0802, *0901, *1101, *1302, *1501, HLADRB4*0101 and HLADRB5*0101. Epitopes with a binding stronger than IC50=5000 nM were selected. | | | | | | | |

| ***Chlamydia pneumoniae* antigenic protein** | **Putative function (by homology)** | **Predicted immunogenic aa*** | **Predicted class II-restricted T cell epitope / regions**** | **No. of selected clones per ORF and screen** | **Location of identified immunogenic region (aa)** | **Seq. ID (DNA)** | **Seq. ID (Protein)** |
|---|---|---|---|---|---|---|---|
| CP0019 | conserved hypothetical protein | 20-28, 32-40, 45-56, 85-96, 102-118, 120-130, 132-154, 158-172, 174-181, 193-202, 208-221, 228-241, 250-259, 274-283, 285-291, 296-309, | 48-59, 267-277, 452-467, 497-512, 659-673 | A:1 | 249-260 | 25 | 123 |
| | | 317-324, 328-339, 348-354, 383-391, 402-412, 418-428, 443-455, 467-482, 484-490, 519-525, 545-557, 568-582, 601-606, 613-638, 646-656, 661-676, 687-694, 706-724, 741-766, 776-782, 789-798, 812-822, 847-869 | | | | | |
| CP0020 | conserved hypothetical protein | 28-37, 44-88, 95-102, 107-115, 142-150, 212-226, 228-233, 235-245, 262-269, 278-311, 319-338, 343-354, 372-405, 409-418, 426-435, 439-445, 447-462, 471-478, 481- | 315-326, 428-442, 741-756, 789-808 | B:9 | 569-636 | 26 | 124 |
| | | 490, 502-514, 522-529, 539-565, 568-589, 595-605, 608-622, 645-651, 656-662, 665-670, 679-685, 696-715, 731-744, 775-781, 789-809 | | | | | |
| CP0052 | penicillin-binding protein, putative | 6-14, 21-44, 52-70, 74-93, 106-132, 136-157, 162-168, 174-184, 186-223, 232-248, 250-262, 264-270, 278-284, 303-324, 342-348, 352-357, 409-415, 423-442, 444-454, 458-478, 480-494, 499-509, 514-521, 529-538, 541-559, 561-575, | 24-38, 108-123, 599-620, 775-788, 939-949 | B:3 | 697-758, 808-875 | 27 | 125 |
| | | 582-605, 611-619, 630-638, 647-665, 672-683, 696-719, 727-739, 748-791, 800-806, 815-822, 848-855, 860-868, 876-884, 891-896, 898-950, 956-962, 971-980, 995-1008, 1036-1043, 1045-1061, 1069-1074 | | | | | |
| CP0057 | aminotransferase, class V | 7-13, 21-27, 33-57, 63-72, 74-80, 82-88, 94-105, 107-119, 121-132, 134-152, 162-199, 201-225, 229-237, 239-246, 252-258, 261-267, 277-298, 300-325, 340-366, 373-383, 391-401 | 17-30, 40-59, 71-83, 93-113, 216-231, 260-277, 371-385, 392-404 | A:3 | 3-23 | 28 | 126 |
| CP0058 | conserved hypothetical protein | 4-17, 22-32, 45-58, 60-70, 72-86, 88-98, 100-108, 112-121, 128-138, 148-162, 167-176, 184-198, 216-231, 241-248 | 3-37, 42-52, 58-67, 72-83, 94-114, 123-137, 142-160, 197-209,218-250 | B:3 | 139-199 | 29 | 127 |
| CP0064 | peptide ABC transporter, ATP-binding protein | 6-23, 32-52, 60-77, 84-90, 92-107, 117-163, 165-239, 249-254, 273-280, 289-308 | 7-22, 24-35, 78-93, 97-110, 124-136, 162-176, 193-209, 213-225, 230-239 | B:2 | 20-89 | 30 | 128 |
| CP0075 | D-alanyl-D-alanine carboxypeptidase | 5-49, 52-59, 65-95, 102-108, 115-123, 126-139, 142-153, 180-186, 193-204, 206-214, 225-247, 270-278, 280-297, 303-319, 321-337, 344-358, 363-371, 378-389, 399-406, 412-419 | 12-27, 125-138, 190-202, 260-277, 394-417 | A:2 | 168-178 | 31 | 129 |
| CP0129 | lipoate-protein ligase-related protein | 4-30, 32-38, 43-50, 55-62, 64-72, 76-83, 97-119, 125-132, 139-156, 158-172, 204-210, 221-227 | 9-21,26-37, 49-63, 84-97, 107-120, 127-137, 142-154, 197-208, 223-235 | B:3 | 112-182, 167-216 | 32 | 130 |
| CP0154 | phenylalanyl-tRNA synthetase beta chain | 4-16, 21-31, 39-57, 61-67, 73-99, 105-111, 119-128, 135-147, 150-159, 163-196, 213-246, 248-255, 257-284, 286-293, 295-317, 333-360, 369-387, 389-413, 415-422, 424-438, 443-454, | 526-543, 609-624, 654-673, 682-699 | A:2 | 518-538 | 33 | 131 |
| | | 461-467, 478-490, 508-520, 527-532, 534-551, 557-564, 566-576, 578-588, 596-684, 687-711, 713-724, 729-741, 749-758, 770-782 | | | | | |
| CP0159 | conserved hypothetical protein | 4-13, 38-59, 80-90, 95-102, 108-123, 130-183, 190-223, 239-249 | 7-25, 45-63, 91-122, 173-183, 216-227 | B:2 | 31-105 | 34 | 132 |
| CP0168 | tRNA pseudouridine synthase A | 4-13, 27-38, 43-48, 53-74, 82-100, 104-110, 112-137, 147-154, 169-179, 184-192, 196-223, 234-249, 252-262 | 1-11, 30-43, 70-86, 142-150, 192-207, 217-229 | B:2 | 119-187 | 35 | 133 |
| CP0186 | secDF protein, putative | 7-36, 45-50, 52-92, 103-125, 129-138, 140-154, 163-176, 178-188, 194-203, 207-222, 230-239, 244-297, 299-314, 324-332, 335-343, 361-368, 370-404, 409-416, 426-443, 451-456, 459-469, 477-493, 496-513, 515-522, 529-535, 540-546, 549-561, 578-586, 588-596, 609-628, 636-645, 659-665, 668-687, | 428-440, 561-572, 859-870, 919-930, 1055-1067 | A:1 | 34-43 | 36 | 134 |
| | | 708-724, 726-739, 761-770, 788-795, 818-825, 842-869, 872-883, 887-897, 905-972, 976-984, 988-998, 1015-1030, 1033-1051, 1076-1082, 1084-1107, 1141-1151, 1167-1180, 1184-1189, 1193-1205, 1221-1228, 1235-1254, 1258-1305, 1307-1314, 1326-1335, 1350-1369, 1371-1395 | | | | | |
| CP0210 | ABC transporter, ATP-binding protein | 4-14, 22-32, 48-61, 63-71, 79-111, 119-145, 147-167, 179-186, 188-201, 217-223, 227-235 | 12-25, 44-60, 72-88, 97-117, 145-158, 178-201, 211-221 | A:50 | 223-241 | 37 | 135 |
| CP0212 | polymorphic membrane protein B/C family | 14-29, 31-38, 50-56, 68-74, 81-89, 108-118, 125-138, 144-151, 167-175, 215-228, 252-262, 272-279, 281-288, 322- | 6-26, 1430-1441, 1648-1666 | A:23, B:4 | 91-170, 1369-1393, 1423-1486 | 38 | 136 |
| | | 344, 358-374, 386-395, 406-418, 426-441, 455-465, 485-490, 513-519, 521-528, 538-543, 548-555, 558-574, 577-583, 585-596, 608-624, 636-643, 665-672, 680-686, 699-707, 715-730, 747-755, 775-786, 812-818, 824-841, 856-868, 892-899, 930-946, 963-972, 979-984, 1008-1015, 1032-1038, 1044-1050, 1052-1060, | | | | | |
| | | 1092-1110, 1143-1148, 1157-1181, 1184-1196, 1215-1222, 1229-1244, 1248-1255, 1267-1272, 1282-1295, 1300-1311, 1323-1329, 1390-1396, 1399-1407, 1423-1431, 1452-1458, 1478-1484, 1488-1501, 1504-1511, 1521-1551, 1573-1599, 1622-1641, 1644-1663, 1669-1675, 1681-1688, 1692-1711 | | | | | |
| CP0213 | polymorphic membrane protein A family | 7-33, 39-47, 53-66, 72-104, 117-126, 132-138, 146-154, 160-167, 182-188, 199-213, 259-270, 283-289, 291-308, 314-326, 328-334, 352-358, 364-370, 372-379, 407-417, | 84-94, 113-125, 141-152, 331-342, 716-728, 743-763 | B:2 | 397-456 | 39 | 137 |
| | | 424-445, 461-475, 477-485, 488-497, 510-516, 539-553, 567-581, 589-595, 668-687, 697-707, 709-731, 733-752, 756-765, 772-780, 797-827, 845-852, 860-876, 884-891, 896-903, 915-922 | | | | | |
| CP0225 | 2-oxo acid dehydrogenase, E2 component, lipoamide | 17-23, 26-34, 36-42, 45-51, 53-63, 69-79, 89-97, 106-119, 146-161, 175-184, 186-201, 217-244, 252-265, 268-278, 280-294, 304-315, 324-340, 343-351, 353-374 | 17-27, 110-120, 166-179, 220-234, 251-262, 282-294, 322-339, 354-366, 379-387 | A:2 | 32-48 | 40 | 138 |
| CP0238 | ubiquinone/ menaquinone biosynthesis methyltransferase | 17-27, 34-40, 47-65, 68-112, 114-126, 133-148, 150-157, 163-187, 190-204, 207-234 | 5-25, 34-51, 115-125, 140-152, 166-181, 191-201,215-233 | A:2, B:2 | 5-66 | 41 | 139 |
| CP0242 | conserved hypothetical protein | 4-16, 20-42, 60-66, 75-108, 112-117, 131-138, 148-153, 157-174, 201-206, 213-228, 235-243, 246-259, 266-271, 295-301, | 20-31, 159-177, 235-244, 362-371, 444-454 | B:5 | 271-367 | 42 | 140 |
| | | 311-316, 331-337, 344-353, 364-377, 383-389, 405-412, 452-458, 470-487, 491-505, 508-517, 540-555, 557-565, 576-587, 593-603, 612-617 | | | | | |
| CP0251 | dnaK protein | 7-14, 18-25, 30-35, 43-48, 82-98, 102-111, 139-149, 165-185, 189-197, 203-219, 231-236, 268-277, 291-301, 303-311, | 8-23, 122-130, 289-300, 289-300, 336-345, 388-398, 590-614 | A:9 | 270-279, 621-632 | 43 | 141 |
| | | 315-330, 332-340, 344-354, 365-374, 381-400, 404-409, 419-426, 431-441, 457-467, 469-475, 481-490, 556-562, 605-613, 649-655 | | | | | |
| CP0285 | polymorphic membrane protein E/F family | 4-15, 23-32, 48-67, 76-83, 86-94, 97-105, 107-120, 127-136, 180-185, 206-212, 251-257, 313-322, 325-331, 340-353, 374-381, 397-403, 407-413, 420-432, 450-457, 471-483, 492-503, 505-515, 519-530, 539-548, 558-579, 602-611, | 84-97, 141-152, 181-189, 318-328, 439-447, 498-512, 656-668, 819-828 | B:15 | 146-221, 782-847 | 44 | 142 |
| | | 625-633, 636-643, 650-656, 658-666, 677-683, 686-694, 707-714, 716-730, 745-763, 765-786, 791-830, 841-857, 864-870, 873-885, 887-895, 912-919, 921-938, 940-949 | | | | | |
| CP0294 | hypothetical protein | 4-32, 34-61, 65-75, 81-91, 99-106, 121-132, 150-156, 162-170, 174-182, 189-196, 201-214, 230-236, 239-246, | 3-17, 53-72, 187-199,452-465, 468-483, 592-601 | B:2 | 216-283 | 45 | 143 |
| | | 276-285, 287-295, 301-310, 335-354, 366-383, 390-398, 408-433, 439-445, 466-478, 499-519, 525-544, 550-555, 572-580, 589-603, 624-633, 648-655, 659-671, 677-682 | | | | | |
| CP0301 | polymorphic membrane protein G family | 5-29, 42-60, 72-77, 114-143, 151-157, 162-174, 177-182, 185-193, 233-251, 266-274, 279-298, 301-308, 321-335, 338-365, 378-386, 388-396, 401-407, 412-434, 439-459, 475-480, 499-505 | 7-21, 74-84, 91-101, 119-130, 363-372, 387-396 | A:3 | 245-264, 367-374 | 46 | 144 |
| CP0307 | polymorphic membrane protein G family | 4-26, 28-34, 57-64, 75-81, 95-100, 126-141, 144-150, 158-163, 200-210, 216-221, 243-249, 291-309, 326-339, 363-369, 380-389, 396-403, 410-422, 436-455, 485-498, 516-525, 541-576, | 2-13, 542-552, 627-637, 652-660, 798-807 | B:7 | 716-781 | 47 | 145 |
| | | 608-619, 635-641, 664-674, 683-692, 697-703, 707-723, 737-749, 760-765, 775-808, 827-839, 851-859, 869-875, 877-884, 886-906, 910-927 | | | | | |
| CP0308 | polymorphic membrane protein G family | 4-29, 43-48, 56-66, 70-82, 93-102, 107-113, 127-152, 159-164, 179-184, 201-207, 216-224, 231-239, 244-250, 262-269, 291-305, 307-313, 333-340, 368-374, 379-387, 414-422, 432-438, | 7-23, 173-185, 530-540, 726-734 | B:1 | 692-760 | 48 | 146 |
| | | 440-461, 487-498, 518-524, 539-548, 557-567, 610-615, 644-651, 667-675, 681-693, 709-718, 721-726, 734-758, 780-817, 833-843, 856-865, 872-879, 893-900, 902-908, 913-921 | | | | | |
| CP0309 | polymorphic membrane protein G family | 4-22, 58-73, 85-106, 115-129, 139-145, 170-181, 185-191, 199-204, 242-249, 270-276, 293-303, 332-339, 353-359, 387-392, 424-439, 459-472, 532-538, 555-563, 593-604, | 10-24, 1065-1079, 1101-1110, 1141-1156 | B:2 | 1189-1244 | 49 | 147 |
| | | 631-636, 654-659, 679-686, 689-705, 718-733, 740-751, 756-771, 789-796, 798-805, 834-842, 851-856, 885-894, 922-930, 934-947, 953-960, 963-975, 983-988, 1008-1019, 1026-1038, 1041-1083, 1086-1098, 1121-1157, 1159-1166, 1169-1182, 1192-1207, 1209-1216, 1232-1260 | | | | | |
| CP0378 | 2-oxoglutarate dehydrogenase, E1 component | 5-12, 38-50, 70-91, 109-157, 169-182, 185-191, 205-229, 234-251, 262-279, 287-314, 321-331, 335-351, 356-364, 366-373, 377-383, | 80-90, 212-221, 233-243, 286-296, 356-367, 531-541, 859-869 | A:5 | 446-462 | 50 | 148 |
| | | 386-394, 396-415, 417-429, 437-448, 456-467, 490-496, 504-512, 518-530, 533-543, 545-555, 577-586, 603-610, 617-628, 635-662, 670-682, 689-699, 713-719, 724-741, 745-769, 779-784, 790-808, 816-850, 866-879, 894-905 | | | | | |
| CP0392 | hypothetical protein | 6-23, 26-31, 35-99, 108-120, 134-146, 154-167, 171-178, 183-189, 194-203, 206-212, 241-250, 252-270, 278-298, 302-338 | 3-14, 31-45, 51-69, 70-91, 152-165, 257-268, 299-311 | A:2 | 79-94, 136-145 | 51 | 149 |
| CP0405 | hypothetical protein | 21-26, 31-37, 42-92, 100-127, 131-149, 182-188, 190-214, 216-231, 249-257, 264-289, 305-326, 332-342, 348-357, 360-368, 379-385, 387-393, 396-410, 421-427, 435-452 | 42-51,62-75, 79-88, 264-276, 336-353, 398-406 | B:2 | 97-165 | 52 | 150 |
| CP0409 | conserved hypothetical protein | 4-9, 16-21, 23-29, 34-41, 46-51, 58-73, 104-113, 115-122, 125-134, 141-168 | 17-32, 44-70, 89-103, 122-133, 150-165 | A:1 | 106-123 | 53 | 151 |
| CP0444 | thioredoxin reductase | 4-11, 16-34, 82-98, 103-114, 132-147, 151-158, 164-182, 215-221, 236-244, 253-259, 261-267, 272-283, 285-307 | 4-13,23-40,61-74, 169-191, 203-212, 289-297 | B:16 | 16-80 | 54 | 152 |
| CP0457 | cationic outer membrane protein OmpH, putative | 4-18, 20-34, 50-56, 64-69, 100-109, 114-131, 142-150, 159-165 | 3-20, 48-59, 75-83, 104-122 | B:1 | 36-113 | 55 | 153 |
| CP0484 | DNA gyrase, subunit B | 10-23, 36-48, 55-66, 68-75, 95-109, 116-151, 156-169, 173-192, 199-205, 208-213, 219-226, 232-238, 246-256, 276-284, 295-314, | 191-200, 276-285, 307-321, 523-535, 568-577, 721-729, 772-780 | A:1 | 303-330 | 56 | 154 |
| | | 332-343, 358-373, 381-393, 406-426, 431-438, 454-469, 481-492, 519-533, 536-551, 556-561, 567-572, 594-608, 612-619, 629-638, 665-681, 685-697, 710-727, 759-777, 792-798 | | | | | |
| CP0504 | conserved hypothetical protein | 7-13, 29-39, 54-60, 62-68, 99-104, 126-138, 140-153, 159-190, 204-210, 217-223, 234-240, 243-262, 270-278 | 6-17,79-88, 98-107, 125-137, 159-167, 173-191, 267-276 | A:4 | 154-168 | 57 | 155 |
| CP0529 | conserved hypothetical protein | 4-22, 50-58, 71-77, 83-93, 108-127, 143-153, 158-191, 205-213, 221-228 | 6-27, 36-45, 147-157, 172-197 | A:6 | 28-49 | 58 | 156 |
| CP0559 | pyrophosphate--fructose 6-phosphate 1-phosphotran sferase | 4-22, 26-35, 38-61, 63-76, 85-99, 101-109, 145-151, 153-160, 162-172, | 175-187, 242-254, 377-389, 450-467, 493-503, 528-538 | B:3 | 406-485 | 59 | 157 |
| | | 180-187, 194-202, 208-215, 223-231, 235-247, 253-273, 278-295, 302-323, 337-358, 366-404, 420-434, 436-450, 454-468, 474-485, 491-514, 524-533, 539-545 | | | | | |
| CP0569 | peptide ABC transporter, periplasmic peptide-binding protein | 7-30, 35-45, 53-70, 78-105, 120-127, 129-143, 151-158, 162-193, 208-216, 219-249, 252-258, 277-308, 311-321, 323-347, 359-364, 375-385, 389-395, 401-409, 414-420, 436-449, 467-474, 491-507, 513-519 | 6-27, 94-106, 135-145,255-265, 289-311 | B:2 | 421-488 | 60 | 158 |
| CP0625 | conserved hypothetical protein (S/T proteinkinase) | 12-22, 27-35, 38-53, 60-81, 87-94, 96-113, 115-136, 144-172, 189-196, 208-237, 239-251, 258-265, 272-292, 296-305, 319-331, 351-371, 378-389, 404-425, 428-436, 443-448, 458-477, 501-513, 535-541, 547-554, 557-565, 574-584, 590-598 | 36-49, 59-70, 100-113, 121-130, 147-155 351-' 360 | B:2 | 442-492, 526-610 | 61 | 159 |
| CP0664 | signal peptidase I, putative | 10-30, 36-46, 56-67, 78-105, 116-124, 126-142, 146-160, 166-172, 186-198, | 17-28, 291-300, 340-352, 362-371, 387-396 | A:2 | 194-207 | 62 | 160 |
| | | 207-224, 249-256, 278-289, 297-304, 307-315, 318-350, 352-367, 373-388, 395-413, 427-438, 444-455, 457-472, 482-490, 495-501, 512-522, 529-535, 537-552, 572-580, 589-620 | | | | | |
| CP0678 | excinuclease ABC, subunit A | 4-14, 16-22, 26-34, 40-50, 56-65, 75-86, 88-101, 105-117, 126-132, 134-142, 145-170, 172-191, 193-205, 214-223, | 425-443 | B:2 | 1058-1076, 1643-1700 | 63 | 161 |
| | | 227-235, 247-290, 294-319, 332-339, 345-355, 363-369, 394-403, 407-412, 429-466, 468-475, 494-510, 515-522, 528-534, 539-545, 556-562, 574-589, | | | | | |
| | | 596-604, 609-627, 637-647, 663-671, 679-689, 694-702, 708-715, 718-727, 738-744, 750-758, 774-808, 815-836, 840-883, 890-906, 910-929, 933-943, 951-967, 972-986, 988-1027, 1032-1041, 1045-1058, 1069-1079, | | | | | |
| | | 1081-1102, 1117-1123, 1125-1143, 1145-1168, 1173-1178, 1186-1199, 1202-1209, 1214-1220, 1225-1242, 1244-1251, 1257-1266, 1269-1275, 1295-1313, 1339-1345, 1364-1377, 1390-1410, 1412-1431, 1438-1459, 1472-1499, 1511-1537, 1543-1549, 1553-1560, 1566-1579, 1585-1609, 1621-1632, 1636-1665, 1669-1718, 1721-1730, 1734-1761, 1763-1787, 1796-1802, 1805-1823 | | | | | |
| CP0694 | DNA-directed RNA polymerase, beta subunit | 11-21, 27-48, 58-69, 73-89, 100-110, 142-152, 165-178, 187-196, 203-216, 238-246, 254-262, 278-288, 294-299, 308-315, | 685-693, 699-709, 1065-1075, 1147-1158 | A:2 | 1068-1077 | 64 | 162 |
| | | 334-340, 366-374, 376-386, 392-401, 406-412, 444-459, 463-469, 474-479, 484-490, 500-517, 524-534, 542-552, 566-578, 586-594, 603-628, 643-664, 668-692, 699-706, 714-726, 729-735, 747-755, 765-771, 778-785, 804-814, 822-837, 848- | | | | | |
| | | 855, 860-886, 898-909, 912-919, 925-933, 936-942, 945-957, 966-975, 978-997, 1015-1035, 1045-1055, 1066-1076, 1082-1107, 1141-1179, 1193-1214, 1240-1246, 1252-1259 | | | | | |
| CP0719 | phosphoglu comutase/phosphoman nomutase, putative | 7-15, 17-28, 34-39, 83-96, 100-107, 119-149, 152-158, 168-174, 176-213, 220-231, | 66-80, 169-178, 262-271, 333-348, 360-370, 554-564 | B:2 | 228-287 | 65 | 163 |
| | | 237-262, 264-273, 280-286, 318-327, 338-348, 358-369, 371-386, 405-410, 413-421, 427-443, 445-462, 467-473, 483-490, 498-503, 513-527, 529-537, 540-546, 581-590 | | | | | |
| CP0761 | polymorphic membrane protein G family | 4-16, 18-24, 57-69, 121-140, 151-157, 159-166, 175-183, 206-212, 261-272, 276-283, 285-305, 321-327, | 5-17, 131-141, 183-198, 376-385 | B:9 | 786-836 | 66 | 164 |
| | | 343-350, 352-367, 383-389, 410-416, 424-435, 448-455, 457-464, 469-478, 493-499, 509-528, 543-555, 564-575, 584-594, 608-631, 635-658, 667-676, 683-689, 692-698, 708-731, 737-750, 757-774, 778-784, 798-805, 809-815 | | | | | |
| CP0790 | 1-deoxyxylulose-5-phosphate synthase | 4-30, 35-47, 49-67, 73-86, 114-120, 123-132, 140-160, 168-176, 195-201, 211-218, 220-256, 261-285, 313-328, 330-349, 367-373, 375-385, 388-422, 449-488, 509-547, 553-569, 582-597, 599-606 | 33-43, 187-200, 223-232, 259-272, 306-316, 443-452, 461-472 | B:5 | 306-370 | 67 | 165 |
| CP0815 | chorismate synthase | 6-11, 19-44, 62-72, 78-87, 101-108, 128-142, 149-185, 191-198, 204-222, 224-241, 284-294, 296-301, 304-311, 327-356 | 62-70, 141-161, 224-238, 297-305, 339-354 | A:3 | 259-277, 298-320 | 68 | 166 |
| CP0837 | conserved hypothetical protein | 6-23, 34-59, 66-75, 79-96, 106-117, 119-130, 133-143, 164-172, 179-193, | 12-22, 82-94, 186-194, 371-382, 434-447, 580-589 | A:3 | 498-506 | 69 | 167 |
| | | 195-207, 215-241, 247-261, 285-293, 300-307, 317-329, 346-352, 357-362, 368-384, 397-408, 416-424, 432-453, 461-485, 493-501, 518-527, 533-544, 552-562, 574-588, 604-613 | | | | | |
| CP0879 | hypothetical protein | 4-13, 15-21, 32-41, 43-54, 56-84, 86-93, 95-113, 120-126, 130-165, 175-189, 195-202, 210-222, 244-250, 253-270, 273-280, 283-291, 301-313, 315-339 | 27-38, 56-86-93, 38 77-102 174-185, 259-270, 298-309, 319-331 | B:2 | 138-205 | 70 | 168 |
| CP0938 | conserved hypothetical protein | 6-25, 28-87, 93-100, 105-112, 118-133, 138-145, 157-165, 167-184, 198-209, 220-237, 245-252, 254-262, 265-277, 288-299, 310-326, 335-362 | 10-18,28-51, 59-68, 127-138, 167-177, 338-347 | B:3 | 247-293 | 71 | 169 |
| CP0946 | 3(2),5-bisphosphate nucleotidase | 8-19, 21-27, 29-36, 49-67, 84-101, 103-122, 128-157, 159-166, 168-177, 181-187, 192-205, 207-217, 222-251, 255-266, 272-280, 284-297, 306-322 | 52-67, 86-98, 127-138, 144-167,227-237, 282-296, 307-319 | A:2 | 61-74 | 72 | 170 |
| CP0977 | oxygen-independent coproporph | 9-25, 33-41, 48-73, 78-86, 89-97, 100-106, 117-135, 141-147, 164-181, 189- | 1-18, 124-137, 244-258, 298- | B:4 | 201-262 | 73 | 171 |
| | yrinogen III oxidase | 195, 200-215, 226-251, 267-283, 289-300, 318-324, 332-340, 355-362, 374-382, 389-394, 397-402, 422-448, 450-455 | 310, 324-343, 368-384,431-450 | | | | |
| CP1002 | cell shape-determining protein MrdB | 4-31, 36-45, 52-76, 78-118, 120-135, 145-164, 167-228, 241-250, 269-279, 283-312, 315-333, 337-362, 364-376 | 9-25, 75-88, 104-116, 121-133, 180-197,215-227, 324-338 | B:13 | 208-288 | 74 | 172 |
| CP1020 | helicase, Snf2 family | 24-34, 39-50, 52-60, 70-93, 96-105, 124-130, 146-168, 173-183, 195-213, 217-231, 248-259, 267-285, 304-312, 314-322, 334-350, 365-388, 391-403, 408-416, 418-443, 450-477, 496-503, | 234-244, 336-346, 457-468, 1002-1012 | A:12, B:4 | 938-1000 | 75 | 173 |
| | | 516-522, 544-550, 565-592, 594-600, 614-635, 639-670, 691-705, 707-714, 733-744, 751-764, 777-787, 797-829, 848-856, 864-887, 901-913, 919-938, 941-950, 955-961, 966-990, 1004-1023, 1027-1033, 1040-1047, 1053-1058, 1063-1073, 1079-1090, 1104-1109, 1112-1124, 1128-1134, 1138-1145, 1149-1155 | | | | | |
| CP1056 | general secretion pathway protein D | 4-28, 59-66, 93-98, 128-134, 140-164, 166-186, 198-208, 211-227, 233-240, 246-258, 264-277, 282-291, 297-302, 318-349, | 3-18, 332-340, 445-454, 580-591 | B:2 | 309-374 | 76 | 174 |
| | | 362-370, 372-386, 393-417, 420-427, 437-462, 464-473, 481-503, 508-514, 519-533, 543-549, 554-568, 584-592, 636-647, 655-662, 680-688, 702-707, 716-723, 726-731, 738-746 | | | | | |
| CP1062 | conserved hypothetical protein | 16-31, 78-84, 131-140, 145-155, 168-174, 176-193, 219-225, 253-292, 303-368, 376-406, 420-436, 444-453, 468-485 | 251-270, 342-352, 380-405, 407-417, 443-451 | A:1, B:2 | 36-109, 375-388 | 77 | 175 |
| | | | | | | | |
| ARF0008 | hypothetical protein (27 aa) | 4-14 | 1-27 | A:5 | 10-21 | 78 | 176 |
| ARF0009 | hypothetical protein (143 aa) | 18-35, 47-55, 65-82, 106-112, 117-140 | 22-46, 56-70, 79-96, 98-107, 119-135 | B:2 | 71-134 | 79 | 177 |
| ARF0014 | hypothetical protein (41 aa) | 15-38 | 10-38 | A:2 | 5-26 | 80 | 178 |
| ARF0044 | hypothetical protein (58 aa) | 31-48, 50-55 | 10-20, 29-43 | A:2 | 21-36 | 81 | 179 |
| ARF0138 | hypothetical protein (20 aa) | none | 3-18 | A:2 | 1-10 | 82 | 180 |
| ARF0195 | hypothetical protein (23 aa) | 6-14 | 8-19 | A:2 | 13-23 | 83 | 181 |
| ARF0199 | hypothetical protein (39 aa) | 4-10 | 5-22 | A:20 | 5-11 | 84 | 182 |
| ARF0297 | hypothetical protein (34 aa) | 9-18,21-30 | 7-34 | A:1 | 22-32 | 85 | 183 |
| ARF0533 | hypothetical protein (94) | 4-13, 31-39, 56-74, 76-83, 85-91 | 21-34, 61-72, 74-92 | B:1 | 11-72 | 86 | 184 |
| ARF0965 | hypothetical protein (69 aa) | 8-32, 38-49, 57-66 | 5-14, 21-34, 42-54 | A:2 | 4-18 | 87 | 185 |
| ARF1099 | hypothetical protein (34 aa) | 4-19, 25-31 | 4-32 | A:2 | 13-26 | 88 | 186 |
| CRF0082 | hypothetical protein (101 aa) | 1-9, 17-25, 38-49, 52-58 | 1-9, 38-55 | B:9 | 8-77 | 89 | 187 |
| CRF0151 | hypothetical protein (36 aa) | 4-16,20-27 | 4-12,20-30 | A:50 | 5-27 | 90 | 188 |
| CRF0265 | hypothetical protein (47 aa) | 20-26, 32-41 | 20-42 | A:8 | 4-18 | 91 | 189 |
| CRF0302 | hypothetical protein (133 aa) | 4-15, 21-41, 45-55, 63-92, 105-123 | 41-52, 66-81, 104-121 | A:3 | 30-45 | 92 | 190 |
| CRF0306 | hypothetical protein (18 aa) | none | 1-17 | A:4 | 4-16 | 93 | 191 |
| CRF0484 | hypothetical protein (32 aa) | 4-17, 19-25 | 4-32 | A:9 | 12-25 | 94 | 192 |
| CRF0796 | hypothetical protein (53 aa) | 5-31, 33-50 | 10-45 | A:69 | 37-48 | 95 | 193 |
| CRF0819 | hypothetical protein (23 aa) | 10-17 | 2-23 | A:18 | 4-21 | 96 | 194 |
| CRF0823 | hypothetical protein (72 aa) | 4-21, 52-69 | 8-29, 41-50, 52-72 | A:7 | 18-44 | 97 | 195 |
| CRF1103 | hypothetical protein (32 aa) | 11-29 | 3-26 | A:5 | 11-22 | 98 | 196 |

**Table 2. List of strains used for gene distribution analysis.**

| Table 2 shows strains of *C*. *pneumoniae* and *C*. *trachomatis* analyzed for the gene distribution study. The species and source of the strains are given. *C*. *pneumoniae* AR39 was used for generating genomic libraries. | | | |
|---|---|---|---|
| No | Strain ID | Species | Description |
| 1 | MUL-1 | *C. pneumoniae* | Clinical isolate |
| 2 | MUL-250 | *C*. *pneumoniae* | Clinical isolate |
| 3 | PB-2 | *C*. *pneumoniae* | Clinical isolate |
| 4 | PB-4 | *C*. *pneumoniae* | Clinical isolate |
| 5 | CV-2 | *C*. *pneumoniae* | Clinical isolate |
| 6 | CV-3 | *C*. *pneumoniae* | Clinical isolate |
| 7 | CV-4 | *C*. *pneumoniae* | Clinical isolate |
| 8 | CV-5 | *C*. *pneumoniae* | Clinical isolate |
| 9 | CV-6 | *C*. *pneumoniae* | Clinical isolate |
| 10 | CV-14 | *C*. *pneumoniae* | Clinical isolate |
| 11 | L2 | *C*. *trachomatis* | Reference strain |
| 12 | C/TW | *C*. *trachomatis* | Reference strain |
| 13 | CM-1 | *C*. *pneumoniae* | Reference strain |
| 14 | AR 39 | *C. pneumoniae* | Reference strain |
| 15 | TWAR | *C*. *pneumoniae* | Reference strain |
| 16 | CWL-029 | *C*. *pneumoniae* | Reference strain |
| 17 | 2043 | *C*. *pneumoniae* | Reference strain |

**Table 3. Gene distribution analysis for a selected number of antigens in various strains of C. pneumoniae and C. trachomatis.**

| | | |
|---|---|---|
| *17 Chlamydia* strains including the AR39 strain as a positive PCR control as shown in Table 2 were tested by PCR with oligonucleotides specific for the genes encoding relevant antigens. The gene distribution table lists the number of positive PCR results from 15 *C*. *pneumoniae* and 2 *C*. *trachomatis* strains for each gene and is an indication of the presence and conservation of the gene in diverse isolates of *C*. *pneumoniae* and *C*. *trachomatis* (e.g.: 14/1; 14 *C*. *pneumoniae* and 1 *C*. *trachomatis* strains positive). | | |

| **ORF** | **SEQ ID** NO (DNA) | Gene distribution |
|---|---|---|
| CP0019 | 25 | 15/1 |
| CP0020 | 26 | 15/1 |
| CP0052 | 27 | 15/1 |
| CP0057 | 28 | 15/1 |
| CP0058 | 29 | 15/1 |
| CP0064 | 30 | 15/2 |
| CP0075 | 31 | 15/2 |
| CP0129 | 32 | 15/1 |
| CP0154 | 33 | 15/1 |
| CP0159 | 34 | 14/1 |
| CP0186 | 36 | 14/2 |
| CP0210 | 37 | 6/1 |
| CP0225 | 40 | 15/2 |
| CP0238 | 41 | 13/1 |
| CP0242 | 42 | 15/2 |
| CP0409 | 53 | 15/2 |
| CP0504 | 57 | 15/1 |
| CP0529 | 58 | 12/1 |
| CP0559 | 59 | 15/1 |
| CP0664 | 62 | 14/2 |
| CP0790 | 67 | 15/1 |
| CP0815 | 68 | 15/2 |
| CP0879 | 70 | 15/1 |
| CP0938 | 71 | 15/1 |
| CP0946 | 72 | 13/1 |
| CP0977 | 73 | 15/2 |
| CP1002 | 74 | 15/2 |

### EXAMPLES

### Example 1: General screening procedure for the identification of the peptides according to the present invention

The approach, which has been employed for the present invention, is based on the interaction of proteins or peptides encoded by *C. pneumoniae* with the antibodies present in human sera. The antibodies produced against *C. pneumoniae* by the human immune system and present in human sera are indicative of the *in vivo* expression of the antigenic proteins and their immunogenicity. In addition, the antigenic proteins, as identified by the bacterial surface display expression libraries using pools of pre-selected sera, are processed in a second and third round of screening by individual selected or generated sera. Thus the present invention supplies an efficient, relevant, comprehensive set of antigens as a pharmaceutical composition, especially a vaccine preventing infections caused by *C. pneumoniae.*

In the antigen identification program for identifying a comprehensive set of antigens according to the present invention, at least two different bacterial surface expression libraries from *C*. *pneumoniae* are screened with several serum pools or plasma fractions (antibody pools). The antibody pools are derived from a serum collection, which has been tested against antigenic compounds of *C*. *pneumoniae,* such as whole cell, total extracts. Sera determined to have high ELISA titre have to react with multiple proteins in immunoblotting in order to be considered relevant in the screening method applied for the present invention.

The expression libraries as used in the present invention should allow expression of all potential antigens, e.g. derived from all secreted and surface proteins of *C*. *pneumoniae.* Bacterial surface display libraries will be represented by a recombinant library of a bacterial host displaying a (total) set of expressed peptide sequences of *C*. *pneumoniae* on two selected outer membrane proteins (LamB and FhuA) at the bacterial host membrane (Georgiou, G., 1997; Etz, H. et al., 2001). One of the advantages of using recombinant expression libraries is that the identified serum-reactive antigens may be instantly produced by expression of the coding sequences of the screened and selected clones expressing the antigens without further recombinant DNA technology or cloning steps necessary.

The comprehensive set of antigens identified by the described program according to the present invention may be analyzed further by one or more additional rounds of screening. Therefore individual antibody preparations or antibodies generated against selected peptides, which were identified as immunogenic can be used. According to a preferred embodiment the individual antibody preparations for the second round of screening are derived from healthy adults and/or challenged adults who show an antibody titre above a certain minimum level, for example an antibody titre being higher than 80 percentile, preferably higher than 90 percentile, especially higher than 95 percentile of the human (patient or healthy individual) sera tested. Using such high titre individual antibody preparations in the second screening round allows a very selective identification of the antigens and fragments thereof from *C. pneumoniae.*

Following the comprehensive screening procedure, the selected antigenic proteins, expressed as recombinant proteins, or -in case they can not be expressed in prokaryotic expression systems-*in vitro* translated products, or synthetically produced antigenic peptides can be tested in a second screening by a series of ELISA and Western blotting assays for the assessment of their immunogenicity with a large human serum collection (minimum -20 healthy and patients sera).

It is important that the individual antibody preparations (which may also be the selected serum) allow a selective identification of the most promising candidates of all the serum-reactive antigens from all the promising candidates from the first round. Therefore, preferably at least 10 individual antibody preparations (i.e. antibody preparations (e.g. sera) from at least 10 different individuals exposed to the chosen pathogen) should be used in identifying these antigens in the second screening round. Of course, it is possible to use also less than 10 individual preparations, however, selectivity of the step may not be optimal with a low number of individual antibody preparations. On the other hand, if a given antigen (or an antigenic fragment thereof) is recognized by at least 10 individual antibody preparations, preferably at least 30, especially at least 50 individual antibody preparations, identification of the antigen is also selective enough for a proper identification. Serum-reactivity may of course be tested with as many individual preparations as possible (e.g. with more than 100 or even with more than 1,000).

Therefore, the relevant portion of the antibody preparations according to the method of the present invention should preferably be at least 10, more preferably at least 30, especially at least 50 individual antibody preparations. Alternatively (or in combination) antigens may preferably be also identified with at least 20%, preferably at least 30%, especially at least 40% of all individual antibody preparations used in the second screening round.

According to a preferred embodiment of the present invention, the sera from which the individual antibody preparations for the second round of screening are prepared (or which are used as antibody preparations), are selected by their titre against *C. pneumoniae* (e.g. against a preparation of this pathogen, such as a lysate, cell wall components and recombinant proteins). Preferably, some are selected with an IgG titre above 1,000 U, especially above 5,000 U (U = units, calculated from the OD₄₀₅ₙₘ reading at a given dilution) when the whole organism (total lysate or whole cells) is used as antigen in the ELISA.

The recognition of linear epitopes recognized by serum antibodies can be based on sequences as short as 4-5 amino acids. Of course it does not necessarily mean that these short peptides are capable of inducing the given antibody *in vivo.* For that reason the defined epitopes, polypeptides and proteins are further to be tested in animals (mainly in mice) for their capacity to induce antibodies against the selected proteins *in vivo.*

The preferred antigens for extracellular bacteria are located on the cell surface or secreted, and are therefore accessible extracellularly. Antigens from bacteria with an intracellular stage in host cells may also be derived from intracellular locations, but need to be presented on the host cell as antigens. Antibodies against cell wall proteins are expected to serve multiple purposes: to inhibit adhesion, to interfere with nutrient acquisition, to inhibit immune evasion and to promote phagocytosis (Hornef, M. et al., 2002). Antibodies against secreted proteins are beneficial in neutralisation of their function as toxin or virulence component. It is also known that bacteria communicate with each other through secreted proteins. Neutralizing antibodies against these proteins will interrupt growth-promoting cross-talk between or within infection causing pathogen species. Bioinformatic analyses (signal sequences, cell wall localisation signals, and transmembrane domains) proved to be very useful in assessing cell surface localisation or secretion. The experimental approach includes the isolation of antibodies with the corresponding epitopes and proteins from human serum, and the generation of immune sera in mice against (poly) peptides selected by the bacterial surface display screens. These sera are then used in a third round of screening as reagents in at least one of the following assays: cell surface staining of *C*. *pneumoniae* grown under different conditions (FACS or microscopy), determination of neutralizing capacity (toxin, adherence), and promotion of opsonisation and phagocytosis *(in vitro* phagocytosis assay).

For that purpose, bacterial *E. coli* clones are directly injected into mice and immune sera are taken and tested in the relevant *in vitro* assay. Alternatively, specific antibodies may be purified from human or mouse sera using peptides or proteins as substrate.

According to the antigen identification method used herein, the present invention can surprisingly provide a set of comprehensive novel nucleic acids and novel proteins, antigens and fragments thereof of *C*. *pneumoniae,* among other things, as described herein. The nucleotide sequences according to the present invention encoding such antigens have preferably a nucleotide sequence which is individually set forth in Seq ID Nos 1 to 98, whereby the corresponding encoded amino acid sequences have an amino acid sequence as set forth in Seq ID Nos 99 to 196.

All linear fragments of a particular antigen may be identified by analysing the entire sequence of the protein antigen by a set of peptides overlapping by 1 amino acid with a length of at least 10 amino acids. Subsequently, non-linear epitopes can be identified by analysis of the antigen with sera using the expressed full-length protein or domain polypeptides thereof. Assuming that a distinct domain of a protein is sufficient to form the 3D structure independent from the native protein, the analysis of the respective recombinant or synthetically produced domain polypeptide with serum would allow the identification of conformational epitopes within the individual domains of multi-domain proteins. For those antigens where a domain possesses linear as well as conformational epitopes, competition experiments with peptides corresponding to the linear epitopes may be used to confirm the presence of conformational epitopes.

### Example 2: Characterization and selection of human serum samples based on anti-Chlamydia antibodies and preparation of antibody screening reagents

### Experimental procedures

*Enzyme-linked immunosorbent assay (ELISA).* A commercially available ELISA kit, Chlamydien-IgG-ELISA medac (Medac Gmbh, Germany), which employs a highly purified and specific antigen was used to measure anti-C. *pneumoniae* antibody titres. Three dilutions of sera, 400x, 200x, 100x were tested, and reactivities were expressed as titres > 1:400; 1:400; 1:200; 1:100 and <1:100.

*Immunoblotting.* Elementary bodies (EB), used as bacterial antigen extract were isolated from *C*. *pneumoniae* AR39 infected HeLa cell cultures according to Wang et al. (1991). The infectivity of EBs was destroyed and proteins were solubilised by adding SDS-PAGE sample buffer containing SDS and 2-mercaptoethanol. Approximately 5 µg total proteins were separated by SDS-PAGE using the BioRad Mini-Protean 3 Cell electrophoresis system and proteins were transferred to nitrocellulose membrane (ECL, Amersham Pharmacia). After overnight blocking in 5% milk, human sera were added at 5,000x dilution, and HRP labelled anti-human IgG was used for detection.

*Purification of antibodies for genomic screening.* Five sera were selected based on the overall anti-chlamydial titres for a serum pool used in the screening procedure. Antibodies against *E. coli* proteins were removed by incubating the heat-inactivated sera with whole cell *E. coli* cells (DH5 alpha, transformed with pHIE11, grown under the same condition as used for bacterial surface display). Highly enriched preparations of IgAs from the pooled, depleted sera were purified by affinity chromatography using biotin-labeled anti-human IgA (Southern Biotech) immobilized on Streptavidin-agarose (GIBCO BRL). The efficiency of IgA purification was checked by SDS-PAGE and protein concentration measurements (OD₂₈₀ₙₘ).

### Results

As stated above, the antibodies produced against *C*. *pneumoniae* by the human immune system and present in human sera are indicative of the *in vivo* expression of the antigenic proteins and their immunogenicity. These molecules are essential for the identification of individual antigens in the approach as described in the present invention, which is based on the interaction of the specific anti-chlamydial antibodies and the corresponding *C*. *pneumoniae* peptides or proteins. To gain access to relevant antibody repertoires, human sera were collected from patients with symptoms of *C*. *pneumoniae* related infections, such as pneumonia, and bronchitis. *C*. *pneumoniae* was indicated to be the causative agent by medical serological tests.

Infections with *Chlamydia pneumoniae* are detected and diagnosed by serology, since the pathogen is not culturable with routine microbiological methods. Highly specific and sensitive diagnostic kits based on antigen detection have been developed and are available commercially. We have selected patients' sera having a high titre against *C. pneumoniae* detected by a standard *Chlamydia* ELISA kit routinely used in the clinic for diagnosis of acute, chronic and persistent infections caused by *Chlamydia* species. 185 serum samples were tested, all derived from individuals selected for diagnostic testing for the presence of *Chlamydia pneumoniae* specific antibodies based on clinical symptoms. 83 sera showed antibody titres > 1:400; 34 sera showed titres of approximately 1:400; 14 sera of 1:200; 20 sera of 1:100 and 34 sera had titres < 1:100. According to epidemiologic studies *C. pneumoniae* carriage and infection is widespread, with frequent reinfection during lifetime. For that reason, primary selection of sera aimed at the identification of samples with the highest IgG titre (> 400) to reduce the risk of non-specific, false positive diagnosis.

Subsequently, pre-selected sera were analyzed by immunoblotting to ensure antibody reactivities against multiple proteinaceous antigens present in *C. pneumoniae.* The representative immunoblot shown in Figure 1 demonstrates that different patterns of reactivities were detected with the individual sera when tested against proteins of elementary bodies, isolated from infected human cells (HeLa) in *in vitro* cultures. Special attention was made to select sera displaying different pattern of reactivities based on these immunoblot analysis.

Five selected sera were pooled to further enrich for abundant antibodies, but still having a representation of antibody repertoires of different individuals. Based on the data obtained for IgG antibodies, IgAs were purified from the pooled serum (comprising the individual sera P922, P923, P925, P928, and P949; the latter three are shown in Fig. 1) by affinity chromatography and depleted of E. *coli* -reactive antibodies to avoid background in the bacterial surface display screen.

### Example 3: Generation of highly random, frame-selected, small-fragment, genomic DNA libraries of C. pneumoniae

### Experimental procedures

*Preparation of chlamydial genomic DNA. C. pneumoniae* AR39 was cultivated as described in Campbell, L.A. et al. (1989). Elementary bodies (EB) were isolated and used for the preparation of genomic DNA. Genomic DNA from *C. pneumoniae* EBs was prepared as described by Cox, R.L. et al (1988). The final genomic DNA preparation was dissolved in ddH₂O.

*Preparation of small genomic DNA fragments.* Genomic DNA from *C*. *pneumoniae* AR39 was mechanically sheared into fragments ranging in size between 150 and 300 bp using a cup-horn sonicator (Bandelin Sonoplus UV 2200 sonicator equipped with a BB5 cup horn, 10 sec. pulses at 100% power output) or into fragments of size between 50 and 70 bp by mild DNase I treatment (Novagen). It was observed that sonication yielded a much tighter fragment size distribution when breaking the DNA into fragments of the 150-300 bp size range. However, despite extensive exposure of the DNA to ultrasonic wave-induced hydromechanical shearing force, subsequent decrease in fragment size could not be efficiently and reproducibly achieved. Therefore, fragments of 50 to 70 bp in size were obtained by mild DNase I treatment using Novagen's shotgun cleavage kit. A 1:20 dilution of DNase I provided with the kit was prepared and the digestion was performed in the presence of MnCl₂ in a 60 µL volume at 20°C for 5 min to ensure double-stranded cleavage by the enzyme. Reactions were stopped with 2 µL of 0.5 M EDTA and the fragmentation efficiency was evaluated on a 2% TAE-agarose gel. This treatment resulted in total fragmentation of genomic DNA into near 50-70 bp fragments. Fragments were then blunt-ended twice using T4 DNA Polymerase in the presence of 100 µM each of dNTPs to ensure efficient flushing of the ends. Fragments were used immediately in ligation reactions or frozen at -20°C for subsequent use.

*Description of the vectors.* The vector pMAL4.31 was constructed on a pASK-IBA backbone (Skerra, A., 1994) with the beta-lactamase *(bla)* gene exchanged with the Kanamycin resistance gene. In addition, the *bla* gene was cloned into the multiple cloning site. The sequence encoding mature beta-lactamase is preceded by the leader peptide sequence of *ompA* to allow efficient secretion across the cytoplasmic membrane. Furthermore a sequence encoding the first 12 amino acids (spacer sequence) of mature beta-lactamase follows the *ompA* leader peptide sequence to avoid fusion of sequences immediately after the leader peptidase cleavage site, since e.g. clusters of positive charged amino acids in this region would decrease or abolish translocation across the cytoplasmic membrane (Kajava, A. et al., 2000). A *Sma*I restriction site serves for library insertion. An upstream FseI site and a downstream NotI site, which were used for recovery of the selected fragment, flank the *Sma*I site. The three restriction sites are inserted after the sequence encoding the 12 amino acid spacer sequence in such a way that the *bla* gene is transcribed in the -1 reading frame resulting in a stop codon 15 bp after the *Not*I site. A +1 bp insertion restores the *bla* ORF so that beta-lactamase protein is produced with a consequent gain of Ampicillin resistance.

The vector pMAL9.1 was constructed by cloning the *lamB* gene into the multiple cloning site of pEH1 (Hashemzadeh-Bonehi, L. et al., 1998). Subsequently, a sequence was inserted in *lamB* after amino acid 154, containing the restriction sites *Fse*I*, Sma*I and *Not*I*.* The reading frame for this insertion was constructed in such a way that transfer of frame-selected DNA fragments excised by digestion with *Fse*I and *Not*I from plasmid pMAL4.31 yields a continuous reading frame of *lamB* and the respective insert.

The vector pHIE11 was constructed by cloning the *fhuA* gene into the multiple cloning site of pEH1. Thereafter, a sequence was inserted in *fhuA* after amino acid 405, containing the restriction site *Fse*I*, Xba*I and *Not*I*.* The reading frame for this insertion was chosen in a way that transfer of frame-selected DNA fragments excised by digestion with *Fse*I and *Not*I from plasmid pMAL4.31 yields a continuous reading frame of *fhuA* and the respective insert.

*Cloning and evaluation of the library for frame selection.* Genomic *C. pneumoniae* AR39 DNA fragments were ligated into the *Sma*I site of the vector pMAL4.31. Recombinant DNA was electroporated into DH10B electrocompetent *E. coli* cells (GIBCO BRL) and transformants plated on LB-agar supplemented with Kanamycin (50 µg/ml) and Ampicillin (50 µg/ml). Plates were incubated over night at 37°C and colonies collected for large scale DNA extraction. A representative plate was stored and saved for collecting colonies for colony PCR analysis and large-scale sequencing. A simple colony PCR assay was used to initially determine the rough fragment size distribution as well as insertion efficiency. From sequencing data the precise fragment size was evaluated, junction intactness at the insertion site as well as the frame selection accuracy (*3n*+*1 rule).*

*Cloning and evaluation of the library for bacterial surface display.* Genomic DNA fragments were excised from the pMAL4.31 vector, containing the *C. pneumoniae* library with the restriction enzymes *Fse*I and *Not*I*.* The entire population of fragments was then transferred into plasmids pMAL9.1 (LamB) or pHIE11 (FhuA), which have been digested with *Fse*I and *Not*I*.* Using these two restriction enzymes, which recognise an 8 bp GC rich sequence, the reading frame that was selected in the pMAL4.31 vector is maintained in each of the platform vectors. The plasmid library was then transformed into *E. coli* DH5alpha cells by electroporation. Cells were plated onto large LB-agar plates supplemented with 50 µg/mL Kanamycin and grown over night at 37°C at a density yielding clearly visible single colonies. Cells were then scraped off the surface of these plates, washed with fresh LB medium and stored in aliquots for library screening at -80°C.

### Results

*Libraries for frame selection.* Two libraries (LCPn-50 and LCPn-300) were generated in the pMAL4.31 vector with sizes of approximately 50 and 300 bp, respectively. For each library, ligation and subsequent transformation of approximately 1 µg of pMAL4.31 plasmid DNA and 50 ng of fragmented genomic *C*. *pneumoniae* AR39 DNA yielded 6x 10⁴ to 3x 10⁵ clones after frame selection. To assess the randomness of the libraries, 480 randomly chosen clones of LCPn-50 were sequenced. After trimming of the vector sequences, 390 could be subjected to bioinformatic analysis, showing that of these clones only very few were present more than once. Furthermore, it was shown that 98% of the clones fell in the size range between 25 and 100 bp with an average size of 46 bp (Figure 2). Almost all sequences followed the 3n+1 rule, showing that all clones were properly frame selected.

*Bacterial surface display libraries.* The display of peptides on the surface of *E. coli* required the transfer of the inserts from the LCPn libraries from the frame selection vector pMAL4.31 to the display plasmids pMAL9.1 (LamB) or pHIE11 (FhuA). Genomic DNA fragments were excised by *Fse*I and *Not*I restriction and ligation of 5 ng inserts with 0.1 µg plasmid DNA and subsequent transformation into DH5alpha cells resulted in 2x 10⁵ to 2x 10⁶ clones. The clones were scraped off the LB plates and frozen without further amplification.

### Example 4: Identification of highly immunogenic peptide sequences from C. pneumoniae using bacterial surface displayed genomic libraries and human serum

### Experimental procedures

*MACS screening.* Approximately 2.5x 10⁸ cells from a given library were grown in 5 mL LB-medium supplemented with 50 µg/mL Kanamycin for 2 h at 37°C. Expression was induced by the addition of 1 mM IPTG for 30 min. Cells were washed twice with fresh LB medium and approximately 2x 10⁷ cells re-suspended in 100 µL LB medium and transferred to an Eppendorf tube.

10 to 20 µg of biotinylated, human IgAs purified from serum was added to the cells and the suspension incubated overnight at 4°C with gentle shaking. 900 µL of LB medium was added, the suspension mixed and subsequently centrifuged for 10 min at 6,000 rpm at 4°C. Cells were washed once with 1 mL LB and then re-suspended in 100 µL LB medium. 10 µL of MACS microbeads coupled to streptavidin (Miltenyi Biotech, Germany) were added and the incubation continued for 20 min at 4°C. Thereafter 900 µL of LB medium was added and the MACS microbead cell suspension was loaded onto the equilibrated MS column (Miltenyi Biotech, Germany) which was fixed to the magnet. (The MS columns were equilibrated by washing once with 1 mL 70% EtOH and twice with 2 mL LB medium.)

The column was then washed three times with 3 mL LB medium. After removal of the magnet, cells were eluted by washing with 2 mL LB medium. After washing the column with 3 mL LB medium, the 2 mL eluate was loaded a second time on the same column and the washing and elution process repeated. The loading, washing and elution process was performed a third time, resulting in a final eluate of 2 ml.

Cells selected after two rounds of selection were plated onto LB-agar plates supplemented with 50 µg/mL Kanamycin and grown over night at 37°C.

*Evaluation of selected clones by sequencing and Western blot analysis.* Randomly selected clones were grown overnight at 37°C in 3 mL LB medium supplemented with 50 µg/mL Kanamycin to prepare plasmid DNA using standard procedures. Sequencing was performed at MWG (Germany) or Agowa (Germany).

For Western blot analysis approximately 10 to 20 µg of total cellular protein was separated by 10% SDS-PAGE and blotted onto HybondC membrane (Amersham Pharmacia Biotech, England). The LamB or FhuA fusion proteins were detected using human serum as the primary antibody at a dilution of approximately 1:3,000 to 1:5,000 and anti-human IgA antibodies coupled to HRP at a dilution of 1:5,000 as secondary antibodies. Detection was performed using the ECL detection kit (Amersham Pharmacia Biotech, England). Alternatively, rabbit anti-FhuA or rabbit anti-LamB polyclonal immune sera were used as primary antibodies in combination with the respective secondary antibodies coupled to HRP for the detection of the fusion proteins.

### Results

*Screening of bacterial surface display libraries by magnetic activated cell sorting (MACS) using biotinylated IgAs.* The libraries LCPn-50 in pMAL9.1 and LCPn-300 in pHIE11 were screened with a pool of biotinylated, human IgAs from patient sera (see Example 2: *Purification of antibodies for genomic screening,* and the last paragraph of the results of Example 2). The selection procedure was performed as described under Experimental procedures. Figure 3A shows the data obtained with the screen of the LCPn-50 library and P14-IgAs. As can be seen from the colony count after the first selection cycle from MACS screening, the total number of cells recovered at the end is drastically reduced from 1x10⁸ cells to approximately 5x 10⁴ cells, while the selection without antibodies added showed a reduction to a number of about 1x 10 cells (Figure 3A). After the second round, app. 1x 10⁴ cells were recovered with P14-IgAs, while only 1-2x 10³ cells were recovered when no IgAs from human serum were added, clearly showing that selection was dependent on *C*. *pneumoniae* specific antibodies. To evaluate the performance of the screen, 25 selected clones were picked randomly and subjected to immunoblot analysis with the screening IgA pool (P14-IgA) (Figure 3B). This analysis revealed that app. 80% of the selected clones showed reactivity with antibodies present in the relevant serum, whereas the control strain expressing LamB without a *C*. *pneumoniae* specific insert did not react with the same serum (not shown). In general, the rate of reactivity was observed to lie within the range of 40 to 80%. Colony PCR analysis showed that all selected clones contained an insert in the expected size range.

Subsequent sequencing of a larger number of randomly picked clones (app. 600 clones per screen) led to the identification of the gene and the corresponding peptide or protein sequence that was specifically recognized by the human serum antibodies used for screening. The frequency with which a specific clone is selected reflects at least in part the abundance and/or affinity of the specific antibodies in the serum used for selection and recognizing the epitope presented by this clone. In that regard it is striking that clones derived from some ORFs (e.g. CP0210, CP0212) were picked very frequently (27 to 50 times), indicating their highly immunogenic property. Table 1 summarizes the data obtained for the two performed screens. All clones that are presented in Table 1 have been verified by immunoblot analysis using whole cellular extracts from single clones to show the indicated reactivity with the pool of human serum used in the respective screen. As can be seen from Table 1, distinct regions of the respective ORF are identified as immunogenic, since variably sized fragments of the proteins are displayed on the surface by the platform proteins.

It is further worth noticing that a large number of the genes identified by the bacterial surface display screen encode proteins of *C*. *pneumoniae,* which have no assigned function or may even constitute proteins, which have not been predicted by previous bioinformatic analysis. Thus, many of these candidates constitute novel antigenic proteins of *C*. *pneumoniae.*

### Example 5: Gene distribution studies with highly immunogenic proteins identified from C. pneumoniae

### Experimental procedures

*Gene distribution of antigens by PCR.* An ideal vaccine antigen would be an antigen that is present in all, or the vast majority of strains of the target organism the vaccine is directed to. In order to establish whether the genes encoding the identified *C*. *pneumoniae* antigens occur ubiquitously in the relevant strains, PCR was performed on a series of independent bacterial isolates with primers specific for the gene of interest. Oligonucleotide sequences as primers were designed for all identified ORFs yielding products of approximately 1,000 bp, if possible covering all identified immunogenic epitopes.

For the preparation of genomic DNA, *Chlamydia* cells cultured in one 6-well plate were harvested and transferred to a Falcon tube. Cells were disrupted by shaking for 10 min in the presence of glass beads and cell debris removed by subsequent centrifugation for 5 min at 900 rpm at 4°C. The supernatant was centrifuged for 1 hour at 13,000 rpm at 10°C to collect chromosomal DNA. The pellet was re-suspended in 1 mL PBS und stored in 200 µL aliquots. The extraction of genomic DNA was performed with the QIAamp DNA Mini Kit (Qiagen) according to the manufacturer's recommendations.

PCR was performed in a reaction volume of 25 µL using Taq polymerase (1 U), 200 nM dNTPs, 10 pMol of each oligonucleotide and the kit according to the manufacturer's instructions (Invitrogen, The Netherlands). As standard, 30 cycles (1x: 5 min. 95°C, 30x: 30 sec. 95°C, 30 sec. 56°C, 30 sec. 72°C, 1x 4 min. 72°C) were performed, unless conditions had to be adapted for individual primer pairs.

### Results

A selection of 27 identified genes encoding immunogenic proteins was tested by PCR for their presence in 17 different *Chlamydia* strains (15 *C. pneumoniae* and 2 *C*. *trachomatis;* Table 2). All negative PCR data were confirmed by an independent experiment to eliminate false negative results. Due to the use of specific oligonucleotides for each specific gene, a negative PCR may also result from a mismatch in the 3' region of the oligonucleotide. Yet in most cases it was not considered essential to confirm the absence of the gene by further means. 85% (23 of 27) were detected in >90% of *C*. *pneumoniae* strains (>13/15), while only 1 was missing in > 75% of the *C*. *pneumoniae* strains (<12/17) and therefore categorized as not sufficiently conserved. In addition several genes were also present in the two *C*. *trachomatis* strains analyzed (e.g. CP0064, CP0664). As an example, Figure 4 shows the PCR reaction for the two *C*. *pneumoniae* antigens CP0238 and CP0242 with all indicated 17 strains. As clearly visible, the CP0242 gene is present in all strains analyzed, whereas the CP0238 gene is absent in 3 strains and only weakly amplified from an additional 5-6 strains. All results with the selected antigens are summarized in Table 3. Importantly, more than 90% of the tested antigens were well conserved among the analyzed strains of *Chlamydia pneumoniae,* concerning the presence and size of the gene-specific PCR products. Therefore these antigens represent valuable candidates to warrant further studies to evaluate their vaccine potential.

### Example 6: Identification of HLA class II-restricted T cell epitopes or epitope regions within the selected antigens

### Experimental procedures

### HLA class II-restricted epitope prediction

The prediction of HLA class II-restricted epitopes within the antigens identified by bacterial display was performed using the program IEDB Analysis resource (Bui, H.H. et al., 2005). The prediction was performed for the twelve MHC II types HLA DRB1*0101, *0301, *0401, *0405, *0701, *0802, *0901, *1101, *1302, *1501, HLADRB4*0101 and HLADRB5*0101. Epitopes with a binding stronger than IC50=5000 nM were selected. To determine the regions where most of the epitopes were located, the localization of the ten strongest binding epitopes for each allele were determined. This enabled us to see if epitopes from different alleles bound to the same region. These regions were then selected and analyzed in the IEDB program for MHCII binding peptides. Regions were chosen that contain a hit in at least four MHCII alleles. Only in cases where epitopes overlap continuously in a larger region, the whole region (potentially larger than 25 amino acids) is depicted.

### Results

T cell epitopes are the minimal essential units of information derived from nonself (or self) proteins that stimulate cellular (T cell) immune responses. They are presented in the cleft of MHC class I or class II molecules at the surface of the antigen-presenting cell to the T cell receptor (TCR). The following cascade of cellular events triggered by the interaction of a TCR and the pathogen-derived peptide epitope in the cleft of an MHC molecule serves to inform the cellular immune system that bacteria, viruses or parasites are present. Induction of epitope-specific T cell responses may improve immune responses to pathogens for which no conventional vaccines currently exist and thus provide a means to allow protection from infection or to clear an infection by the respective pathogen. The accuracy of the bioinformatic prediction methods for T cell epitopes are remarkable (Martin, W. et al., 2003) and thus offer a complementary method to the described antigen identification approach by bacterial surface display, which is based on the experimental identification on B cell epitopes. Since the ORFs, corresponding to the antigens identified on the basis of recognition by antibodies in human sera, most likely also contain linear T-cell epitopes it was the aim of this invention to provide also a set of T cell epitopes for the listed antigens.

The molecular definition of the corresponding HLA class II helper-epitopes is useful for the design of synthetic anti-chlamydial vaccines, which can induce immunological memory, because the helper-epitopes derived from the chlamydial antigens provide "cognate help" to the B-cell response against these antigens or fragments thereof. Moreover it is possible to use these helper-epitopes to induce memory to T-independent antigens like for instance carbohydrates (conjugate vaccines). MHC class II molecules bind peptides consisting of 11 to 25 amino acids (with a recognition sequence of 9 amino acids) and are predominantly recognized by CD4+ helper T cells. As is evident from Table 1, almost all antigens identified by bacterial surface display contain a number of potential MHC class II-restricted epitopes, which may also overlap with the identified B cell epitopes (e.g. CP0444).

In addition, intracellular *Chlamydia pneumoniae* can be eliminated by CD8+ cytotoxic T-cells, which recognize HLA class I-restricted epitopes. MHC class I molecules present in general peptides of 8 to 10 amino acids in length with two conserved anchor residues. In the context of a protective immune response, epitope-specific T cells can persist as memory cells, thus allowing a more rapid response to the pathogen upon encounter. Therefore and since the two types of cellular immune response are complementary, preventive as well as therapeutic vaccines should be designed to contain both class I-restricted and class II-restricted epitopes. Epitopes can also be predicted using bioinformatic algorithms, but are not specifically listed here.

The identified peptides or fragments of an antigen (for instance overlapping 15-mers) can be synthesized and tested for their ability to bind to various MHC molecules *in vitro.* Their immunogenicity can be tested by assessing the peptide (antigen)-driven proliferation (BrdU or 3H-thymidine incorporation) or the secretion of cytokines (ELIspot, intracellular cytokine staining) of T-cells *in vitro* (Schmittel, A. et al., 2000; Sester, M. et al., 2000). In this regard it will be interesting to determine quantitative and qualitative differences in the T-cell response to the chlamydial antigens or the selected promiscuous peptides or fragments thereof e.g. in populations of patients with different chlamydial infections, or in colonized versus healthy individuals neither recently infected nor colonized. In addition, the immunogenicity of the predicted peptides can be tested in HLA-transgenic mice (Sonderstrup, G. et al., 1999).

Furthermore, the antigens/epitopes may be injected into mice and the induced antibodies and T cell responses can then be determined. The protective capacity of the antibodies and T cells induced by the antigens through vaccination can be assessed in animal models. All these approaches are well available to the skilled man in the art.

### References

**The following references which have been recited in the present specification in a truncated version are incorporated herein by reference in their entirety.**
Aldous, M.B., et al. (1992). J Infect Dis 166: 646-9.
Altschul, S., et al. (1990). Journal of Molecular Biology 215: 403-10.
Amit, A.G., et al. (1986). Science 233: 747-753.
Bennett, D., et al. (1995). J Mol Recognit 8: 52-8.
Block, S., et al. (1995). Pediatr Infect Dis J 14: 471-477.
Boman, J., et al. (1999). J Clin Microbiol 37: 3791-3799.
Braun, J., et al. (1994). Ann Rheum Dis 53: 100-5.
Brunelle, B.W., et al. (2006). Infect Immun 74: 578-585.
Bui, H.H., et al. (2005). Immunogenetics 57: 304-314.
Campbell, L.A., et al. (1989). Infect Immun 57: 71-5.
Carlson, J.H., et al. (2005). Infect Immun 73: 6407-18.
Carter, P., et al. (1985). Nucl. Acids Res. 13: 4431-4443.
Ciarrocchi, G., et al. (2004). New Microbiol 27: 335-343.
Clackson, T., et al. (1991). Nature 352: 624-8.
Cohen, J. (1993). Science 259: 1691-1692.
Cox, R.L., et al. (1988). Int J Syst Bacteriol 38: 265-8.
Current Protocols in Molecular Biology, John Wiley and Sons, Inc. (1987).
Devereux, J., et al. (1984). Nucleic acids research 12: 387-95.
Doherty, E., et al. (2001). Annu Rev Biophys Biomol Struct 30: 457-475.
Dowell, S.F. (2001). Clin Infect Dis 33: 492-503.
Eisenbraun, M., et al. (1993). DNA Cell Biol 12: 791-7.
Etz, H., et al. (2001). J Bacteriol 183: 6924-35.
Everett, K.D., et al. (1999). Int J Syst Bacteriol 49: 415-40.
Falsey, A.R., et al. (1993). J Infect Dis 168: 493-6.
Ganz, T. (1999). Science 286: 420-421.
Gaydos, C.A., et al. (1994). J Clin Microbiol 32: 903-5.
Georgiou, G. (1997). Nature Biotechnology 15: 29-34.
Grayston, J.T. (1992). Clin Infect Dis 15: 757-61.
Grayston, J.T., et al. (1986). N Engl J Med 315: 161-8.
Grayston, J.T., et al. (1996). Rev Med Interne 17: 45S-47S.
Hahn, D.L., et al. (1991). JAMA 266: 225-30.
Haidl, S., et al. (1992). N Engl J Med 326: 576-7.
Hashemzadeh-Bonehi, L., et al. (1998). Mol Microbiol 30: 676-678.
Hauer, A.D., et al. (2006). Cardiovasc Res 69: 280-288.
Hemmer, B., et al. (1999). Nat Med 5: 1375-82.
Hornef, M., et al. (2002). Nat Immunol 3: 1033-40.
Huse, W.D., et al. (1988). Science 246: 1275-1281.
Ishibashi, S., et al. (1993). J. Clin. Invest. 92: 883-893.
Johanson, K., et al. (1995). J Biol Chem 270: 9459-71.
Jones, P., et al. (1986). Nature 321: 522-5.
Kajava, A., et al. (2000). J Bacteriol 182: 2163-9.
Kalman, S., et al. (1999). Nature 21: 385-9.
Kay, M., et al. (1994). Proc. Natl. Acad. Sci. USA 91: 2353-2357.
Kleemola, M., et al. (1988). J Infect Dis 157: 230-6.
Köhler, G., et al. (1975). Nature 256: 495-7.
Kolaskar, A.S., and Tongaonkar, P.C. (1990). FEBS Lett 276: 172-4.
Kuo, C.-C., et al. (1995). Clin Microbiol Rev 8: 451-61.
Lewin, A., et al. (2001). Trends Mol Med 7: 221-8.
Little, C.S., et al. (2004). Neurobiol Aging 25: 419-429.
Marangoni, A., et al. (2006). World J Gastroenterol 12: 6453-6457.
Marks, J., et al. (1992). Biotechnology (N Y) 10: 779-83.
Martin, W., et al. (2003). Methods 29: 289-98.
McCafferty, J., et al. (1990). Nature 348: 552-4.
Montigiani, S., et al. (2002). Infect Immun 70: 368-79.
Murdin, A.D., et al. (2000). J Infect Dis 181: S544-51.
Nagy, E., et al. (2003). Identification of the "antigenome" - a novel tool for design and development of subunit vaccines against bacterial pathogens. In Genomics, Proteomics and Vaccines (G. Grandi, ed), John Wiley & Sons Ltd., UK
Ogawa, H., et al. (1992). J Laryngol Oto 106: 490-2.
Okano, H., et al. (1991). J Neurochem 56: 560-7.
Oligodeoxynucleotides as Antisense Inhibitors of Gene Expression; CRC Press, Boca Tation, FL (1988).
Queen, C., et al. (1989). Proc. Natl. Acad. Sci. USA 86: 10029-10033.
Rammensee, H., et al. (1999). Immunogenetics 50: 213-9.
Read, T.D., et al. (2000). Nucleic Acids Research 28: 1397-1406.
Read, T.D., et al. (2003). Nucleic Acids Research 31: 2134-47.
Remington's Pharmaceutical Sciences, by E. W. Martin, Mack Publishing Co., Easton, PA, 15th Edition (1975).
Riechmann, L., et al. (1988). Nature 332: 323-327.
Roblin, P.M., et al. (1992). J Clin Microbiol 30: 1968-1971.
Rodriguez. A., et al. (2006). Scand J Immunol 63: 177-183.
Schmittel, A., et al. (2000). J Immunother 23: 289-95.
Seeger, C., et al. (1984). Proc Natl Acad Sci USA 81: 5849-52.
Sester, M., et al. (2000). AIDS 14: 2653-60.
Shirai, M., et al. (2000). Nucleic Acids Research 28: 2311-4.
Shor, A., et al. (1992). S Afr Med J 82: 158-61.
Skerra, A. (1994). Gene 151: 131-5.
Sonderstrup, G., et al. (1999). Immunol Rev 172: 335-43.
Stephens, R.S., et al. (1998). Science 282: 754-9.
Sundelof, B., et al. (1993). Scand J Infect Dis 25: 259-61.
Tang, D., et al. (1992). Nature 356: 152-4.
Tempest, P., et al. (1991). Biotechnology (N Y) 9: 266-71.
Tong, C.Y., et al. (1993). J Clin Pathol 46: 313-7.
Tourdot, S., et al. (2000). Eur J Immunol 30: 3411-21.
Tuuminen, T., et al. (2000). J Microbiol Methods 42: 265-279.
von Heinje, G. (1987). Sequence Analysis in Molecular Biology, Academic Press.
Wang, S.P., et al. (1991). J Clin Microbiol 29: 1539-41.
Wells, J.A., et al. (1985). Gene 34: 315-323.
Wells, J.A., et al. (1986). Philos. Trans. R. Soc. London Ser.A 317: 415.
Wizel, B., et al. (2002). J Immunol 169: 2524-35.
Yang, Z.P., et al. (1993). Infect Immun 61: 2037-2040.
Zoller, M.J., et al. (1987). Nucl. Acids Res. 10: 6487-6500.

The features of the present invention disclosed in the specification, the claims and/or the drawings may both separately and in any combination thereof be material for realizing the invention in various forms thereof.

## Claims

1. An antigen consisting of the amino acid sequence 569-636 of Seq ID No: 124.

2. The antigen of claim 1, whereby the antigen further consists of 1 to 50 additional amino acid residue(s), preferably 1 to 40, more preferably 1 to 30, even more preferably at most 1 to 25, still more preferably at most 1 to 10, most preferably 1, 2, 3, 4 or 5 additional amino acid residue(s), particularly wherein the additional amino acid residue(s) is/are flanking the antigen N-terminally, C-terminally or N- and C-terminally.

3. An antigen for use as a medicament, wherein the antigen comprises
a) an isolated polypeptide of Seq ID No: 124; or
b) an active variant of the isolated polypeptide of Seq ID No: 124, wherein the active variant has at least 95% sequence identity to the polypeptide of Seq ID No: 124; or
c) an active fragment of the isolated polypeptide of Seq ID No: 124 consisting of amino acids 569-636 of Seq ID No: 124.

4. The antigen for use as medicament according to claim 3, whereby the antigen further consists of 1 to 50 additional amino acid residue(s), preferably 1 to 40, more preferably 1 to 30, even more preferably at most 1 to 25, still more preferably at most 1 to 10, most preferably 1, 2, 3, 4 or 5 additional amino acid residue(s), particularly wherein the additional amino acid residue(s) is/are flanking the antigen N-terminally, C-terminally or N- and C-terminally.

5. A nucleic acid for use as a medicament, wherein said nucleic acid encodes the antigen according to any one of claims 1 to 4.

6. An antibody wherein said antibody comprises an isolated antibody, or at least an effective part thereof, which binds to at least a selective part of an antigen according to claim 1.

7. An antibody for use as a medicament wherein said antibody comprises an isolated antibody, or at least an effective part thereof, which binds to at least a selective part of an antigen according to claim 3 or 4.

8. The antibody of claim 6 or 7
a) wherein the antibody is a monoclonal antibody;
b) wherein said effective part comprises an Fab fragment, an F(ab) fragment, an F(ab)N fragment, an F(ab)2 fragment or an Fv fragment;
c) wherein the antibody is a chimeric antibody;
d) wherein the antibody is a humanized antibody; and/or
e) wherein the antibody is an IgA antibody.

9. A process for producing an antigen according to any one of claims 1 to 4 comprising expressing a nucleic acid molecule encoding said antigen.

10. A pharmaceutical composition comprising the antigen according to claim 3 or 4, the nucleic acid molecule as defined in claim 5, a vector comprising said nucleic acid as defined in claim 5 or an antibody according to claim 6, 7 or 8 for use as a medicament.

11. A vaccine comprising the antigen according to claim 3 or 4, the nucleic acid molecule as defined in claim 5 or a vector comprising said nucleic acid as defined in claim 5 for use as a medicament.

12. The pharmaceutical composition or vaccine according to claim 10 or 11 comprising at least one additional antigen against a *Chlamydia* species or an antigen against a further pathogen.

13. The pharmaceutical composition or vaccine according to any one of claims 10 to 12 **characterised in that** it further comprises an immunostimulatory substance, preferably polycationic polymers, especially polycationic peptides, immunostimulatory oligodeoxynucleotides (ODNs), especially oligo(dIdC)₁₃, peptides containing at least two LysLeuLys motifs, especially peptide KLKLLLLLKLK, neuroactive compounds, especially human growth hormone, alum, Freund's complete or incomplete adjuvants, or combinations thereof, particularly wherein said immunostimulatory substance is a combination of either a polycationic polymer and an immunostimulatory oligodeoxynucleotide or of a peptide containing at least two LysLeuLys motifs and an immunostimulatory oligodeoxynucleotide, preferably a combination of KLKLLLLLKLK and oligo(dIdC)₁₃ and/or particularly wherein said polycationic peptide is polyarginine.

14. The antigen according to any one of claims 1 to 4, the nucleic acid according to claim 5, the antibody according to claims 6, 7 or 8 or the pharmaceutical composition or vaccine of any one of claims 10 to 13 for the treatment or prevention of an infection with *Chlamydia,* preferably *C. pneumoniae, C. trachomatis, C. psittaci, C. pecorum, C. muridarum,* or *C*. *suis,* and more preferably *C. pneumoniae, C. trachomatis* or C. *psittaci.*

15. The antigen according to any one of claims 1 to 4, the nucleic acid according to claim 5 or the antibody according to claims 6, 7 or 8 for the diagnosis of an infection with *Chlamydia,* preferably *C. pneumoniae, C. trachomatis, C. psittaci, C. pecorum, C. muridarum,* or *C*. *suis,* and more preferably *C. pneumoniae, C. trachomatis* or *C*. *psittaci.*
